# EUROPEAN PATENT SPECIFICATION

(11) **EP 1 047 301 B1**
(45) Date of publication and mention of the grant of the patent: **29.08.2007**
(21) Application number: 99905840.7
(22) Date of filing: 08.02.1999
(51) Int. Cl.: A01N 63/00, A61K 39/12, C07H 21/04

(54) **PROTEINS ENCODED BY POLYNUCLEIC ACIDS OF PORCINE REPRODUCTIVE AND RESPIRATORY SYNDROME VIRUS (PRRSV)**
DURCH POLYNUKLEINSÄUREN VOM REPRODUKTIVEN UND RESPIRATORISCHEN SYNDROMVIRUS DES SCHWEINES KODIERTE PROTEINE
PROTEINES CODEES PAR DES ACIDES POLYNUCLEIQUES DU VIRUS DU SYNDROME DYSGENESIQUE RESPIRATOIRE PORCIN (SDRP)

(30) Priority: 06.02.1998 US 19793
(43) Date of publication of application: 02.11.2000
(73) Proprietor: Iowa State University Research Foundation, Inc., Ames, IA 50011-2131 (US)
(72) Inventor: PAUL, Prem, S., Ames, IA 50014 (US); ZHANG, Yanjin, San Antonio, TX 78229 (US)
(74) Representative: Crump, Julian Richard John
(86) International application number: PCT/US1999/002630
(87) International publication number: WO 1999/039582

(56) References cited:
- EP-A2- 0 595 436
- WO-A-96/06619
- WO-A1-96/40932
- US-A- 5 695 766
- MARDASSI H ET AL: "MOLECULAR ANALYSIS OF THE ORFS 3 TO 7 PORCINE REPRODUCTIVE AND RESPIRATORY SYNDROME VIRUS, QUEBEC REFERENCE STRAIN" ARCHIVES OF VIROLOGY,NEW YORK, NY,US, vol. 140, no. 8, 1995, pages 1405-1418, XP000827987 ISSN: 0304-8608
- MOROZOV I ET AL: "SEQUENCE ANALYSIS OF OPEN READING FRAMES(ORFS)2 TO 4 OF A U.S. ISOLATE OF PORCINE REPRODUCTIVE AND RESPIRATORY SYNDROME VIRUS" ARCHIVES OF VIROLOGY,NEW YORK, NY,US, vol. 140, no. 7, 1995, pages 1313-1319, XP000827986 ISSN: 0304-8608
- KAPUR et al., "Genetic Variation in Porcine Reproductive and Respiratory Syndrome Virus Isolates in the Midwestern United States", JOURNAL OF GENERAL VIROLOGY, 1996, Vol. 77, pages 1271-1276, XP002919855

### SEQUENCE LISTING

<110> IOWA STATE UNIVERSITY RESEARCH FOUNDATION AMERICAN CYANAMID COMPANY
<120> PROTEINS ENCODED BY POLYNUCLEIC ACIDS OF PORCINE REPRODUCTIVE AND RESPIRATORY SYNDROME VIRUS (PPRSV)
<130> 8199-0005-55XCIP WO
<140> PCT/US99/02630
   <141> 1999-02-08
<150> US 09/019, 793
   <151> 1998-02-06
<150> US 08/478,316
   <151> 1995-06-07
<150> US 08/301,435
   <151> 1994-09-01
<150> US 08/131,625
   <151> 1993-10-05
<150> US 07/969,071
   <151> 1992-10-30
<160> 175
<170> PatentIn Ver. 2.0
<210> 1
   <211> 2352
   <212> DNA
   <213> Porcine reproductive and respiratory syndrome virus
<400> 1
<210> 2
   <211> 2349
   <212> DNA
   <213> Porcine reproductive and respiratory syndrome virus
<400> 2
   cctatcattg aaccaacttt gggtctagac tgaaatgcaa tggggtccat gcaaagcctt 60
   tttgacaaga tcggtcaact ttttgtggat gctttcacgg agttcttggt gtccattgtt 120
   gatatcatca tatttttggc cattttgttt ggcttcacca ttgccggctg gctggtggtc 180
   ttttgcatca gattggtttg ctccgcgata ctccgtgcgc gccctgccat tcaccctgag 240
   caattacaga agatcctatg aggcctttct ttctcagtgc caggtggaca ttcccgcctg 300
   gggaacaaga catcctttag ggatgctttg gcaccacaag gtgtcaaccc tgattgatga 360
   aatggtgtcg cgtcgaatgt accgcatcat ggaaaaagca ggacaggctg cctggaaaca 420
   ggtggtgagt gaggctacgc tgtctcgcat tagtggtttg gatgtggtgg cccattttca 480
   gcaccttgcc gccattgaag ccgagacttg taaatatttg gcctctcggt tgcccatgct 540
   acacaacctg cgtattacag ggtcaaatgt aaccatagtg cataatagta ctttgaatca 600
   ggtgtttgct attttcccaa cccccggttc tcggccaaag ctccatgatt ttcagcaatg 660
   gctaatagct gtacattcct cgatatcctc ctctgttgca gcttcttgta ctctttttgt 720
   tgtgttgtgg ttacggatgc caatgctacg ttctgttttt ggtttccgct ggttaggggc 780
   aatttttcct tcgagctcat ggtgaattac acggtgtgcc caccttgcct cacccggcaa 840
   gcagccgcac agatctacga acccaacagg tctctttggt gcaggatcgg gaatgatcga 900
   tgtggtgagg acgatcacga cgaactagga tttacagtac cgcctggcct ctccaaagaa 960
   gtccatttga ccagtgttta cgcctggttg gcgtttctgt ccttcagtaa cacggcccag 1020
   tttcatcccg agatattcgg aatagggaat gtgagtaagg tctatgttga catcaatcat 1080
   caactcattt gtgctgttca tgacgggcag aacaccacct tgcctcgcca tgacaacatt 1140
   tctgccgtgt ttcagaccta ttaccaacac caagtcgatg gtggcaactg gtttcaccta 1200
   gaatggctgc gtcccttctt ttcctcttgg ttggttttga atgtctcctg gtttctcagg 1260
   cgttcgcctg caagccatgt ttcagttcga gtctttcaga catcaagacc aacaccaccg 1320
   cggcagcaaa tttcgctgtc ctccaggaca tcggctgcct taggcatggc aactcgacca 1380
   ctgaggcgtt tcgcaaaatc cctcagtgcc gcacggcgat agggacaccc gtgtatatca 1440
   ctatcacagc caatgtaaca gatgagaact atttgcattc ttctgatctt ctcatgcttt 1500
   cctcttgcct tttctacgct tctgagatga gtgaaaaggg gtttaaggtg gtgtttggca 1560
   atgtgtcagg caccgtggct gtgtgcatca attttaccag ctatgtccaa cacgtcaagg 1620
   agtttaccca acgctcctta gtggtcgacc atgtgcggct gctccatttc atgacacctg 1680
   aaactatgag gtgggcaact gttttagcct gtcttttcgc cattctgttg gcaatttgaa 1740
   tgtttaagta tgttggggaa atgcttgacc gcgggctgtt gctcgcgatc gctttttttg 1800
   tggtgtatcg tgccgttctg tcttgctgcg ctcgtcagcg ccaacaacag cagctcccat 1860
   ttacagttga tttataacct gacgctatgt gagctgaatg gcacagactg gctggctaat 1920
   aaatttgatt gggcagtgga gagttttgtc atctttcccg tgttgactca cattgtttcc 1980
   tatggtgcac tcaccaccag ccatttcctt gacacagtcg gtctggttac tgtgtctacc 2040
   gccgggtttc atcacgggcg gtatgttctg agtagcatct acgcggtctg tgccctggct 2100
   gcgtttattt gcttcgtcat taggtttgcg aagaactgca tgtcctggcg ctactcttgt 2160
   accagatata ccaacttcct tctggacact aagggcagcc tctatcgttg gcggtcacct 2220
   gtcatcatag agaaaggggg taaggttgag gtcgaaggtc atctgatcga cctaaaaaaa 2280
   gttgtgcttg atggttccgc ggcaacccct ttaaccagag tttcagcgga acaatggggt 2340
   cgtccctag                                                         2349
<210> 3
   <211> 2352
   <212> DNA
   <213> Porcine reproductive and respiratory syndrome virus
<400> 3
   cctatcattg aaccaacttt aggcctgaat tgaaatgaaa tggggtctat gcaaagcctt 60
   tttgacaaaa ttggccaact tttcgtggat gctttcacgg agttcttggt gtccattgtt 120
   gatatcatta tatttttggc cattttgttt ggcttcacca tcgccggttg gctggtggtc 180
   ttttgcatca gattggtttg ctccgcgata ctccgtgcgc gccctgccat tcactctgag 240
   caattacaga agatcctatg aggcctttct ttctcagtgc caggtggaca ttcccacctg 300
   gggaattaaa catcctttgg ggatgctttg gcaccataag gtgtcaaccc tgattgatga 360
   aatggtgtcg cgtcgaatgt accgcatcat ggaaaaagca ggacaggctg cctggaaaca 420
   ggtggtgagc gaggctacgc tgtctcgcat tagtagtttg gatgtggtgg ctcactttca 480
   gcatcttgcc gccattgaag ccgagacctg taaatatttg gcctctcggc tgcccatgct 540
   acacaacctg cgcatgacag ggtcaaatgt aaccatagtg tataatagta ctttgaatca 600
   ggtgcttgct attttcccaa cccctggttc ccggccaaag cttcatgatt ttcagcaatg 660
   gctaatagct gtacattcct ctatattttc ctctgttgca gcttcttgta ctctttttgt 720
   tgtgctgtgg ttgcgggttc caatgctacg tattgctttt ggtttccgct ggttaggggc 780
   aatttttctt tcgaactcac agtgaactac acggtgtgtc caccttgcct cacccggcaa 840
   gcagccacag aggcctacga acctggcagg tctctttggt gcaggatagg gtatgatcgc 900
   tgtggggagg acgatcatga tgaactaggg tttgtggtgc cgtctggcct ctccagcgaa 960
   ggccacttga ccagtgttta cgcctggttg gcgttcctgt ctttcagtta cacagcccag 1020
   ttccatcctg agatattcgg gatagggaat gtgagtcaag tttatgttga catcaggcat 1080
   caattcattt gcgccgttca cgacgggcag aacgccactt tgcctcgcca tgacaatatt 1140
   tcagccgtgt tccagactta ttaccaacat caagtcgacg gcggcaattg gtttcaccta 1200
   gaatggctgc gtcccttctt ttcctcttgg ttggttttaa atgtctcttg gtttctcagg 1260
   cgttcgcctg caagccatgt ttcagttcga gtcttgcaga cattaagacc aacaccaccg 1320
   cagcggcagg ctttgctgtc ctccaagaca tcagttgcct taggtatcgc aactcggcct 1380
   ctgaggcgtt tcgcaaaatc cctcagtgtc gtacggcgat agggacaccc atgtatatta 1440
   ctgtcacagc caatgtaacc gatgagaatt atttgcattc ctctgacctt ctcatgcttt 1500
   cttcttgcct tttctacgct tctgagatga gtgaaaaggg atttaaagtg gtatttggca 1560
   atgtgtcagg catcgtggct gtgtgcgtca actttaccag ctacgtccaa catgtcaagg 1620
   aatttaccca acgctccttg gtagtcgacc atgtgcggct gctccatttc atgacacctg 1680
   agaccatgag gtgggcaact gttttagcct gtctttttgc cattctgttg gccatttgaa 1740
   tgtttaagta tgttggggaa atgcttgacc gcgggctatt gctcgtcatt gctttttttg 1800
   tggtgtatcg tgccgtcttg gtttgttgcg ctcgccagcg ccaacagcag caacagctct 1860
   catttacagt tgatttataa cttgacgcta tgtgagctga atggcacaga ttggttagct 1920
   ggtgaatttg actgggcagt ggagtgtttt gtcatttttc ctgtgttgac tcacattgtc 1980
   tcctatggtg ccctcaccac cagccatttc cttgacacag tcggtctggt cactgtgtct 2040
   accgccggct tttcccacgg gcggtatgtt ctgagtagca tctacgcggt ctgtgccctg 2100
   gctgcgttga tttgcttcgt cattaggttt acgaagaatt gcatgtcctg gcgctactca 2160
   tgtaccagat ataccaactt tcttctggac actaagggca gactctatcg ttggcggtcg 2220
   cctgtcatca tagagaaaag gggtaaagtt gaggtcgaag gtcatctgat cgacctcaag 2280
   agagttgtgc ttgatggttc cgcggcaacc cctataacca aagtttcagc ggagcaatgg 2340
   ggtcgtcctt ag                                                    2352
<210> 4
   <211> 2351
   <212> DNA
   <213> Porcine reproductive and respiratory syndrome virus
<400> 4
   cctgtcattg aaccaacttt aggcctgaat tgaaatgaaa tgggggccat gcaaagcctt 60
   tttgacaaaa ttggccaact ttttgtggat gctttcacgg agttcttggt gtccattgtt 120
   gatatcatta tatttttggc cattttgttt ggcttcacca tcgccggttg gctggtggtc 180
   ttttgcatca gattggtttg ctccgcgata ctccgtgcgc gccctgccat tcactctgag 240
   caattacaga agatcttatg aggcctttct ttcccagtgc caagtggaca ttcccacctg 300
   gggaactaaa catcctttgg ggatgttgtg gcaccataag gtgtcaaccc tgattgatga 360
   aatggtgtcg cgtcgaatgt accgcatcat ggaaaaagca gggcaggctg cctggaaaca 420
   ggtggtgagc gaggctacgc tgtctcgcat tagtagtttg gatgtggtgg ctcattttca 480
   gcatcttgct gccattgaag ccgagacctg taaatatttg gcctcccggc tgcccatgct 540
   acacaacctg cgcatgacag ggtcaaatgt aaccatagtg tataatagta ctttgaatca 600
   ggtgtttgct attttcccaa cccctggttc ccggccaaag cttcatgatt ttcagcaatg 660
   gttaatagct gtacattcct ccatattttc ctctgttgca gcttcctgta ctctttttgt 720
   tgtgctgtgg ttgcgggttc caatactacg ttctgttttt ggtttccgct ggttaggggc 780
   aatttttctt tcgagctcac ggtgaattac acggtgtgtc caccttgcct cacccggcaa 840
   gcagccgcag agatctacga acccggtagg tctctttggt gcaggatagg gtatgaccga 900
   tgtggggagg acgatcatga cgagctaggg tttatggtac cacctggctt ctccagcgaa 960
   ggccacttga ctagtgttta cgcctggttg gcgtttttgt ccttcagcta cacggcccag 1020
   ttccatcccg agatattcgg gatagggaac gtgagtcgag tttatgttga catcaaacat 1080
   caactcatct gcgccgaaca tgacgggcaa aacaccacct tgcctcgtca tgacaacatt 1140
   tcagccgtgt ttcagaccta ttaccaacat caagtcgacg gtggcaattg gtttcaccta 1200
   gaatggcttc gtcccttctt ttcctcatgg ttggttttaa atgtctcttg gtttctcagg 1260
   cgttcgcctg caaaccatgt ttcagttcga gtcttgcaga tattaagacc aacaccaccg 1320
   cagcggcaag ctttgctgtc ctccaagaca tcggttgcct taggcatcgc gactcggcct 1380
   ctgaggcgat tcgcaaaatc cctcagtgcc gtacggcgat agggacaccc gtgtatatta 1440
   ccatcacagc caatgtgaac gatgagaatt atttacattc ttctgatctc ctcatgcttt 1500
   cttcttgcct tttctatgct tctgagatga gtgaaaaggg gtttaaggtg gtatttggca 1560
   atgtgtcagg catcgtggct gtgtgtgtca attttaccag ctatgtccaa catgtcaggg 1620
   agtttaccca acgctccttg gtggtcgacc atgtgcggtt gctccatttc atgacacctg 1680
   agaccatgag gtgggcaact gttttagcct gtctttttgc cattctgttg gcaatttgaa 1740
   tgtttaagca tgttggggaa atgcttgacc gcgggctgtt gctcgcgatt gctttctttg 1800
   tggtttatcg tgccgttctg ttttgctgtg ctcgccagcg ccagcaacag cagcagctcc 1860
   catctacagt tgatttataa cttgacgcta tgtgagctga atggcacaga ttggttagct 1920
   aataaatttg attgggcagt ggagagtttt gtcatctttc ccgttttgac tcacattgtc 1980
   tcctatggtg ccctcactac cagccatttc cttgacacag tcgctttagt cactgtgtct 2040
   accgccgggt ttgttcacgg gcggtatgtc ctgagtagca tctacgcggt ctgtgccctg 2100
   gctgcgttga cttgcttcat catcaggttt gcaaagaatt gcatgtcctg gcgctactcg 2160
   tgtaccagat ataccaactt tctcctggac actaagggca gactctatcg ttggcggtcg 2220
   cctgtcatca tagagaaaag gggcaaagtt gaggtcgaag gtcactgatc gacctcaaaa 2280
   gagttgtgct tgatggttcc gtggcaaccc ctataaccag agattcagcg gaacaatggg 2340
   gtcgtcctta g                                                      2351
<210> 5
   <211> 1947
   <212> DNA
   <213> Porcine reproductive and respiratory syndrome virus
<400> 5
   cctgtcattg aaccaacttt aggcctgaat tgaaatgaaa tggggtccat gcaaagcctt 60
   tttgacaaaa ttggccaact ttttgtggat gctttcacgg agttcttggt gtccattgtt 120
   gatatcatta tattcttggc cattttgttt ggcttcacca tcgccggttg gctggtggtc 180
   ttttgcatca gattggtttg ctccgcgata ctccgtacgc gccctgccat tcactctgag 240
   caattacaga agatcttatg aggcctttct ttcccagtgc caagtggaca ttcccacctg 300
   gggaactaaa catcctttgg ggatgttttg gcaccataag gtgtcaaccc tgattgatga 360
   gatggtgtcg cgtcgaatgt accgcatcat ggaaaaagca ggacaggctg cctggaaaca 420
   ggtggtgagc gaggctacgc tgtctcgcat tagtagtttg gatgtggtgg ctcattttca 480
   gcatcttgcc gccatcgaag ccgagacctg taaatatttg gcctcccggc tgcccatgct 540
   acacaacctg cgcatgacag ggtcaaatgt aaccatagtg tataatagta ctttgaatcg 600
   ggtgtttgct attttcccaa cccctggttc ccggccaaag cttcatgact ttcagcaatg 660
   gctaatagct gtgcattcct ccatattttc ctctgttgca gcttcttgta ctctctttgt  720
   tgtgctgtgg ttgcgggttc caatactacg tactgttttt ggtttccgct ggttaggggc  780
   aatttttctt tcgaactcat agtgaattac acggtgtgcc caccttgcct cacccggcaa  840
   gcagccgcag aggcctacga acccggtagg tctctttggt gcaggatagg gtacgatcga  900
   tgtggagagg acgaccatga cgagctaggg tttatgatac cgtctggcct ctccagcgaa  960
   ggccacttga ccagtctgag gcgattcgca aaatccctca gtgccgtacg gcgataggga 1020
   cacctatgta tattaccatc acagccaatg tgacagatga aaattattta cattcttctg 1080
   atctcctcat gctctcttct tgccttttct atgcttctga gatgagtgaa aagggatttg 1140
   aggtggtttt tggcaatgtg tcaggcatcg tggctgtgtg tgtcaatttt accagctacg 1200
   ttcaacatgt cagggagttt acccaacgct ccttgatggt cgaccatgtg cggctgctcc 1260
   atttcatgac acctgagacc atgaggtggg caaccgtttt agcctgtctt tttgctattc 1320
   tgttggcaat ttgaatgttt aagtatgttg gggaaatgct tgaccgtggg ctgttgctcg 1380
   cgattgcttt ctttgtggtg tatcgtgccg ttctgtttta ctgtgctcgc cgacgcccac 1440
   agcaacagca gctctcatct gcaattgatt tacaacttga cgctatgtga gctgaatggc 1500
   acagattggc tagctgatag atttgattgg gcagtggaga gctttgtcat ctttcctgtt 1560
   ttgactcaca ttgtctccta tggcgccctc accaccagcc atttccttga cacaattgct 1620
   ttagtcactg tgtctaccgc cgggtttgtt cacgggcggt atgtcctaag tagcatctac 1680
   gcggtctgtg ccctggctgc gttgacttgc ttcgtcatta ggtttgtgaa gaattgcatg 1740
   tcctggcgct actcatgtac tagatatacc aactttcttc tggatactaa gggcagactc 1800
   tatcgttggc ggtcgcctgt catcatagag aagaggggca aagttgaggt cgaaggtcat 1860
   ctgatcgatc tcaaaagagt tgtgcttgat ggttccgtgg caacccctat aaccagagtt 1920
   tcagcggaac aatggggtcg tccttag                                    1947
<210> 6
   <211> 2352
   <212> DNA
   <213> Porcine reproductive and respiratory syndrome virus
<400> 6
   cctgtcattg aaccaacttt aggcctgaat tgagatgaaa tggggtctat gcaaagcctt 60
   tttgacaaaa ttggccaact ttttgtggat gctttcacgg agttcttggt gtccattgtt 120
   gatatcatta tatttttggc cattttgttt ggcttcacca tcgcaggttg gctggtggtc 180
   ttttgcatca gattggtttg ctccgcgata ctccgtgcgc gccctgccat tcactctgag 240
   caattacaga agatcctatg aggcctttct ctctcagtgc caggtggaca ttcccacctg 300
   gggaactaaa catcctttgg ggatgctttg gcaccataag gtgtcaaccc tgattgatga 360
   aatggtgtcg cgtcgaatgt accgcatcat ggaaaaagca ggacaggctg cctggaaaca 420
   ggtagtgagc gaggctacgc tgtctcgcat tagtagtttg gatgtggtgg ctcattttca 480
   gcatcttgcc gccattgaag ccgagacctg taaatatctg gcctctcggc tgcccatgct 540
   acaccacctg cgcatgacag ggtcaaatgt aaccatagtg tataatagta ctttgaatca 600
   ggtgtttgct gttttcccaa cccctggttc ccggccaaag cttcatgatt tccagcaatg 660
   gctaatagct gtacattcct ctatattttc ctctgttgca gcttcttgta ctctttttgt 720
   tgtgctgtgg ttgcgggttc caatgctacg tactgttttt ggtttccgct ggttaggggc 780
   aatttttctt tcgaactcac ggtgaattac acggtgtgcc cgccttgcct cacccggcaa 840
   gcagccgcag aggcctacga acccggcagg tccctttggt gcaggatagg gcatgatcga 900
   tgtggggagg acgatcatga tgaactaggg tttgtggtgc cgtctggcct ctccagcgaa 960
   ggccacttga ccagtgctta cgcctggttg gcgtccctgt ccttcagcta tacggcccag 1020
   ttccatcccg agatattcgg gatagggaat gtgagtcgag tctatgttga catcaagcac 1080
   caattcattt gcgctgttca tgatgggcag aacaccacct tgccccacca tgacaacatt 1140
   tcagccgtgc ttcagaccta ttaccagcat caggtcgacg ggggcaattg gtttcaccta 1200
   gaatgggtgc gtcccttctt ttcctcttgg ttggttttaa atgtctcttg gtttctcagg 1260
   cgttcgcctg caagccatgt ttcagttcga gtctttcaga catcaagacc aacaccaccg 1320
   cagcggcagg ctttgctgtc ctccaagaca tcagttgcct taggcatcgc aactcggcct 1380
   ctgaggcgat tcgcaaagtc cctcagtgcc gcacggcgat agggacaccc gtgtatatca 1440
   ctgtcacagc caatgttacc gatgagaatt atttgcattc ctctgatctt ctcatgcttt 1500
   cttcttgcct tttctatgct tctgagatga gtgaaaaggg atttaaggtg gtatttggca 1560
   atgtgtcagg catcgtggca gtgtgcgtca acttcaccag ttacgtccaa catgtcaagg 1620
   aatttaccca acgttccttg gtagttgacc atgtgcggct gctccatttc atgacgcccg 1680
   agaccatgag gtgggcaact gttttagcct gtctttttac cattctgttg gcaatttgaa 1740
   tgtttaagta tgttggggaa atgcttgacc gcgggctgtt gctcgcaatt gcttttttta 1800
   tggtgtatcg tgccgtcttg ttttgttgcg ctcgtcagcg ccaacgggaa cagcggctca 1860
   aatttacagc tgatttacaa cttgacgcta tgtgagctga atggcacaga ttggctagct 1920
   aataaatttg actgggcagt ggagtgtttt gtcatttttc ctgtgttgac tcacattgtc 1980
   tcttatggtg ccctcactac tagccatttc cttgacacag tcggtctggt cactgtgtct 2040
   accgctgggt ttgttcacgg gcggtatgtt ctgagtagca tgtacgcggt ctgtgccctg 2100
   gctgcgttga tttgcttcgt cattaggctt gcgaagaatt gcatgtcctg gcgctactca 2160
   tgtaccagat ataccaactt tcttctggac actaagggca gactctatcg ttggcggtcg 2220
   cctgtcatca tagagaaaag gggcaaagtt gaggtcgaag gtcacctgat cgacctcaaa 2280
   agagttgtgc ttgatggttc cgcggctacc cctgtaacca gagtttcagc ggaacaatgg 2340
   agtcgtcctt ag 2352
<210> 7
   <211> 2352
   <212> DNA
   <213> Porcine reproductive and respiratory syndrome virus
<400> 7
   cccgtcattg aaccaacttt aggcctgaat tgaaatgaaa tggggtccgt gcaaagcctt 60
   tttgacaaaa ttggccaact ttttgtggat gctttcacgg agttcctggt gtccattgtt 120
   gatatcatca tatttttggc cattttgttt ggcttcacca tcgccggttg gctggtggtc 180
   ttttgcatca gattggtttg ctccgcgata ctccgtacgc gccctgccat tcactctgag 240
   caattacaga agatcttatg aggccttttt atcccagtgc caagtggaca ttcccacctg 300
   gggaactaaa catcctttgg ggatgttttg gcaccataag gtgtcaaccc tgattgatga 360
   aatggtgtcg cgtcgcatgt accgcatcat ggaaaaagca gggcaggctg cctggaaaca 420
   ggtggtgagc gaggctacgc tgtcccgcat tagtagtttg gatgtggtgg ctcattttca 480
   gcatcttgcc gccattgaag ccgagacttg taaatatttg gcctcccggc tgcccatgct 540
   acataacctg cgcataacag ggtcaaatgt aaccatagtg tataatagta cttcggagca 600
   ggtgtttgct attttcccaa cccctggttc ccggccaaag cttcatgatt ttcagcaatg 660
   gttaatagct gtacattcct ccatattttc ctctgttgca gcttcttgta ctctttttgt 720
   tgtgctgtgg ctgcgggttc caatgctacg tactgttttt ggtttccgct ggttaggggg 780
   aatttttcct tcgaactcat ggtgaattac acggtgtgtc caccttgcct cacccggcaa 840
   gcagccgcag aggtctacga acccggtagg tctctttggt gcaggatagg gtatgaccga 900
   tgtggggagg acgatcatga cgagctaggg tttatgatac cgcctggcct ctccagcgaa 960
   ggccacttga ctagtgttta cgcctggttg gcgtttttgt ccttcagcta cacggcccag 1020
   ttccatcccg agatattcgg gatagggaat gtgagtcgag tttatgttga catcaaacat 1080
   caactcattt gcgccgaaca tgacggacag aacgccacct tgcctcgtca tgacaatatt 1140
   tcagccgtgt ttcagaccta ttaccaacat caagtcgatg gcggcaattg gtttcaccta 1200
   gaatggcttc gtcccttctt ttcctcatgg ttggttttaa atgtctcttg gtatctcagg 1260
   cgttcgcctg caaaccatgc ttcagttcga gtcttgcaga tattaagacc aacactaccg 1320
   cagcggcaag ctttgctgtc ctccaagaca tcagttgcct taggcatcgc aactcggcct 1380
   ctgaggcgat tcgcaaaatc cctcagtgcc gtacggcgat agggacaccc gtgtatatta 1440
   ccatcacagc caatgtgaca gatgagaatt atttacattc ttctgatctc ctcatgcttt 1500
   cttcttgcct tttctacgct tctgagatga gtgaaaaagg attcaaggtg gtatttggca 1560
   atgtgtcagg catcgtggct gtgtgtgtca attttaccag ctacgtccaa catgtcaggg 1620
   agtttaccca acgctccctg gtggtcgacc atgtgcggtt gctccatttc atgacacctg 1680
   aaaccatgag gtgggcaact gttttagcct gtctttttgc cattctgctg gcaatttgaa 1740
   tgtttaagta tgttggggaa atgcttgacc gcgggctgtt gctcgcgatt gctttctttg 1800
   tggtgtatcg tgccgttctg ttttgctgtg ctcgccaacg ccagcgccaa cagcagctcc 1860
   catctacagc tgatttacaa cttgacgcta tgtgagctga atggcacaga ttggctagct 1920
   gataaatttg attgggcagt ggagagtttt gtcatctttc ccgttttgac tcacattgtc 1980
   tcctatggtg ccctcactac tagccatctc cttgacacag tcgccttagt cactgtgtct 2040
   accgccgggt ttgttcacgg gcggtatgtc ctaagtagca tctacgcggt ctgtgccctg 2100
   gctgcgttag cttgcttcgt cattaggttt gcaaagaatt gcatgtcctg gcgctattcg 2160
   tgtaccagat ataccaactt tcttctggac actaagggca gactctatcg ttggcattcg 2220
   cctgtcatca tagagaaaag gggcaaagtt gaggtcgaag gtcatctgat cgacctcaaa 2280
   agagttgtgc ttgacggttc cgtggcaacc cctataacca gagtttcagc ggaacaatgg 2340
   ggtcgtcctt ag                                                     2352
<210> 8
<211> 256
   <212> PRT
   <213> Porcine reproductive and respiratory syndrome virus
<400> 8
   Met Lys Trp Gly Leu Cys Lys Ala Phe Leu Thr Lys Leu Ala Asn Phe
   1 5 10 15
   Leu Trp Met Leu Ser Arg Ser Ser Trp Cys Pro Leu Leu Ile Ser Leu
   20 25 30
   Tyr Phe Trp Pro Phe Cys Leu Ala Ser Pro Ser Gln Val Gly Trp Trp
   35 40 45
   Ser Phe Ala Ser Asp Trp Phe Ala Pro Arg Tyr Ser Val Arg Ala Leu
   50 55 60
   Pro Phe Thr Leu Ser Asn Tyr Arg Arg Ser Tyr Glu Ala Phe Leu Ser
   65 70 75 80
   Gln Cys Gln Val Asp Ile Pro Thr Trp Gly Thr Lys His Pro Leu Gly
   85 90 95
   Met Leu Trp His His Lys Val Ser Thr Leu Ile Asp Glu Met Val Ser
   100 105 110
   Arg Arg Met Tyr Arg Ile Met Glu Lys Ala Gly Gln Ala Ala Trp Lys
   115 120 125
   Gln Val Val Ser Glu Ala Thr Leu Ser Arg Ile Ser Ser Leu Asp Val
   130 135 140
   Val Ala His Phe Gln His Leu Ala Ala Ile Glu Ala Glu Thr Cys Lys
   145 150 155 160
   Tyr Leu Ala Ser Arg Leu Pro Met Leu His Met Leu Arg Met Thr Gly
   165 170 175
   Ser Asn Val Thr Ile Val Tyr Asn Ser Thr Leu Asn Gln Val Phe Ala
   180 185 190
   Val Phe Pro Thr Pro Gly Ser Arg Pro Lys Leu His Asp Phe Gln Gln
   195 200 205
   Trp Leu Ile Ala Val His Ser Ser Ile Phe Ser Ser Val Ala Ala Ser
   210 215 220
   Cys Thr Leu Phe Val Val Leu Trp Leu Arg Val Pro Met Leu Arg Thr
   225 230 235 240
   Val Phe Gly Phe Arg Trp Leu Gly Ala Ile Phe Leu Ser Asn Ser Arg
   245 250 255
<210> 9
   <211> 256
   <212> PRT.
   <213> Porcine reproductive and respiratory syndrome virus
<400> 9
   Met Lys Trp Gly Pro Cys Lys Ala Phe Leu Thr Lys Leu Ala Asn Phe
   1 5 10 15
   Leu Trp Met Leu Ser Arg Ser Ser Trp Cys Pro Leu Leu Ile Ser Leu
   20 25 30
   Tyr Phe Trp Pro Phe Cys Leu Ala Ser Pro Ser Pro Val Gly Trp Trp
   35 40 45
   Ser Phe Ala Ser Asp Trp Phe Ala Pro Arg Tyr Ser Val Arg Ala Leu
   50 55 60
   Pro Phe Thr Leu Ser Asn Tyr Arg Arg Ser Tyr Glu Ala Phe Leu Ser
   65 70 75 80
   Gln Cys Gln Val Asp Ile Pro Thr Trp Gly Thr Lys His Pro Leu Gly
   85 90 95
   Met Leu Trp His His Lys Val Ser Thr Leu Ile Asp Glu Met Val Ser
   100 105 110
   Arg Arg Met Tyr Arg Ile Met Glu Lys Ala Gly Gln Ala Ala Trp Lys
   115 120 125
   Gln Val Val Ser Glu Ala Thr Leu Ser Arg Ile Ser Ser Leu Asp Val
   130 135 140
   Val Ala His Phe Gln His Leu Ala Ala Ile Glu Ala Glu Thr Cys Lys
   145 150 155 160
   Tyr Leu Ala Ser Arg Leu Pro Met Leu His Asn Leu Arg Met Thr Gly
   165 170 175
   Ser Asn Val Thr Ile Val Tyr Asn Ser Thr Leu Asn Gln Val Phe Ala
   180 185 190
   Ile Phe Pro Thr Pro Gly Ser Arg Pro Lys Leu His Asp Phe Gln Gln
   195 200 205
   Trp Leu Ile Ala Val His Ser Ser Ile Phe Ser Ser Val Ala Ala Ser
   210 215 220
   Cys Thr Leu Phe Val Val Leu Trp Leu Arg Val Pro Ile Leu Arg Ser
   225 230 235 240
   Val Phe Gly Phe Arg Trp Leu Gly Ala Ile Phe Leu Ser Ser Ser Arg
   245 250 255
<210> 10
   <211> 255
   <212> PRT
   <213> Porcine reproductive and respiratory syndrome virus
<400> 10
   Met Lys Trp Gly Pro Cys Lys Ala Phe Leu Thr Lys Leu Ala Asn Phe
   1 5 10 15
   Leu Trp Met Leu Ser Arg Ser Ser Trp Cys Pro Leu Leu Ile Ser Leu
   20 25 30
   Ser Phe Trp Pro Phe Cys Leu Ala Ser Pro Ser Pro Val Gly Trp Trp
   35 40 45
   Ser Phe Ala Ser Asp Trp Phe Ala Pro Arg Tyr Ser Val Arg Ala Leu
   50 55 60
   Pro Phe Thr Leu Ser Asn Tyr Arg Arg Ser Tyr Glu Ala Phe Leu Ser
   65 70 75 80
   Gln Cys Gln Val Asp Ile Pro Thr Trp Gly Thr Lys His Pro Leu Gly
   85 90 95
   Met Phe Trp His His Lys Val Ser Thr Leu Ile Asp Glu Met Val Ser
   100 105 110
   Arg Arg Met Tyr Arg Ile Met Glu Lys Ala Gly Gln Ala Ala Trp Lys
   115 120 125
   Gln Val Val Ser Glu Ala Thr Leu Ser Arg Ile Ser Ser Leu Asp Val
   130 135 140
   Val Ala His Phe Gln His Leu Ala Ala Ile Glu Ala Glu Thr Cys Lys
   145 150 155 160
   Tyr Leu Ala Ser Arg Leu Pro Met Leu His Asn Leu Arg Met Thr Gly
   165 170 175
   Ser Asn Val Thr Ile Val Tyr Asn Ser Thr Leu Asn Arg Val Phe Ala
   180 185 190
   Ile Phe Pro Thr Pro Gly Ser Arg Pro Lys Leu His Asp Phe Gln Gln
   195 200 205
   Trp Leu Ile Ala Val His Ser Ser Ile Phe Ser Ser Val Ala Ala Ser
   210 215 220
   Cys Thr Leu Phe Val Val Leu Trp Leu Arg Val Pro Ile Leu Arg Thr
   225 230 235 240
   Val Phe Gly Phe Arg Trp Leu Gly Ala Ile Phe Leu Ser Asn Ser
   245 250 255
<210> 11
   <211> 256
   <212> PRT
   <213> Porcine reproductive and respiratory syndrome virus
<400> 11
   Met Lys Trp Gly Leu Cys Lys Ala Phe Leu Thr Lys Leu Ala Asn Phe
   1 5 10 15
   Ser Trp Met Leu Ser Arg Ser Ser Trp Cys Pro Leu Leu Ile Ser Leu
   20 25 30
   Tyr Phe Trp Pro Phe Cys Leu Ala Ser Pro Ser Pro Val Gly Trp Trp
   35 40 45
   Ser Phe Ala Ser Asp Trp Phe Ala Pro Arg Tyr Ser Val Arg Ala Leu
   50 55 60
   Pro Phe Thr Leu Ser Asn Tyr Arg Arg Ser Tyr Glu Ala Phe Leu Ser
   65 70 75 80
   Gln Cys Gln Val Asp Ile Pro Thr Trp Gly Ile Lys His Pro Leu Gly
   85 90 95
   Met Phe Trp His His Lys Val Ser Thr Leu Ile Asp Glu Met Val Ser
   100 105 110
   Arg Arg Met Tyr Arg Ile Met Glu Lys Ala Gly Gln Ala Ala Trp Lys
   115 120 125
   Gln Val Val Ser Glu Ala Thr Leu Ser Arg Ile Ser Ser Leu Asp Val
   130 135 140
   Val Ala His Phe Gln His Leu Ala Ala Ile Glu Ala Glu Thr Cys Lys
   145 150 155 160
   Tyr Leu Ala Ser Arg Leu Pro Met Leu His Asn Leu Arg Met Thr Gly
   165 170 175
   Ser Asn Val Thr Ile Val Tyr Asn Ser Thr Leu Asn Gln Val Leu Ala
   180 185 190
   Ile Phe Pro Thr Pro Gly Ser Arg Pro Lys Leu His Asp Phe Gln Gln
   195 200 205
   Trp Leu Ile Ala Val His Ser Ser Ile Phe Ser Ser Val Ala Ala Ser
   210 215 220
   Cys Thr Leu Phe Val Val Leu Trp Leu Arg Val Pro Met Leu Arg Ile
   225 230 235 240
   Ala Phe Gly Phe Arg Trp Leu Gly Ala Ile Phe Leu Ser Asn Ser Gln
   245 250 255
<210>, 12
   <211> 256
   <212> PRT
   <213> Porcine reproductive and respiratory syndrome virus
<400> 12
<210> 13
   <211> 256
   <212> PRT
   <213> Porcine reproductive and respiratory syndrome virus
<400> 13
   Met Gln Trp Gly Pro Cys Lys Ala Phe Leu Thr Arg Ser Val Asn Phe
   1 5 10 15
   Leu Trp Met Leu Ser Arg Ser Ser Trp Cys Pro Leu Leu Ile Ser Ser
   20 25 30
   Tyr Phe Trp Pro Phe Cys Leu Ala Ser Pro Leu Pro Ala Gly Trp Trp
   35 40 45
   Ser Phe Ala Ser Asp Trp Phe Ala Pro Arg Tyr Ser Val Arg Ala Leu
   50 55 60
   Pro Phe Thr Leu Ser Asn Tyr Arg Arg Ser Tyr Glu Ala Phe Leu Ser
   65 70 75 80
   Gln Cys Gln Val Asp Ile Pro Ala Trp Gly Thr Arg His Pro Leu Gly
   85 90 95
   Met Leu Trp His His Lys Val Ser Thr Leu Ile Asp Glu Met Val Ser
   100 105 110
   Arg Arg Met Tyr Arg Ile Met Glu Lys Ala Gly Gln Ala Ala Trp Lys
   115 120 125
   Gln Val Val Ser Glu Ala Thr Leu Ser Arg Ile Ser Gly Leu Asp Val
   130 135 140
   Val Ala His Phe Gln His Leu Ala Ala Ile Glu Ala Glu Thr Cys Lys
   145 150 155 160
   Tyr Leu Ala Ser Arg Leu Pro Met Leu His Asn Leu Arg Ile Thr Gly
   165 170 175
   Ser Asn Val Thr Ile Val His Asn Ser Thr Leu Asn Gln Val Phe Ala
   180 185 190
   Ile Phe Pro Thr Pro Gly Ser Arg Pro Lys Leu His Asp Phe Gln Gln
   195 200 205
   Trp Leu Ile Ala Val His Ser Ser Ile Ser Ser Ser Val Ala Ala Ser
   210 215 220
   Cys Thr Leu Phe Val Val Leu Trp Leu Arg Met Pro Met Leu Arg Ser
   225 230 235 240
   Val Phe Gly Phe Arg Trp Leu Gly Ala Ile Phe Pro Ser Ser Ser Trp
   245 250 255
<210> 14
   <211> 256
   <212> PRT
   <213> Porcine reproductive and respiratory syndrome virus
<400> 14
   Met Lys Trp Gly Pro Cys Lys Ala Phe Leu Thr Lys Leu Ala Asn Phe
   1 5 10 15
   Leu Trp Met Leu Ser Arg Ser Ser Trp Cys Pro Leu Leu Ile Ser Leu
   20 25 30
   Tyr Phe Trp Pro Phe Cys Leu Ala Ser Pro Ser Pro Val Gly Trp Trp
   35 40 45
   Ser Phe Ala Ser Asp Trp Phe Ala Pro Arg Tyr Ser Val Arg Ala Leu
   50 55 60
   Pro Phe Thr Leu Ser Asn Tyr Arg Arg Ser Tyr Glu Ala Phe Leu Ser
   65 70 75 80
   Gln Cys Gln Val Asp Ile Pro Thr Trp Gly Thr Lys His Pro Leu Gly
   85 90 95
   Met Leu Trp His His Lys Val Ser Thr Leu Ile Asp Glu Met Val Ser
   100 105 110
   Arg Arg Met Tyr Arg Ile Met Glu Lys Ala Gly Gln Ala Ala Trp Lys
   115 120 125
   Gln Val Val Ser Glu Ala Thr Leu Ser Arg Ile Ser Ser Leu Asp Val
   130 135 140
   Val Ala His Phe Gln His Leu Ala Ala Ile Glu Ala Glu Thr Cys Lys
   145 150 155 160
   Tyr Leu Ala Ser Arg Leu Pro Met Leu His Asn Leu Arg Met Thr Gly
   165 170 175
   Ser Asn Val Thr Ile Val Tyr Asn Ser Thr Leu Asn Gln Val Phe Ala
   180 185 190
   Ile Phe Pro Thr Pro Gly Ser Arg Pro Lys Leu His Asp Phe Gln Gln
   195 200 205
   Trp Leu Ile Ala Val His Ser Ser Ile Phe Ser Ser Val Ala Ala Ser
   210 215 220
   Cys Thr Leu Phe Val Val Leu Trp Leu Arg Val Pro Ile Leu Arg Thr
   225 230 235 240
   Val Phe Gly Phe Arg Trp Leu Gly Ala Ile Phe Leu Ser Asn Ser Gln
   245 250 255
<210> 15
   <211> 249
   <212> PRT
   <213> Porcine reproductive and respiratory syndrome virus
<400> 15
   Met Gln Trp Gly His Cys Gly Val Lys Ser Ala Ser Cys Ser Trp Thr
   1 5 10 15
   Pro Ser Leu Ser Ser Leu Leu Val Trp Leu Ile Leu Pro Phe Ser Leu
   20 25 30
   Pro Tyr Cys Leu Gly Ser Pro Ser Gln Asp Gly Tyr Trp Ser Phe Phe
   35 40 45
   Ser Glu Trp Phe Ala Pro Arg Phe Ser Val Arg Ala Leu Pro Phe Thr
   50 55 60
   Leu Pro Asn Tyr Arg Arg Ser Tyr Glu Gly Leu Leu Pro Asn Cys Arg
   65 70 75 80
   Pro Asp Val Pro Gln Phe Ala Val Lys His Pro Leu Gly Met Phe Trp
   85 90 95
   His Met Arg Val Ser His Leu Ile Asp Glu Met Val Ser Arg Arg Ile
   100 105 110
   Tyr Gln Thr Met Glu His Ser Gly Gln Ala Ala Trp Lys Gln Val Val
   115 120 125
   Gly Glu Ala Thr Leu Thr Lys Leu Ser Gly Leu Asp Ile Val Thr His
   130 135 140
   Phe Gln His Leu Ala Ala Val Glu Ala Asp Ser Cys Arg Phe Leu Ser
   145 150 155 160
   Ser Arg Leu Val Met Leu Lys Asn Leu Ala Val Gly Asn Val Ser Leu
   165 170 175
   Gln Tyr Asn Thr Thr Leu Asp Arg Val Glu Leu Ile Phe Pro Thr Pro
   180 185 190
   Gly Thr Arg Pro Lys Leu Thr Asp Phe Arg Gln Trp Leu Ile Ser Val
   195 200 205
   His Ala Ser Ile Phe Ser Ser Val Ala Ser Ser Val Thr Leu Phe Ile
   210 215 220
   Val Leu Trp Leu Arg Ile Pro Ala Leu Arg Tyr Val Phe Gly Phe His
   225 230 235 240
   Trp Pro Thr Ala Thr His His Ser Ser
   245
<210> 16
   <211> 254
   <212> PRT
   <213> Porcine reproductive and respiratory syndrome virus
<400> 16
   Met Ala Asn Ser Cys Thr Phe Leu Tyr Ile Phe Leu Cys Cys Ser Phe
   1 5 10 15
   Leu Tyr Ser Phe Cys Cys Ala Val Val Ala Gly Ser Asn Ala Thr Tyr
   20 25 30
   Cys Phe Trp Phe Pro Leu Val Arg Gly Asn Phe Ser Phe Glu Leu Thr
   35 40 45
   Val Asn Tyr Thr Val Cys Pro Pro Cys Leu Thr Arg Gln Ala Ala Ala
   50 55 60
   Glu Ala Tyr Glu Pro Gly Arg Ser Leu Trp Cys Arg Ile Gly His Asp
   65 70 75 80
   Arg Cys Gly Glu Asp Asp His Asp Glu Leu Gly Phe Val Val Pro Ser
   85 90 95
   Gly Leu Ser Ser Glu Gly His Leu Thr Ser Ala Tyr Ala Trp Leu Ala
   100 105 110
   Ser Leu Ser Phe Ser Tyr Thr Thr Gln Phe His Pro Glu Ile Phe Gly
   115 120 125
   Ile Gly Asn Val Ser Arg Val Tyr Val Asp Ile Lys His Gln Phe Ile
   130 135 140
   Cys Ala Val His Asp Gly Gln Asn Thr Thr Leu Pro His His Asp Asn
   145 150 155 160
   Ile Ser Ala Val Leu Gln Thr Tyr Tyr Gln His Gln Val Asp Gly Gly
   165 170 175
   Asn Trp Phe His Leu Glu Trp Val Arg Pro Phe Phe Ser Ser Trp Leu
   180 185 190
   Val Leu Asn Val Ser Trp Phe Leu Arg Arg Ser Pro Ala Ser His Val
   195 200 205
   Ser Val Arg Val Phe Gln Thr Ser Arg Pro Thr Pro Pro Gln Arg Gln
   210 215 220
   Ala Leu Leu Ser Ser Lys Thr Ser Val Ala Leu Gly Ile Ala Thr Arg
   225 230 235 240
   Pro Leu Arg Arg Phe Ala Lys Ser Leu Ser Ala Ala Arg Arg
   245 250
<210> 17
   <211> 254
   <212> PRT
   <213> Porcine reproductive and respiratory syndrome virus
<400> 17
   Met Ala Asn Ser Cys Thr Phe Leu Tyr Ile Phe Leu Cys Cys Ser Phe
   1 5 10 15
   Leu Tyr Ser Phe Cys Cys Ala Val Val Ala Gly Ser Asn Ala Thr Tyr
   20 25 30
   Cys Phe Trp Phe Pro Leu Val Arg Gly Asn Phe Ser Phe Glu Leu Thr
   35 40 45
   Val Asn Tyr Thr Val Cys Pro Pro Cys Leu Thr Arg Gln Ala Ala Thr
   50 55 60
   Glu Ala Tyr Glu Pro Gly Arg Ser Leu Trp Cys Arg Ile Gly Tyr Asp
   65 70 75 80
   Arg Cys Gly Glu Asp Asp His Asp Glu Leu Gly Phe Val Val Pro Ser
   85 90 95
   Gly Leu Ser Ser Glu Gly His Leu Thr Ser Val Tyr Ala Trp Leu Ala
   100 105 110
   Phe Leu Ser Phe Ser Tyr Thr Ala Gln Phe His Pro Glu Ile Phe Gly
   115 120 125
   Ile Gly Asn Val Ser Gln Val Tyr Val Asp Ile Arg His Gln Phe Ile
   130 135 140
   Cys Ala Val His Asp Gly Gln Asn Ala Thr Leu Pro Arg His Asp Asn
   145 150 155 160
   Ile Ser Ala Val Phe Gln Thr Tyr Tyr Gln His Gln Val Asp Gly Gly
   165 170 175
   Asn Trp Phe His Leu Glu Trp Leu Arg Pro Phe Phe Ser Ser Trp Leu
   180 185 190
   Val Leu Asn Val Ser Trp Phe Leu Arg Arg Ser Pro Ala Ser His Val
   195 200 205
   Ser Val Arg Val Leu Gln Thr Leu Arg Pro Thr Pro Pro Gln Arg Gln
   210 215 220
   Ala Leu Leu Ser Ser Lys Thr Ser Val Ala Leu Gly Ile Ala Thr Arg
   225 230 235 240
   Pro Leu Arg Arg Phe Ala Lys Ser Leu Ser Val Val Arg Arg
   245 250
<210> 18
   <211> 254
   <212> PRT
   <213> Porcine reproductive and respiratory syndrome virus
<400> 18
   Met Ala Asn Ser Cys Ala Phe Leu His Ile Phe Leu Cys Cys Ser Phe
   1               5                   10                  15
   Leu Tyr Ser Leu Cys Cys Ala Val Val Ala Gly Ser Asn Thr Thr Tyr
               20                  25                  30
   Cys Phe Trp Phe Pro Leu Val Arg Gly Asn Phe Ser Phe Glu Leu Ile
           35                  40                  45
   Val Asn Tyr Thr Val Cys Pro Pro Cys Leu Thr Arg Gln Ala Ala Ala
       50                  55                  60
   Glu Ala Tyr Glu Pro Gly Arg Ser Leu Trp Cys Arg Ile Gly Tyr Asp
   65                  70                  75                  80
   Arg Cys Gly Glu Asp Asp His Asp Glu Leu Gly Phe Met Ile Pro Ser
                   85                  90                  95
   Gly Leu Ser Ser Glu Gly His Leu Thr Ser Val Tyr Ala Trp Leu Ala
               100                 105                 110
   Phe Leu Ser Phe Ser Tyr Thr Ala Gln Phe His Pro Glu Ile Phe Gly
           115                 120                 125
   Ile Gly Asn Val Ser Arg Val Tyr Val Asp Ile Lys His Gln Leu Ile
       130                 135                 140
   Cys Ala Glu His Asp Gly Gln Asn Thr Thr Leu Pro Arg His Asp Asn
   145                 150                 155                 160
   Ile Ser Ala Val Phe Gln Thr Tyr Tyr Gln His Gln Val Asp Gly Gly
                   165                 170                 175
   Asn Trp Phe His Leu Glu Trp Leu Arg Pro Phe Phe Ser Ser Trp Leu
               180                 185                 190
   Val Leu Asn Val Ser Trp Phe Leu Arg Arg Ser Pro Ala Asn His Val
           195                 200                 205
   Ser Val Arg Val Leu Gln Thr Leu Arg Pro Thr Pro Pro Gln Arg Gln
       210                 215                 220
   Ala Leu Leu Ser Ser Lys Thr Ser Val Ala Leu Gly Ile Ala Thr Arg
   225                 230                 235                 240
   Pro Leu Arg Arg Phe Ala Lys Ser Leu Ser Ala Val Arg Arg
                   245                 250
<210> 19
   <211> 254
   <212> PRT
   <213> Porcine reproductive and respiratory syndrome virus
<400> 19
   Met Val Asn Ser Cys Thr Phe Leu His Ile Phe Leu Cys Cys Ser Phe
   1               5                   10                  15
   Leu Tyr Ser Phe Cys Cys Ala Val Ala Ala Gly Ser Asn Ala Thr Tyr
               20                  25                  30
   Cys Phe Trp Phe Pro Leu Val Arg Gly Asn Phe Ser Phe Glu Leu Met
           35                  40                  45
   Val Asn Tyr Thr Val Cys Pro Pro Cys Leu Thr Arg Gln Ala Ala Ala
       50                  55                  60
   Glu Val Tyr Glu Pro Gly Arg Ser Leu Trp Cys Arg Ile Gly Tyr Asp
   65                  70                  75                  80
   Arg Cys Gly Glu Asp Asp His Asp Glu Leu Gly Phe Met Ile Pro Pro
                   85                  90                  95
   Gly Leu Ser Ser Glu Gly His Leu Thr Ser Val Tyr Ala Trp Leu Ala
               100                 105                 110
   Phe Leu Ser Phe Ser Tyr Thr Ala Gln Phe His Pro Glu Ile Phe Gly
           115                 120                 125
   Ile Gly Asn Val Ser Arg Val Tyr Val Asp Ile Lys His Gln Leu Ile
       130                 135                 140
   Cys Ala Glu His Asp Gly Gln Asn Ala Thr Leu Pro Arg His Asp Asn
   145                 150                 155                 160
   Ile Ser Ala Val Phe Gln Thr Tyr Tyr Gln His Gln Val Asp Gly Gly
                   165                 170                 175
   Asn Trp Phe His Leu Glu Trp Leu Arg Pro Phe Phe Ser Ser Trp Leu
               180                 185                 190
   Val Leu Asn Val Ser Trp Tyr Leu Arg Arg Ser Pro Ala Asn His Ala
           195                 200                 205
   Ser Val Arg Val Leu Gln Ile Leu Arg Pro Thr Leu Pro Gln Arg Gln
       210                 215                 220
   Ala Leu Leu Ser Ser Lys Thr Ser Val Ala Leu Gly Ile Ala Thr Arg
   225                 230                 235                 240
   Pro Leu Arg Arg Phe Ala Lys Ser Leu Ser Ala Val Arg Arg
                   245                 250
<210> 20
   <211> 254
   <212> PRT
   <213> Porcine reproductive and respiratory syndrome virus
<400> 20
   Met Val Asn Ser Cys Thr Phe Leu His Ile Phe Leu Cys Cys Ser Phe
   1               5                   10                  15
   Leu Tyr Ser Phe Cys Cys Ala Val Val Ala Gly Ser Asn Thr Thr Phe
               20                  25                  30
   Cys Phe Trp Phe Pro Leu Val Arg Gly Asn Phe Ser Phe Glu Leu Thr
           35                  40                  45
   Val Asn Tyr Thr Val Cys Pro Pro Cys Leu Thr Arg Gln Ala Ala Ala
       50                  55                  60
   Glu Ile Tyr Glu Pro Gly Arg Ser Leu Trp Cys Arg Ile Gly Tyr Asp
   65                  70                  75                  80
   Arg Cys Gly Glu Asp Asp His Asp Glu Leu Gly Phe Met Val Pro Pro
                   85                  90                  95
   Gly Phe Ser Ser Glu Gly His Leu Thr Ser Val Tyr Ala Trp Leu Ala
               100                 105                 110
   Phe Leu Ser Phe Ser Tyr Thr Ala Gln Phe His Pro Glu Ile Phe Gly
           115                 120                 125
   Ile Gly Asn Val Ser Arg Val Tyr Val Asp Ile Lys His Gln Leu Ile
       130                 135                 140
   Cys Ala Glu His Asp Gly Gln Asn Thr Thr Leu Pro Arg His Asp Asn
   145                 150                 155                 160
   Ile Ser Ala Val Phe Gln Thr Tyr Tyr Gln His Gln Val Asp Gly Gly
                   165                 170                 175
   Asn Trp Phe His Leu Glu Trp Leu Arg Pro Phe Phe Ser Ser Trp Leu
               180                 185                 190
   Val Leu Asn Val Ser Trp Phe Leu Arg Arg Ser Pro Ala Asn His Val
           195                 200                 205
   Ser Val Arg Val Leu Gln Ile Leu Arg Pro Thr Pro Pro Gln Arg Gln
       210                 215                 220
   Ala Leu Leu Ser Ser Lys Thr Ser Val Ala Leu Gly Ile Ala Thr Arg
   225                 230                 235                 240
   Pro Leu Arg Arg Phe Ala Lys Ser Leu Ser Ala Val Arg Arg
                   245                 250
<210> 21
   <211> 254
   <212> PRT
   <213> Porcine reproductive and respiratory syndrome virus
<400> 21
   Met Ala Asn Ser Cys Thr Phe Leu His Ile Leu Leu Cys Cys Ser Phe
   1 5 10 15
   Leu Tyr Ser Phe Cys Cys Val Val Val Thr Asp Ala Asn Ala Thr Phe
   20 25 30
   Cys Phe Trp Phe Pro Leu Val Arg Gly Asn Phe Ser Phe Glu Leu Met
   35 40 45
   Val Asn Tyr Thr Val Cys Pro Pro Cys Leu Thr Arg Gln Ala Ala Ala
   50 55 60
   Gln Ile Tyr Glu Pro Asn Arg Ser Leu Trp Cys Arg Ile Gly Asn Asp
   65 70 75 80
   Arg Cys Gly Glu Asp Asp His Asp Glu Leu Gly Phe Thr Val Pro Pro
   85 90 95
   Gly Leu Ser Lys Glu Val His Leu Thr Ser Val Tyr Ala Trp Leu Ala
   100 105 110
   Phe Leu Ser Phe Ser Tyr Thr Ala Gln Phe His Pro Glu Ile Phe Gly
   115 120 125
   Ile Gly Asn Val Ser Lys Val Tyr Val Asp Ile Asn His Gln Leu Ile
   130 135 140
   Cys Ala Val His Asp Gly Gln Asn Thr Thr Leu Pro Arg His Asp Asn
   145 150 155 160
   Ile Ser Ala Val Phe Gln Thr Tyr Tyr Gln His Gln Val Asp Gly Gly
   165 170 175
   Asn Trp Phe His Leu Glu Trp Leu Arg Pro Phe Phe Ser Ser Trp Leu
   180 185 190
   Val Leu Asn Val Ser Trp Phe Leu Arg Arg Ser Pro Ala Ser His Val
   195 200 205
   Ser Val Arg Val Phe Gln Thr Ser Arg Pro Thr Pro Pro Arg Gln Gln
   210 215 220
   Ile Ser Leu Ser Ser Arg Thr Ser Ala Ala Leu Gly Met Ala Thr Arg
   225 230 235 240
   Pro Leu Arg Arg Phe Ala Lys Ser Leu Ser Ala Ala Arg Arg
   245 250
<210> 22
   <211> 254
   <212> PRT
   <213> Porcine reproductive and respiratory syndrome virus
<400> 22
   Met Val Asn Ser Cys Thr Phe Leu His Ile Phe Leu Cys Cys Ser Phe
   1 5 10 15
   Leu Tyr Ser Phe Cys Cys Ala Val Val Ala Gly Ser Asn Thr Thr Tyr
   20 25 30
   Cys Phe Trp Phe Pro Leu Val Arg Gly Asn Phe Ser Phe Glu Leu Thr
   35 40 45
   Val Asn Tyr Thr Val Cys Pro Pro Cys Leu Thr Arg Gln Ala Ala Thr
   50 55 60
   Glu Ile Tyr Glu Pro Gly Arg Ser Leu Trp Cys Arg Ile Gly Tyr Asp
   65 70 75 80
   Arg Cys Gly Glu Asp Asp His Asp Glu Leu Gly Phe Met Ile Pro Pro
   85 90 95
   Gly Leu Ser Ser Glu Gly His Leu Thr Gly Val Tyr Ala Trp Leu Ala
   100 105 110
   Phe Leu Ser Phe Ser Tyr Thr Ala Gln Phe His Pro Glu Ile Phe Gly
   115 120 125
   Ile Gly Asn Val Ser Arg Val Tyr Val Asp Ile Lys His Gln Leu Ile
   130 135 140
   Cys Ala Glu His Asp Gly Gln Asn Thr Thr Leu Pro Arg His Asp Asn
   145 150 155 160
   Ile Ser Ala Val Phe Gln Thr Tyr Tyr Gln His Gln Val Asp Gly Gly
   165 170 175
   Asn Trp Phe His Leu Glu Trp Leu Arg Pro Phe Phe Ser Ser Trp Leu
   180 185 190
   Val Leu Asn Val Ser Trp Phe Leu Arg Arg Ser Pro Ala Asn His Val
   195 200 205
   Ser Val Arg Val Leu Gln Ile Leu Arg Pro Thr Pro Pro Gln Arg Gln
   210 215 220
   Ala Leu Leu Ser Ser Lys Thr Ser Val Ala Leu Gly Ile Ala Thr Arg
   225 230 235 240
   Pro Leu Arg Arg Phe Ala Lys Ser Leu Ser Ala Val Arg Arg
   245 250
<210> 23
   <211> 265
   <212> PRT
   <213> Porcine reproductive and respiratory syndrome virus
<400> 23
   Met Ala His Gln Cys Ala Arg Phe His Phe Phe Leu Cys Gly Phe Ile
   1 5 10 15
   Cys Tyr Leu Val His Ser Ala Leu Ala Ser Asn Ser Ser Ser Thr Leu
   20 25 30
   Cys Phe Trp Phe Pro Leu Ala His Gly Asn Thr Ser Phe Glu Leu Thr
   35 40 45
   Ile Asn Tyr Thr Ile Cys Met Pro Cys Ser Thr Ser Gln Ala Ala Arg
   50 55 60
   Gln Arg Leu Glu Pro Gly Arg Asn Met Trp Cys Lys Ile Gly His Asp
   65 70 75 80
   Arg Cys Glu Glu Arg Asp His Asp Glu Leu Leu Met Ser Ile Pro Ser
   85 90 95
   Gly Tyr Asp Asn Leu Lys Leu Glu Gly Tyr Tyr Ala Trp Leu Ala Phe
   100 105 110
   Leu Ser Phe Ser Tyr Ala Ala Gln Phe His Pro Glu Leu Phe Gly Ile
   115 120 125
   Gly Asn Val Ser Arg Val Phe Val Asp Lys Arg His Gln Phe Ile Cys
   130 135 140
   Ala Glu His Asp Gly His Asn Ser Thr Val Ser Thr Gly His Asn Ile
   145 150 155 160
   Ser Ala Leu Tyr Ala Ala Tyr Tyr His His Gln Ile Asp Gly Gly Asn
   165 170 175
   Trp Phe His Leu Glu Trp Leu Arg Pro Leu Phe Ser Ser Trp Leu Val
   180 185 190
   Leu Asn Ile Ser Trp Phe Leu Arg Arg Ser Pro Val Ser Pro Val Ser
   195 200 205
   Arg Arg Ile Tyr Gln Ile Leu Arg Pro Thr Arg Pro Arg Leu Pro Val
   210 215 220
   Ser Trp Ser Phe Arg Thr Ser Ile Val Ser Asp Leu Thr Gly Ser Gln
   225 230 235 240
   Gln Arg Lys Arg Lys Phe Pro Ser Glu Ser Arg Pro Asn Val Val Lys
   245 250 255
   Pro Ser Val Leu Pro Ser Thr Ser Arg
   260 265
<210> 24
   <211> 178
   <212> PRT
   <213> Porcine reproductive and respiratory syndrome virus
<400> 24
   Met Gly Ala Ser Leu Leu Phe Leu Leu Val Gly Phe Lys Cys Leu Leu
   1 5 10 15
   Val Ser Gln Ala Phe Ala Cys Lys Pro Cys Phe Ser Ser Ser Leu Ser
   20 25 30
   Asp Ile Lys Thr Asn Thr Thr Ala Ala Ala Gly Phe Ala Val Leu Gln
   35 40 45
   Asp Ile Ser Cys Leu Arg His Arg Asn Ser Ala Ser Glu Ala Ile Arg
   50 55 60
   Lys Val Pro Gln Cys Arg Thr Ala Ile Gly Thr Pro Val Tyr Ile Thr
   65 70 75 80
   Val Thr Ala Asn Val Thr Asp Glu Asn Tyr Leu His Ser Ser Asp Leu
   85 90 95
   Leu Met Leu Ser Ser Cys Leu Phe Tyr Ala Ser Glu Met Ser Glu Lys
   100 105 110
   Gly Phe Lys Val Val Phe Gly Asn Val Ser Gly Ile Val Ala Val Cys
   115 120 125
   Val Asn Phe Thr Ser Tyr Val Gln His Val Lys Glu Phe Thr Gln Arg
   130 135 140
   Ser Leu Val Val Asp His Val Arg Leu Leu His Phe Met Thr Pro Glu
   145 150 155 160
   Thr Met Arg Trp Ala Thr Val Leu Ala Cys Leu Phe Thr Ile Leu Leu
   165 170 175
   Ala Ile
<210> 25
   <211> 178
   <212> PRT
   <213> Porcine reproductive and respiratory syndrome virus
<400> 25
   Met Ala Ser Ser Leu Leu Phe Leu Val Val Gly Phe Lys Cys Leu Leu
   1 5 10 15
   Val Ser Gln Ala Phe Ala Cys Lys Pro Cys Phe Ser Ser Ser Leu Ala
   20 25 30
   Asp Ile Lys Thr Asn Thr Thr Ala Ala Ala Ser Phe Ala Val Leu Gln
   35 40 45
   Asp Ile Ser Cys Leu Arg His Arg Asp Ser Ala Ser Glu Ala Ile Arg
   50 55 60
   Lys Ile Pro Gln Cys Arg Thr Ala Ile Gly Thr Pro Val Tyr Val Thr
   65 70 75 80
   Ile Thr Ala Asn Val Thr Asp Glu Asn Tyr Leu His Ser Ser Asp Leu
   85 90 95
   Leu Met Leu Ser Ser Cys Leu Phe Tyr Ala Ser Glu Met Ser Glu Lys
   100 105 110
   Gly Phe Lys Val Val Phe Gly Asn Val Ser Gly Ile Val Ala Val Cys
   115 120 125
   Val Asn Phe Thr Ser Tyr Val Gln His Val Lys Glu Phe Thr Gln Arg
   130 135 140
   Ser Leu Val Val Asp His Val Arg Leu Leu His Phe Met Thr Pro Glu
   145 150 155 160
   Thr Met Arg Trp Ala Thr Val Leu Ala Cys Leu Phe Ala Ile Leu Leu
   165 170 175
   Ala Ile
<210> 26
   <211> 178
   <212> PRT
   <213> Porcine reproductive and respiratory syndrome virus
<400> 26
   Met Ala Ala Ser Leu Leu Phe Leu Leu Val Gly Phe Lys Cys Leu Leu
   1 5 10 15
   Val Ser Gln Ala Phe Ala Cys Lys Pro Cys Phe Ser Ser Ser Leu Ala
   20 25 30
   Asp Ile Lys Thr Asn Thr Thr Ala Ala Ala Gly Phe Ala Val Leu Gln
   35 40 45
   Asp Ile Ser Cys Leu Arg Tyr Arg Asn Ser Ala Ser Glu Ala Phe Arg
   50 55 60
   Lys Ile Pro Gln Cys Arg Thr Ala Ile Gly Thr Pro Met Tyr Ile Thr
   65 70 75 80
   Val Thr Ala Asn Val Thr Asp Glu Asn Tyr Leu His Ser Ser Asp Leu
   85 90 95
   Leu Met Leu Ser Ser Cys Leu Phe Tyr Ala Ser Glu Met Ser Glu Lys
   100 105 110
   Gly Phe Lys Val Val Phe Gly Asn Val Ser Gly Ile Val Ala Val Cys
   115 120 125
   Val Asn Phe Thr Ser Tyr Val Gln His Val Lys Glu Phe Thr Gln Arg
   130 135 140
   Ser Leu Val Val Asp His Val Arg Leu Leu His Phe Met Thr Pro Glu
   145 150 155 160
   Thr Met Arg Trp Ala Thr Val Leu Ala Cys Leu Phe Ala Ile Leu Leu
   165 170 175
   Ala Ile
<210> 27
   <211> 178
   <212> PRT
   <213> Porcine reproductive and respiratory syndrome virus
<400> 27
   Met Ala Ser Ser Leu Leu Phe Leu Met Val Gly Phe Lys Cys Leu Leu
   1 5 10 15
   Val Ser Gln Ala Phe Ala Cys Lys Pro Cys Phe Ser Ser Ser Leu Ala
   20 25 30
   Asp Ile Lys Thr Asn Thr Thr Ala Ala Ala Ser Phe Ala Val Leu Gln
   35 40 45
   Asp Ile Ser Cys Leu Arg His Arg Asn Ser Ala Ser Glu Ala Ile Arg
   50 55 60
   Lys Ile Pro Gln Cys Arg Thr Ala Ile Gly Thr Pro Val Tyr Ile Thr
   65 70 75 80
   Ile Thr Ala Asn Val Thr Asp Glu Asn Tyr Leu His Ser Ser Asp Leu
   85 90 95
   Leu Met Leu Ser Ser Cys Leu Phe Tyr Ala Ser Glu Met Ser Glu Lys
   100 105 110
   Gly Phe Lys Val Val Phe Gly Asn Val Ser Gly Ile Val Ala Val Cys
   115 120 125
   Val Asn Phe Thr Ser Tyr Val Gln His Val Arg Glu Phe Thr Gln Arg
   130 135 140
   Ser Leu Val Val Asp His Val Arg Leu Leu His Phe Met Thr Pro Glu
   145 150 155 160
   Thr Met Arg Trp Ala Thr Val Leu Ala Cys Leu Phe Ala Ile Leu Leu
   165 170 175
   Ala Ile
<210> 28
   <211> 178
   <212> PRT
   <213> Porcine reproductive and respiratory syndrome virus
<400> 28
   Met Ala Ser Ser Leu Leu Phe Leu Met Val Gly Phe Lys Cys Leu Leu
   1 5 10 15
   Val Ser Gln Ala Phe Ala Cys Lys Pro Cys Phe Ser Ser Ser Leu Ala
   20 25 30
   Asp Ile Lys Thr Asn Thr Thr Ala Ala Ala Ser Phe Ala Val Leu Gln
   35 40 45
   Asp Ile Gly Cys Leu Arg His Arg Asp Ser Ala Ser Glu Ala Ile Arg
   50 55 60
   Lys Ile Pro Gln Cys Arg Thr Ala Ile Gly Thr Pro Val Tyr Ile Thr
   65 70 75 80
   Ile Thr Ala Asn Val Thr Asp Glu Asn Tyr Leu His Ser Ser Asp Leu
   85 90 95
   Leu Met Leu Ser Ser Cys Leu Phe Tyr Ala Ser Glu Met Ser Glu Lys
   100 105 110
   Gly Phe Lys Val Val Phe Gly Asn Val Ser Gly Ile Val Ala Val Cys
   115 120 125
   Val Asn Phe Thr Ser Tyr Val Gln His Val Arg Glu Phe Thr Gln Arg
   130 135 140
   Ser Leu Val Val Asp His Val Arg Leu Leu His Phe Met Thr Pro Glu
   145 150 155 160
   Thr Met Arg Trp Ala Thr Val Leu Ala Cys Leu Phe Ala Ile Leu Leu
   165 170 175
   Ala Ile
<210> 29
   <211> 178
   <212> PRT
   <213> Porcine reproductive and respiratory syndrome virus
<400> 29
   Met Ala Ala Ser Leu Leu Phe Leu Met Val Gly Phe Lys Cys Leu Leu
   1 5 10 15
   Val Ser Gln Ala Phe Ala Cys Lys Pro Cys Phe Ser Ser Ser Leu Ala
   20 25 30
   Asp Ile Lys Thr Asn Thr Thr Ala Ala Ala Ser Phe Ala Val Leu Gln
   35 40 45
   Asp Ile Ser Cys Leu Arg His Arg Asn Ser Ala Ser Glu Ala Ile Arg
   50 55 60
   Lys Ile Pro Gln Cys Arg Thr Ala Ile Gly Thr Pro Met Tyr Ile Thr
   65 70 75 80
   Ile Thr Ala Asn Val Thr Asp Glu Asn Tyr Leu His Ser Ser Asp Leu
   85 90 95
   Leu Met Leu Ser Ser Cys Leu Phe Tyr Ala Ser Glu Met Ser Glu Lys
   100 105 110
   Gly Phe Glu Val Val Phe Gly Asn Val Ser Gly Ile Val Ala Val Cys
   115 120 125
   Val Asn Phe Thr Ser Tyr Val Gln His Val Arg Glu Phe Thr Gln Arg
   130 135 140
   Ser Leu Met Val Asp His Val Arg Leu Leu His Phe Met Thr Pro Glu
   145 150 155 160
   Thr Met Arg Trp Ala Thr Val Leu Ala Cys Leu Phe Ala Ile Leu Leu
   165 170 175
   Ala Ile
<210> 30
   <211> 178
   <212> PRT
   <213> Porcine reproductive and respiratory syndrome virus
<400> 30
   Met Ala Ala Ser Leu Leu Phe Leu Leu Val Gly Phe Glu Cys Leu Leu
   1 5 10 15
   Val Ser Gln Ala Phe Ala Cys Lys Pro Cys Phe Ser Ser Ser Leu Ser
   20 25 30
   Asp Ile Lys Thr Asn Thr Thr Ala Ala Ala Asn Phe Ala Val Leu Gln
   35 40 45
   Asp Ile Gly Cys Leu Arg His Gly Asn Ser Thr Thr Glu Ala Phe Arg
   50 55 60
   Lys Ile Pro Gln Cys Arg Thr Ala Ile Gly Thr Pro Val Tyr Ile Thr
   65 70 75 80
   Ile Thr Ala Asn Val Thr Asp Glu Asn Tyr Leu His Ser Ser Asp Leu
   85 90 95
   Leu Met Leu Ser Ser Cys Leu Phe Tyr Ala Ser Glu Met Ser Glu Lys
   100 105 110
   Gly Phe Lys Val Val Phe Gly Asn Val Ser Gly Thr Val Ala Val Cys
   115 120 125
   Ile Asn Phe Thr Ser Tyr Val Gln His Val Lys Glu Phe Thr Gln Arg
   130 135 140
   Ser Leu Val Val Asp His Val Arg Leu Leu His Phe Met Thr Pro Glu
   145 150 155 160
   Thr Met Arg Trp Ala Thr Val Leu Ala Cys Leu Phe Ala Ile Leu Leu
   165 170 175
   Ala Ile
<210> 31
   <211> 183
   <212> PRT
   <213> Porcine reproductive and respiratory syndrome virus
<400> 31
   Met Ala Ala Ala Thr Leu Phe Phe Leu Ala Gly Ala Gln His Ile Met
   1 5 10 15
   Val Ser Glu Ala Phe Ala Cys Lys Pro Cys Phe Ser Thr His Leu Ser
   20 25 30
   Asp Ile Glu Thr Asn Thr Thr Ala Ala Ala Gly Phe Met Val Leu Gln
   35 40 45
   Asp Ile Asn Cys Phe Arg Pro His Gly Val Ser Ala Ala Gln Glu Lys
   50 55 60
   Ile Ser Phe Gly Lys Ser Ser Gln Cys Arg Glu Ala Val Gly Thr Pro
   65 70 75 80
   Gln Tyr Ile Thr Ile Thr Ala Asn Val Thr Asp Glu Ser Tyr Leu Tyr
   85 90 95
   Asn Ala Asp Leu Leu Met Leu Ser Ala Cys Leu Phe Tyr Ala Ser Glu
   100 105 110
   Met Ser Glu Lys Gly Phe Lys Val Ile Phe Gly Asn Val Ser Gly Val
   115 120 125
   Val Ser Ala Cys Val Asn Phe Thr Asp Tyr Val Ala His Val Thr Gln
   130 135 140
   His Thr Gln Gln His His Leu Val Ile Asp His Ile Arg Leu Leu His
   145 150 155 160
   Phe Leu Thr Pro Ser Ala Met Arg Trp Ala Thr Thr Ile Ala Cys Leu
   165 170 175
   Phe Ala Ile Leu Leu Ala Ile
   180
<210> 32
   <211> 200
   <212> PRT
   <213> Porcine reproductive and respiratory syndrome virus
<400> 32
   Met Leu Gly Lys Cys Leu Thr Ala Gly Cys Cys Ser Gln Leu Leu Phe
   1               5                   10                  15
   Leu Trp Cys Ile Val Pro Ser Cys Phe Val Ala Leu Val Ser Ala Asn
               20                  25                  30
   Gly Asn Ser Gly Ser Asn Leu Gln Leu Ile Tyr Asn Leu Thr Leu Cys
           35                  40                  45
   Glu Leu Asn Gly Thr Asp Trp Leu Ala Asn Lys Phe Asp Trp Ala Val
       50                  55                  60
   Glu Cys Phe Val Ile Phe Pro Val Leu Thr His Ile Val Ser Tyr Gly
   65                  70                  75                  80
   Ala Leu Thr Thr Ser His Phe Leu Asp Thr Val Gly Leu Val Thr Val
                   85                  90                  95
   Ser Thr Ala Gly Phe Val His Gly Arg Tyr Val Leu Ser Ser Met Tyr
               100                 105                 110
   Ala Val Cys Ala Leu Ala Ala Leu Ile Cys Phe Val Ile Arg Leu Ala
           115                 120                 125
   Lys Asn Cys Met Ser Trp Arg Tyr Ser Cys Thr Arg Tyr Thr Asn Phe
       130                 135                 140
   Leu Leu Asp Thr Lys Gly Arg Leu Tyr Arg Trp Arg Ser Pro Val Ile
   145                 150                 155                 160
   Ile Glu Lys Arg Gly Lys Val Glu Val Glu Gly His Leu Ile Asp Leu
                   165                 170                 175
   Lys Arg Val Val Leu Asp Gly Ser Ala Ala Thr Pro Val Thr Arg Val
               180                 185                 190
   Ser Ala Glu Gln Trp Arg Ser Pro
           195                 200
<210> 33
   <211> 200
   <212> PRT
   <213> Porcine reproductive and respiratory syndrome virus
<400> 33
   Met Leu Glu Lys Cys Leu Thr Ala Gly Cys Cys Ser Arg Leu Leu Ser
    1              5                   10                  15
   Leu Trp Cys Ile Val Pro Phe Cys Phe Ala Val Leu Ala Asn Ala Ser
               20                  25                  30
   Asn Asp Ser Ser Ser His Leu Gln Leu Ile Tyr Asn Leu Thr Leu Cys
           35                  40                  45
   Glu Leu Asn Gly Thr Asp Trp Leu Ala Asn Lys Phe Asp Trp Ala Val
       50                  55                  60
   Glu Ser Phe Val Ile Phe Pro Val Leu Thr His Ile Val Ser Tyr Gly
   65                  70                  75                  80
   Ala Leu Thr Thr Ser His Phe Leu Asp Thr Val Ala Leu Val Thr Val
                   85                  90                  95
   Ser Thr Ala Gly Phe Val His Gly Arg Tyr Val Leu Ser Ser Ile Tyr
               100                 105                 110
   Ala Val Cys Ala Leu Ala Ala Leu Thr Cys Phe Val Ile Arg Phe Ala
           115                 120                 125
   Lys Asn Cys Met Ser Trp Arg Tyr Ala Cys Thr Arg Tyr Thr Asn Phe
       130                 135                 140
   Leu Leu Asp Thr Lys Gly Arg Leu Tyr Arg Trp Arg Ser Pro Val Ile
   145                 150                 155                 160
   Ile Glu Lys Arg Gly Lys Val Glu Val Glu Gly His Leu Ile Asp Leu
                   165                 170                 175
   Lys Arg Val Val Leu Asp Gly Ser Val Ala Thr Pro Ile Thr Arg Val
               180                 185                 190
   Ser Ala Glu Gln Trp Gly Arg Pro
           195                 200
<210> 34
   <211> 200
   <212> PRT
   <213> Porcine reproductive and respiratory syndrome virus
<400> 34
   Met Leu Gly Lys Cys Leu Thr Ala Gly Tyr Cys Ser Ser Leu Leu Phe
   1 5 10 15
   Leu Trp Cys Ile Val Pro Ser Trp Phe Val Ala Leu Ala Ser Ala Asn
   20 25 30
   Ser Ser Asn Ser Ser His Leu Gln Leu Ile Tyr Asn Leu Thr Leu Cys
   35 40 45
   Glu Leu Asn Gly Thr Asp Trp Leu Ala Gly Glu Phe Asp Trp Ala Val
   50 55 60
   Glu Cys Phe Val Ile Phe Pro Val Leu Thr His Ile Val Ser Tyr Gly
   65 70 75 80
   Ala Leu Thr Thr Ser His Phe Leu Asp Thr Val Gly Leu Val Thr Val
   85 90 95
   Ser Thr Ala Gly Phe Ser His Gly Arg Tyr Val Leu Ser Ser Ile Tyr
   100 105 110
   Ala Val Cys Ala Leu Ala Ala Leu Ile Cys Phe Val Ile Arg Phe Thr
   115 120 125
   Lys Asn Cys Met Ser Trp Arg Tyr Ser Cys Thr Arg Tyr Thr Asn Phe
   130 135 140
   Leu Leu Asp Thr Lys Gly Arg Leu Tyr Arg Trp Arg Ser Pro Val Ile
   145 150 155 160
   Ile Glu Lys Arg Gly Lys Val Glu Val Glu Gly His Leu Ile Asp Leu
   165 170 175
   Lys Arg Val Val Leu Asp Gly Ser Ala Ala Thr Pro Ile Thr Lys Val
   180 185 190
   Ser Ala Glu Gln Trp Gly Arg Pro
   195 200
<210> 35
   <211> 200
   <212> PRT
   <213> Porcine reproductive and respiratory syndrome virus
<400> 35
   Met Leu Gly Lys Cys Leu Thr Ala Gly Cys Cys Ser Arg Leu Leu Ser
   1 5 10 15
   Leu Trp Cys Ile Val Pro Phe Cys Phe Ala Val Leu Ala Asn Ala Ser
   20 25 30
   Ala Asn Ser Ser Ser His Leu Gln Leu Ile Tyr Asn Leu Thr Leu Cys
   35 40 45
   Glu Leu Asn Gly Thr Asp Trp Leu Ala Asp Lys Phe Asp Trp Ala Val
   50 55 60
   Glu Ser Phe Val Ile Phe Pro Val Leu Thr His Ile Val Ser Tyr Gly
   65 70 75 80
   Ala Leu Thr Thr Ser His Leu Leu Asp Thr Val Ala Leu Val Thr Val
   85 90 95
   Ser Thr Ala Gly Phe Val His Gly Arg Tyr Val Leu Ser Ser Ile Tyr
   100 105 110
   Ala Val Cys Ala Leu Ala Ala Leu Ala Cys Phe Val Ile Arg Phe Ala
   115 120 125
   Lys Asn Cys Met Ser Trp Arg Tyr Ser Cys Thr Arg Tyr Thr Asn Phe
   130 135 140
   Leu Leu Asp Thr Lys Gly Arg Leu Tyr Arg Trp His Ser Pro Val Ile
   145 150 155 160
   Ile Glu Lys Arg Gly Lys Val Glu Val Glu Gly His Leu Ile Asp Leu
   165 170 175
   Lys Arg Val Val Leu Asp Gly Ser Val Ala Thr Pro Ile Thr Arg Val
   180 185 190
   Ser Ala Glu Gln Trp Gly Arg Pro
   195 200
<210> 36
   <211> 200
   <212> PRT
   <213> Porcine reproductive and respiratory syndrome virus
<400> 36
   Met Leu Gly Lys Cys Leu Thr Val Gly Cys Cys Ser Arg Leu Leu Ser
   1 5 10 15
   Leu Trp Cys Ile Val Pro Phe Cys Phe Thr Val Leu Ala Asp Ala His
   20 25 30
   Ser Asn Ser Ser Ser His Leu Gln Leu Ile Tyr Asn Leu Thr Leu Cys
   35 40 45
   Glu Leu Asn Gly Thr Asp Trp Leu Ala Asp Arg Phe Asp Trp Ala Val
   50 55 60
   Glu Ser Phe Val Ile Phe Pro Val Leu Thr His Ile Val Ser Tyr Gly
   65 70 75 80
   Ala Leu Thr Thr Ser His Phe Leu Asp Thr Ile Ala Leu Val Thr Val
   85 90 95
   Ser Thr Ala Gly Phe Val His Gly Arg Tyr Val Leu Ser Ser Ile Tyr
   100 105 110
   Ala Val Cys Ala Leu Ala Ala Leu Thr Cys Phe Val Ile Arg Phe Val
   115 120 125
   Lys Asn Cys Met Ser Trp Arg Tyr Ser Cys Thr Arg Tyr Thr Asn Phe
   130 135 140
   Leu Leu Asp Thr Lys Gly Arg Leu Tyr Arg Trp Arg Ser Pro Val Ile
   145 150 155 160
   Ile Glu Lys Arg Gly Lys Val Glu Val Glu Gly His Leu Ile Asp Leu
   165 170 175
   Lys Arg Val Val Leu Asp Gly Ser Val Ala Thr Pro Ile Thr Arg Val
   180 185 190
   Ser Ala Glu Gln Trp Gly Arg Pro
   195 200
<210> 37
   <211> 199
   <212> PRT
   <213> Porcine reproductive and respiratory syndrome virus
<400> 37
   Met Leu Gly Lys Cys Leu Thr Ala Gly Cys Cys Ser Arg Leu Leu Ser
   1 5 10 15
   Leu Trp Phe Ile Val Pro Phe Cys Phe Ala Val Leu Ala Ser Ala Ser
   20 25 30
   Asn Ser Ser Ser Ser His Leu Gln Leu Ile Tyr Asn Leu Thr Leu Cys
   35 40 45
   Glu Leu Asn Gly Thr Asp Trp Leu Ala Asn Lys Phe Asp Trp Ala Val
   50 55 60
   Glu Ser Phe Val Ile Phe Pro Val Leu Thr His Ile Val Ser Tyr Gly
   65 70 75 80
   Ala Leu Thr Thr Ser His Phe Leu Asp Thr Val Ala Leu Val Thr Val
   85 90 95
   Ser Thr Ala Gly Phe Val His Gly Arg Tyr Val Leu Ser Ser Ile Tyr
   100 105 110
   Ala Val Cys Ala Leu Ala Ala Leu Thr Cys Phe Ile Ile Arg Phe Ala
   115 120 125
   Lys Asn Cys Met Ser Trp Arg Tyr Ser Cys Thr Arg Tyr Thr Asn Phe
   130 135 140
   Leu Leu Asp Thr Lys Gly Arg Leu Tyr Arg Trp Arg Ser Pro Val Ile
   145 150 155 160
   Ile Glu Lys Arg Gly Lys Val Glu Val Glu Gly His Leu Ile Asp Leu
   165 170 175
   Lys Arg Val Val Leu Asp Gly Ser Val Ala Thr Pro Ile Thr Arg Val
   180 185 190
   Ser Ala Glu Gln Gly Arg Pro
   195
<210> 38
   <211> 199
   <212> PRT
   <213> Porcine reproductive and respiratory syndrome virus
<400> 38
   Met Leu Gly Lys Cys Leu Thr Ala Gly Cys Cys Ser Arg Ser Leu Phe
   1               5                   10                  15
   Leu Trp Cys Ile Val Pro Phe Cys Leu Ala Ala Leu Val Ser Ala Asn
               20                  25                  30
   Asn Ser Ser Ser His Leu Gln Leu Ile Tyr Asn Leu Thr Leu Cys Glu
           35                  40                  45
   Leu Asn Gly Thr Asp Trp Leu Ala Asn Lys Phe Asp Trp Ala Val Glu
       50                  55                  60
   Ser Phe Val Ile Phe Pro Val Leu Thr His Ile Val Ser Tyr Gly Ala
   65                  70                  75                  80
   Leu Thr Thr Ser His Phe Leu Asp Thr Val Gly Leu Val Thr Val Ser
                   85                  90                  95
   Thr Ala Gly Phe His His Gly Arg Tyr Val Leu Ser Ser Ile Tyr Ala
               100                 105                 110
   Val Cys Ala Leu Ala Ala Phe Ile Cys Phe Val Ile Arg Phe Ala Lys
           115                 120                 125
   Asn Cys Met Ser Trp Arg Tyr Ser Cys Thr Arg Tyr Thr Asn Phe Leu
       130                 135                 140
   Leu Asp Thr Lys Gly Arg Leu Tyr Arg Trp Arg Ser Pro Val Ile Ile
   145                 150                 155                 160
   Glu Lys Gly Gly Lys Val Glu Val Glu Gly His Leu Ile Asp Leu Lys
                   165                 170                 175
   Lys Val Val Leu Asp Gly Ser Ala Ala Thr Pro Leu Thr Arg Val Ser
               180                 185                 190
   Ala Glu Gln Trp Gly Arg Pro
           195
<210> 39
   <211> 201
   <212> PRT
   <213> Porcine reproductive and respiratory syndrome virus
<400> 39
   Met Arg Cys Ser His Lys Leu Gly Arg Phe Leu Thr Pro His Ser Cys
   1               5                   10                  15
   Phe Trp Trp Leu Phe Leu Leu Cys Thr Gly Leu Ser Trp Ser Phe Ser
               20                  25                  30
   Asp Asn Gly Gly Asp Ser Ser Thr Tyr Gln Tyr Ile Tyr Asn Leu Thr
           35                  40                  45
   Ile Cys Glu Leu Asn Gly Thr Asp Trp Leu Ser Ser His Phe Gly Trp
       50                  55                  60
   Ala Val Glu Thr Phe Val Leu Tyr Pro Val Ala Thr His Ile Leu Ser
   65                  70                  75                  80
   Leu Gly Phe Leu Thr Thr Ser His Phe Phe Asp Ala Leu Gly Leu Gly
                   85                  90                  95
   Ala Val Ser Thr Ala Gly Phe Val Gly Gly Arg Tyr Val Leu Cys Ser
               100                 105                 110
   Val Tyr Gly Ala Cys Ala Phe Ala Ala Phe Val Cys Phe Val Ile Arg
           115                 120                 125
   Ala Ala Lys Asn Cys Met Ala Cys Arg Tyr Ala Arg Thr Arg Phe Thr
       130                 135                 140
   Asn Phe Ile Val Asp Asp Arg Gly Arg Val His Arg Trp Lys Ser Pro
   145                 150                 155                 160
   Ile Val Val Glu Lys Leu Gly Lys Ala Glu Val Asp Gly Asn Leu Val
                   165                 170                 175
   Thr Ile Lys His Val Val Leu Glu Gly Val Lys Ala Gln Pro Leu Thr
               180                 185                 190
   Arg Thr Ser Ala Glu Gln Trp Glu Ala
           195                 200
<210> 40
   <211> 3293
   <212> DNA
   <213> Porcine reproductive and respiratory syndrome virus
<400> 40
   gttttatttc cctccgggcc ctgtcattga accaacttta ggcctgaatt gaaatgaaat 60
   ggggtccatg caaagccttt ttgacaaaat tggccaactt tttgtggatg ctttcacgga 120
   gttcttggtg tccattgttg atatcattat attcttggcc attttgtttg gcttcaccat 180
   cgccggttgg ctggtggtct tttgcatcag attggtttgc tccgcgatac tccgtacgcg 240
   ccctgccatt cactctgagc aattacagaa gatcttatga ggcctttctt tcccagtgcc 300
   aagtggacat tcccacctgg ggaactaaac atcctttggg gatgttttgg caccataagg 360
   tgtcaaccct gattgatgag atggtgtcgc gtcgaatgta ccgcatcatg gaaaaagcag 420
   gacaggctgc ctggaaacag gtggtgagcg aggctacgct gtctcgcatt agtagtttgg 480
   atgtggtggc tcattttcag catcttgccg ccatcgaagc cgagacctgt aaatatttgg 540
   cctcccggct gcccatgcta cacaacctgc gcatgacagg gtcaaatgta accatagtgt 600
   ataatagtac tttgaatcgg gtgtttgcta ttttcccaac ccctggttcc cggccaaagc 660
   ttcatgactt tcagcaatgg ctaatagctg tgcattcctc catattttcc tctgttgcag 720
   cttcttgtac tctctttgtt gtgctgtggt tgcgggttcc aatactacgt actgtttttg 780
   gtttccgctg gttaggggca atttttcttt cgaactcata gtgaattaca cggtgtgccc 840
   accttgcctc acccggcaag cagccgcaga ggcctacgaa cccggtaggt ctctttggtg 900
   caggataggg tacgatcgat gtggagagga cgaccatgac gagctagggt ttatgatacc 960
   gtctggcctc tccagcgaag gccacttgac cagtgtttac gcctggttgg cgttcttgtc 1020
   cttcagctac acggcccagt tccaccccga gatattcggg atagggaatg tgagtcgagt 1080
   ttatgttgac atcaaacatc aactcatctg cgccgaacat gacgggcaga acaccacctt 1140
   gcctcgtcat gacaacattt cggccgtgtt tcagacctat taccaacatc aagtcgacgg 1200
   cggcaattgg tttcacctag aatggctgcg tcccttcttt tcctcatggt tggttttaaa 1260
   tgtctcttgg tttctcaggc gttcgcctgc aaaccatgtt tcagttcgag tcttgcagac 1320
   attaagacca acaccaccgc agcggcaagc tttgctgtcc tccaagacat cagttgcctt 1380
   aggcatcgca actcggcctc tgaggcgatt cgcaaaatcc ctcagtgccg tacggcgata 1440
   gggacaccta tgtatattac catcacagcc aatgtgacag atgaaaatta tttacattct 1500
   tctgatctcc tcatgctctc ttcttgcctt ttctatgctt ctgagatgag tgaaaaggga 1560
   tttgaggtgg tttttggcaa tgtgtcaggc atcgtggctg tgtgtgtcaa ttttaccagc 1620
   tacgttcaac atgtcaggga gtttacccaa cgctccttga tggtcgacca tgtgcggctg 1680
   ctccatttca tgacacctga gaccatgagg tgggcaaccg ttttagcctg tctttttgct 1740
   attctgttgg caatttgaat gtttaagtat gttggggaaa tgcttgaccg tgggctgttg 1800
   ctcgcgattg ctttctttgt ggtgtatcgt gccgttctgt tttactgtgc tcgccgacgc 1860
   ccacagcaac agcagctctc atctgcaatt gatttacaac ttgacgctat gtgagctgaa 1920
   tggcacagat tggctagctg atagatttga ttgggcagtg gagagctttg tcatctttcc 1980
   tgttttgact cacattgtct cctatggcgc cctcaccacc agccatttcc ttgacacaat 2040
   tgctttagtc actgtgtcta ccgccgggtt tgttcacggg cggtatgtcc taagtagcat 2100
   ctacgcggtc tgtgccctgg ctgcgttgac ttgcttcgtc attaggtttg tgaagaattg 2160
   catgtcctgg cgctactcat gtactagata taccaacttt cttctggata ctaagggcag 2220
   actctatcgt tggcggtcgc ctgtcatcat agagaagagg ggcaaagttg aggtcgaagg 2280
   tcatctgatc gatctcaaaa gagttgtgct tgatggttcc gtggcaaccc ctataaccag 2340
   agtttcagcg gaacaatggg gtcgtcctta gatgacttct gttatgatag tacggctcca 2400
   caaaaggtgc ttttggcatt ttctattacc tacacgccag taatgatata tgccctaaag 2460
   gtgagtcgcg gccgactgct agggcttctg caccttttga ttttcctgaa ctgtgctttc 2520
   accttcgggt acatgacatt catgcacttt cagagtacaa ataaggtcgc gctcactatg 2580
   ggagcagtag ttgcactcct ttggggggtg tactcagcca tagaaacctg gaaattcatc 2640
   acctccagat gccgtttgtg cttgctaggc cgcaagtaca ttctggcccc tgcccaccac 2700
   gttgaaagtg ccgcaggctt tcatccgatt gcggcaaatg ataaccacgc atttgtcgtc 2760
   cggcgtcccg gctccactac ggtcaacggc acattggtgc ccgggttgaa aagcctcgtg 2820
   ttgggtggca gaaaagctgt taaacaggga gtggtaaacc ttgtcaaata tgccaaataa 2880
   caacggcaag cagcagaaga gaaagaaggg ggatggccag ccagtcaatc agctgtgcca 2940
   gatgctgggt aagatcatcg cccagcaaaa ccagtctaga ggcaagggac cgggaaagaa 3000
   aaataagaag aaaaacccgg agaagcccca ttttcctcta gcgactgaag atgatgtcag 3060
   acatcacttt acccctagtg agcggcaatt gtgtctgtcg tcaatccaaa ctgcctttaa 3120
   tcaaggcgct gggacttgca ccctgtcaga ttcagggagg ataagttaca ctgtggagtt 3180
   tagtttgcct acgcatcata ctgtgcgctt gatccgcgtc acagcatcac cctcagcatg 3240
   atgggctggc attcttgagg catcccagtg tttgaattgg aagaatgcgt ggt       3293
<210> 41
   <211> 3293
   <212> DNA
   <213> Porcine reproductive and respiratory syndrome virus
<400> 41
   gttttatttc cctccgggcc ccgtcattga accaacttta ggcctgaatt gaaatgaaat 60
   ggggtccgtg caaagccttt ttgacaaaat tggccaactt tttgtggatg ctttcacgga 120
   gttcctggtg tccattgttg atatcatcat atttttggcc attttgtttg gcttcaccat 180
   cgccggttgg ctggtggtct tttgcatcag attggtttgc tccgcgatac tccgtacgcg 240
   ccctgccatt cactctgagc aattacagaa gatcttatga ggccttttta tcccagtgcc 300
   aagtggacat tcccacctgg ggaactaaac atcctttggg gatgttttgg caccataagg 360
   tgtcaaccct gattgatgaa atggtgtcgc gtcgcatgta ccgcatcatg gaaaaagcag 420
   ggcaggctgc ctggaaacag gtggtgagcg aggctacgct gtcccgcatt agtagtttgg 480
   atgtggtggc tcattttcag catcttgccg ccattgaagc cgagacttgt aaatatttgg 540
   cctcccggct gcccatgcta cataacctgc gcataacagg gtcaaatgta accatagtgt 600
   ataatagtac ttcggagcag gtgtttgcta ttttcccaac ccctggttcc cggccaaagc 660
   ttcatgattt tcagcaatgg ttaatagctg tacattcctc catattttcc tctgttgcag 720
   cttcttgtac tctttttgtt gtgctgtggc tgcgggttcc aatgctacgt actgtttttg 780
   gtttccgctg gttaggggga atttttcctt cgaactcatg gtgaattaca cggtgtgtcc 840
   accttgcctc acccggcaag cagccgcaga ggtctacgaa cccggtaggt ctctttggtg 900
   caggataggg tatgaccgat gtggggagga cgatcatgac gagctagggt ttatgatacc 960
   gcctggcctc tccagcgaag gccacttgac tagtgtttac gcctggttgg cgtttttgtc 1020
   cttcagctac acggcccagt tccatcccga gatattcggg atagggaatg tgagtcgagt 1080
   ttatgttgac atcaaacatc aactcatttg cgccgaacat gacggacaga acgccacctt 1140
   gcctcgtcat gacaatattt cagccgtgtt tcagacctat taccaacatc aagtcgatgg 1200
   cggcaattgg tttcacctag aatggcttcg tcccttcttt tcctcatggt tggttttaaa 1260
   tgtctcttgg tatctcaggc gttcgcctgc aaaccatgct tcagttcgag tcttgcagat 1320
   attaagacca acactaccgc agcggcaagc tttgctgtcc tccaagacat cagttgcctt 1380
   aggcatcgca actcggcctc tgaggcgatt cgcaaaatcc ctcagtgccg tacggcgata 1440
   gggacacccg tgtatattac catcacagcc aatgtgacag atgagaatta tttacattct 1500
   tctgatctcc tcatgctttc ttcttgcctt ttctacgctt ctgagatgag tgaaaaagga 1560
   ttcaaggtgg tatttggcaa tgtgtcaggc atcgtggctg tgtgtgtcaa ttttaccagc 1620
   tacgtccaac atgtcaggga gtttacccaa cgctccctgg tggtcgacca tgtgcggttg 1680
   ctccatttca tgacacctga aaccatgagg tgggcaactg ttttagcctg tctttttgcc 1740
   attctgctgg caatttgaat gtttaagtat gttggggaaa tgcttgaccg cgggctgttg 1800
   ctcgcgattg ctttctttgt ggtgtatcgt gccgttctgt tttgctgtgc tcgccaacgc 1860
   cagcgccaac agcagctccc atctacagct gatttacaac ttgacgctat gtgagctgaa 1920
   tggcacagat tggctagctg ataaatttga ttgggcagtg gagagttttg tcatctttcc 1980
   cgttttgact cacattgtct cctatggtgc cctcactact agccatctcc ttgacacagt 2040
   cgccttagtc actgtgtcta ccgccgggtt tgttcacggg cggtatgtcc taagtagcat 2100
   ctacgcggtc tgtgccctgg ctgcgttagc ttgcttcgtc attaggtttg caaagaattg 2160
   catgtcctgg cgctattcgt gtaccagata taccaacttt cttctggaca ctaagggcag 2220
   actctatcgt tggcattcgc ctgtcatcat agagaaaagg ggcaaagttg aggtcgaagg 2280
   tcatctgatc gacctcaaaa gagttgtgct tgacggttcc gtggcaaccc ctataaccag 2340
   agtttcagcg gaacaatggg gtcgtcctta gatgacttct gccatgatag tacggctcca 2400
   caaaaggtgc ttttggcgtt ttctattacc tacacgccag tgatgatata tgccctaaag 2460
   gtgagtcgcg gccgactgct agggcttctg caccttttga tcttcctgaa ttgtgctttc 2520
   accttcgggt acatgacatt cgtgcacttt cagagtacaa ataaggtcgc gctcactatg 2580
   ggagcagtag ttgcactcct ttggggggtg tactcagcca tagaaacctg gaaattcatc 2640
   acctccagat gccgtttgtg cttgctaggc cgcaagtaca ttctggcccc tgcccaccac 2700
   gttgaaagtg ccgcaggctt tcatccgatt gcggcaaatg ataaccacgc atttgtcgtc 2760
   cggcgtcccg gctccactac ggtcaacggc acattggtgc ccgggttgaa aagcctcgtg 2820
   ttgggtggca gaaaagctgt taaacaggga gtggtaaacc ttgtcaaata tgccaaataa 2880
   caacggcaag cagcagaaga gaaagaaggg ggatggccag ccagtcaatc agctgtgcca 2940
   gatgctgggt aagatcatcg ctcagcaaaa ccagtccaga ggcaagggac cgggaaagaa 3000
   aaacaagaag aaaaacccgg agaagcccca ttttcctcta gcgactgaag atgatgtcag 3060
   acatcacttc acccctagtg agcggcaatt gtgtctgtcg tcaatccaga ccgcctttaa 3120
   tcaaggcgct gggacttgca ccctgtcaga ttcagggagg ataagttaca ctgtggagtt 3180
   tagtttgcca acgcatcata ctgtgcgctt gatccgcgtc acagcatcac cctcagcatg 3240
   atgggctggc attcttgagg catcccagtg tttgaattgg aagaatgcgt ggt        3293
<210> 42
   <211> 30
   <212> DNA.
   <213> Artificial Sequence
<220>
   <223> Description of Artificial Sequence: Synthetic DNA
<400> 42
   gcacggatcc gaattaacat gaaatggggt 30
<210> 43
   <211> 30
   <212> DNA
   <213> Artificial Sequence
<220>
   <223> Description of Artificial Sequence:Synthetic DNA
<400> 43
   cgtcggatcc tcctacaatg gctaatagct 30
<210> 44
   <211> 30
   <212> DNA
   <213> Artificial Sequence
<220>
   <223> Description of Artificial Sequence:Synthetic DNA
<400> 44
   gtatggatcc gcaattggtt tcacctataa 30
<210> 45
   <211> 30
   <212> DNA
   <213> Artificial Sequence
<220>
   <223> Description of Artificial Sequence:Synthetic DNA
<400> 45
   tgccaggatc cgtgtttaaa tatgttgggg 30
<210> 46
   <211> 21
   <212> DNA
   <213> Artificial Sequence
<220>
   <223> Description of Artificial Sequence:Synthetic DNA
<400> 46
   ggggatccag agtttcagcg g 21
<210> 47
   <211> 22
   <212> DNA
   <213> Artificial Sequence
<220>
   <223> Description of Artificial Sequence:Synthetic DNA
<400> 47
   ggggatcctt gttaaatatg cc 22
<210> 48
   <211> 30
   <212> DNA
   <213> Artificial Sequence
<220>
   <223> Description of Artificial Sequence:Synthetic DNA
<400> 48
   ccacctgcag attcaccgtg agttcgaaag 30
<210> 49
   <211> 30
   <212> DNA
   <213> Artificial Sequence
<220>
   <223> Description of Artificial Sequence:Synthetic DNA
<400> 49
   cgcgctgcag tgtccctatc gacgtgcggc 30
<210> 50
   <211> 30
   <212> DNA
   <213> Artificial Sequence
<220>
   <223> Description of Artificial Sequence:Synthetic DNA
<400> 50
   ataggaattc aacaagacgg cacgatacac 30
<210> 51
   <211> 30
   <212> DNA
   <213> Artificial Sequence
<220>
   <223> Description of Artificial Sequence:Synthetic DNA
<400> 51
   cgtggaattc atagaaaacg ccaagagcac 30
<210> 52
   <211> 25
   <212> DNA
   <213> Artificial Sequence
<220>
   <223> Description of Artificial Sequence:Synthetic DNA
<400> 52
   gggaattctg gcacagctga ttgac 25
<210> 53
   <211> 19
   <212> DNA
   <213> Artificial Sequence
<220>
   <223> Description of Artificial Sequence:Synthetic DNA
<400> 53
   gggaattcac cacgcattc 19
<210> 54
   <211> 15420
   <212> DNA
   <213> Porcine reproductive and respiratory syndrome virus
<400> 54
<210> 55
   <211> 15103
   <212> DNA
   <213> Porcine reproductive and respiratory syndrome virus
<400> 55
   nngacgtata ggtgttggct ctatgccttg acatttgtat tgtcaggagc tgtggccatt 60
   ggcacagccc aaaaacttgc tcacggaaac acccttctct gacagcctcc ttcaggggag 120
   cttggggtct gtccctagca ccttgcttcc ggagttgcac tgctttaccg tctctccacc 180
   cctttaacca tgtctgggat gcttgatcgg tgcacgtgta cccccaatgc cagggtgttt 240
   atggcggaag gccaagtcta ctgcacacga tgcctcagtg cacggtctct ccttcccctg 300
   aatctccaag cttctgagct tggggtgcta ggcctattct acaggcccga agagccactc 360
   cggtggacgt tgccacgtgc attccccact gttgagtgct cccccgccgg agcctgctgg 420
   ctttctgcaa tctttccaat tgcacggatg accagtggaa acctgaactt ccaacaaaga 480
   atggtacggg tcgcagctga gtttaacaga gccggccagt tcacccctgc agttttgaag 540
   actctacaag tttatgaacg gggttgccgc tggtacccca ttgttggacc tgtccctgga 600
   gtggccgttt tcgccaactc cctacatgtg agtgataaac ctttcccggg agcaactcac 660
   gtgctaacca acctgccgct cccgcagaga cccaagcctg aagacttttg cccctttgag 720
   tgtgctatgg ctactgtcta tgacattggt catgacgccg tcatgtatgt ggccgagggg 780
   aaagtctcct gggcccctcg tggcggaaat gaagtgaaat ttgaaactgt ccccgaggag 840
   ttgaaattga ttgcggaccg gctccgcacc tccatcccgc cccaccatgt agtggacatg 900
   tctaagttcg ccttcacggc tcctgggcgt ggtgtttcta tgcgggttga acgccaacac 960
   ggctgcctcc ccactgacac tgtccctgaa ggcaactgct ggtggagctt gtttaacttg 1020
   ctcccactgg aagtccagaa caaagaaatc cgccatgcta accaatttgg ctaccagacc 1080
   aagcatggtg tttctggcaa gtacctacag cggaggctgc aagttaatgg tctccgagca 1140
   gtaactgacc caaatggacc tatcgtcgta cagtacttct ccgttaagga gagttggatc 1200
   cgccacttga aactggcggg agaacccagc taccctgggt ttgaggacct cctcagaata 1260
   agggttgagc ccaatacgtc gccattggct gacaaggatg aaaaaatttt ccggtttggc 1320
   agtcacaagt ggtacggcgc tggaaagaga gcaaggaaag cacgctcttg tgcgactgcc 1380
   acagtcgctg gccgcgcttt gtccgttcgt gaaacccggc aggccaaggg gcacgaggtt 1440
   gccggcgcca acaaggctga gcacctcaaa cattattccc cgcctgccga agggaattgt 1500
   ggttggcact gcatttccgc catcgccaac cggatggtga attccaaatt tgaaaccacc 1560
   cttcccgaaa gagtgagacc tccagatgac tgggctactg acgaggatct tgtgaatgcc 1620
   attcaaatcc tcagacttcc tgcggccttg gacaggaacg gtgcttgtgt tagcgccaag 1680
   tacgtactta agctggaagg tgagcattgg actgtcactg tgacccctgg gatgtctcct 1740
   tctttgctcc ctcttgaatg tgttcagggc tgttgtgagc acaagggtgg tcttggttcc 1800
   ccagatgcag tcgaggtctt cggatttgac cctgcctgcc ttgaccggct ggctgaggtg 1860
   atgcacctgc ctagcagtgt tatcccagcc gccctggccg aaatgtccgg cgattccgat 1920
   cgttcggctt ccccggtcac caccgtgtgg actgtttcgc agttctttgc ccgtcacaac 1980
   ggagggaatc accctgacca ggcgcgctta gggaaaatta tcagcctttg tcaggtgatt 2040
   gaggactgct gctgttccca gaacaaaacc aaccgggtca ccccggagga ggtcgcagca 2100
   aagattgacc tgtacctccg tggtgcaaca aatcttgaag aatgcttggc caggcttgag 2160
   aaagcgcgcc cgccacgcgt aatggacacc tcctttgatt gggatgttgt gctccctggg 2220
   gttgaggcgg caactcagac gaccgaactg ccccgggtca accagtgtcg cgctctggtc 2280
   cctgttgtga ctcaaaagtc tctggacaat aactcggttc ctctgaccgc cttctcgctg 2340
   tccaattact actaccgtgc acaaggtgac gagattcgtc accgtgacag gctaaacgcc 2400
   gtactctcta agttggaggg ggctgttcga gaagaatatg ggctcatgcc gactggacct 2460
   ggcccgcgac ccgcactgtc gagcgggctc gatgggctta aagacagatg gagagatctg 2520
   ctgaaactag ccaacgccca gacaacctca gaaatgatgg cctgggcagc cgagcaggtt 2580
   gatctagaag cttgggtcaa aagctaccca cggtggacac caccaccccc tccgccaaga 2640
   gttcagcctc gaaaagcgaa gcctgtcagg agcttgccag agagcaagcc tgtccctgcc 2700
   ccgcgcagga aggttagatc cgatcgtggc agcccggttt tgttgggcga caatgttcct 2760
   aacagttggg aagacttgac tgtcggtggc ccccttgatc tcctgacccc acccgagtca 2820
   gtgacacctc cagtgagctt gcgcttacgt ccgcgccgca acacactttt aggccggtga 2880
   cacctttggg tgaaccggcc ccagttcccg caccgcgcag aactgtgtcc cgaccggtga 2940
   catccttgaa tgggccgatc cttatgtccg caccgcggca caagtttcag caggtggaaa 3000
   aagcaaattt ggcgacagca acgctgacgt accaggacga gcccctagat ttgtctgcat 3060
   cctcacagac tgaatatgag gcttttcctc cagcaccact gcagaacatg ggtattccgg 3120
   aggtggaagg gcaagaagct gaggaagtcc tgagtggaat ctcgatatac tggatgacat 3180
   caattctgcc ctgtatcatc aagcggttcc ctgtcaagcg tagcgatcac acgcccaata 3240
   ggtgcggaga gtgaccttac cattggctca gtcgccactg aagatattcc acgcatcctc 3300
   gggaaaatag aagatgccgg tgagatgtcc aaccagggac ccttggcatt ctccgaggaa 3360
   aaaccggtag atgaccaacc taccaaagac ccccggatgt cgtcgcggag gtcagacaag 3420
   agcgcaccag ctcggtccgc aggcacaggt ggcgtcggct tgtttactga tttgcccctt 3480
   cagacggtgt ggatgcggac ggggggggcc cgttacggac ggtaaaaaca aaaactgaaa 3540
   ggttctttga ccagctgagc cgtcaggttt ttaacctcgt ctcccatctc cctgttttct 3600
   tctcatacct tttcaaacct ggcagtggtt attctccggg tgattggggt tttgcagctt 3660
   ttactctatt gtgcctcttt ttatgttaca gttatccagc ctttggtatt gctcccctct 3720
   tgggtgtatt ttctgggtct tctcggcgcg tccgaatggg ggtttttggt tgctggttgg 3780
   cttttgctgt tggtctgttc aaatctgtgc ccgacccagt cggcactgct tgtgaatttg 3840
   actcgccaga gtgcagaaac atccttcatt cttttgagct tctcaaacct tgggaccctg 3900
   ttcgcagcct tgttgtgggc cccgtcggtc tcggccttgc cattcttggc aggttactgg 3960
   gcggggcacg ctacatctgg cactttttgc ttaggcttgg cattgttgca gattgtatct 4020
   tggctggagc ttatgtgctt tctcaaggta ggtgtaaaaa gtgctgggga tcttgtataa 4080
   gaactgctcc taatgaggtc gcttttaacg tgtttccttt cacacgtgcg accaggtcgt 4140
   cacttgttga cctgtgtgat cggttttgcg cgccaaaagg catggacccc atttttctcg 4200
   ccactgggtg gcgcgggtgc tgggccggcc gaagccccat tgagcaaccc tctgaaaaac 4260
   ctatcgcgtt tgcccagttg gatgaaaaga aaattacggc taggactgtg gtcgcccagc 4320
   cttatgaccc caaccaagcc gtaaagtgct tgcgggtatt gcaggcgggt ggggtgatgg 4380
   tggctgaggc ggtcccaaaa gtggtcaagg tttccgctgt tccattccga gccccctttt 4440
   ttcctaccgg agtgaaagtt gaccctgaat gtagggtcgt ggttgaccct gacactttca 4500
   ctgcagctct ccggtctggc tactccacca caaaccttgt ccttggtgta ggggactttg 4560
   cccagctgaa tggattaaaa atcaggcaaa tttccaagcc ttcaggagga ggcccacatc 4620
   tcatggctgc cctgcatgtt gcctgctcga tggttttgga catgcttgct gggatttatg 4680
   tgactgcggt gggttcttgc ggcaccggca ccaacgatcc gtggtgcgct aacccgtttg 4740
   gcgtccctgg ctacggacct gcctccctct gcacgtccag attgtgcatt tcccagcatg 4800
   cccttaccct gcccttgaca gcacttgtgg cgggattcgg tatccaagaa attgccttag 4860
   tcgttttgat ttttgtttcc atcggaggca tggctcatag gttgagttgt aaagctgata 4920
   tgctgtgtat tttgcttgca attgccagca atgtttgggt acctcttacc tggttgcttt 4980
   gtgtgtttcc ttgctggttg cgctgttttt ctttgcaccc ccttaccatc ctatggttgg 5040
   tgtttttctt gatttctgtg aatatgcctt caggaatctt ggccatggtg ttgttggttt 5100
   ctctttggct tcttggtcgt aatactaatg ttgctggtct tgtcaccccc tacgacattc 5160
   atcattacac cagtggcccc cgcggtgttg ccgccttggc taccgcacca gatgggactt 5220
   acttagccgc tgtccgccgt gctgcgttga ctggccgcac catgctgttc accccgtccc 5280
   agcttgggtc tcttcttgag ggtgctttca gaactcgaaa gccctcactg aacaccgtca 5340
   atgtggtcgg gtcctccatg ggctctggcg gggtgtttac catcgacggg aaagtcaagt 5400
   gcgtaactgc cgcacatgtc cttacgggta actcagctag ggtttccggg gtcggcttca 5460
   atcaaatgct tgactttgac gtaaaggggg atttcgccat agccgattgc ccgaattggc 5520
   aaggggctgc ccccaagacc caattctgcg aggatggatg gactggccgt gcctattggc 5580
   taacatcctc tggcgtcgaa cccggcgtca ttggaaaagg attcgccttc tgcttcaccg 5640
   cgtgcggcga ttccgggtcc ccagtgatca ccgaggccgg tgagcttgtc ggcgttcaca 5700
   cgggatcaaa taaacaaggg ggaggcatcg tcacgcgccc ctcaggccag ttttgtaatg 5760
   tggcacccat caagctaagc gaattaagtg aattctttgc tgggcccaag gtcccgctcg 5820
   gtgatgtgga ggttggcaac catataatta aagacatagg cgaagtgcct tcagatcttt 5880
   gtgccttgct cgctgccaaa cctgaactgg aaggaggcct ctccaccgtc caacttcttt 5940
   gtgtgttttt tctcctgtgg agaatgatgg gacatgcctg gacgcccttg gttgctgtgg 6000
   gtttctttat cttgaatgag gttctcccag ccgtcctggt ccggagtatt ttctcctttg 6060
   gaatgtttgt gctatcctgg ctcactccat ggtctgcgca agttctaatg atcaggcttc 6120
   taacagcagc tcttaacagg aacagatggt cacttgcctt tttcagcctt ggtgcggtga 6180
   ccggttttgt cgcagatctt gcggccactc aggggcatcc gttgcagaca gtgatgaatt 6240
   tgagtaccta tgcattcctg cctcggatga tggttgtgac ctcaccagtc ccagtgatcg 6300
   cgtgcggtgt cgtgcaccta cttgccatca ttttgtactt gtttaagtac cgtggcctgc 6360
   actatatcct tgttggcgat ggagtgttct ctgcggcttt cttcctgcgg tactttgccg 6420
   agggaaagtt gagggaaggg ttgtcccaat cctgcggaat gaatcatgag tccctaactg 6480
   ttgcccttgc tatgagactc aatgacgagg acttggattt ccttacgaaa tggactgatt 6540
   ttaagtgctt tgtttctgcg tccaacatga ggaatgcagc gggtcaattt atcgaggctg 6600
   cctatgctaa agcacttaga gtagaacttg cccagttggt gcaggttgat aaagttcgag 6660
   gtactttggc caaacttgaa gcttttgctg ataccgtggc accccaactc tcgcccggtg 6720
   acattgttgt cgctctcggc catacgcctg ttggcagtat cttcgaccta aaggttggta 6780
   gcaccaagca taccctccaa gccattgaaa ccagagtcct tgcagggtcc aaaatgaccg 6840
   tggcgcgcgt cgtcgacccg acccctacgc ccccacccgc acccctgtcc atccccctcc 6900
   caccgaaagt cctggagaat gcccccaacg cttgggggga tgaggaccgt ttgaataaga 6960
   agaagaggcc caggatggaa gccctcggca tctatgttat gggtgggaaa aagtaccaga 7020
   aattttggga caagaattcc ggtgatgtgt tttatgagga ggtccatgac aacacagatg 7080
   agtgggagtg tctcagagtc ggcgaccctg ccgactttga ccctgagaag ggaactctgt 7140
   gtggacatgt caccattgaa gataaggctt accatgttta cacctcctca tctggtaaga 7200
   agttcttggt ccccgtcaac ccagagaatg gaagagtcca gtgggaagct gccaagcttt 7260
   ccgtggagca gccccttggc atgatgaacg tcgacggtga actgactgcc aaagaactgg 7320
   agaaactgaa aagaataatt gataaactcc agggcctgac taaggagcag tgtttaaact 7380
   gctagccgcc agcggcttga cccgctgtgg tcgcggcggc ttagttgtta ctgagacagc 7440
   ggtgaagatc gtcaaatttc acaaccggac cttcaccttg ggacctgtga atttaaaagt 7500
   ggccagtgag gttgagctga aagacgcggt tgagcacaac cagcacccgg ttgcaagacc 7560
   ggttgatggt ggtgttgtgc tcctgcgttc tgcagttcct tcgcttgtcg acgtcttaat 7620
   ctccggtgct gatgcatctc ccaagttact tgcccatcac gggccgggaa acactgggat 7680
   cgatggcacg ctctgggatt ttgagtccga agccattaaa gaggaagtcg cacttagtgc 7740
   gcaaataata caggcttgtg acattaggcg cggtgacgca cctgaaattg gtctccctta 7800
   caaactatac cctgttaggg gcaaccctga gcgggtaaaa ggagttttgc agaatacaag 7860
   gtttggagac ataccttaca aaacccccag tgacaccgga agcccagtgc acgcggctgc 7920
   ctgccttacg cccaacgcca ccccggtgac tgatgggcgc tctgtcttgg ccacgaccat 7980
   gccctccggg ttcgagttgt atgtacccac cattccggcg tctgttcttg attatcttga 8040
   ttctaggcct gactgcccta aacagttgac agagcacggc tgtgaagatg ccgcattgag 8100
   agatctctcc aagtatgact tgtccaccca aggctttgtt ttgcctggag ttcttcgcct 8160
   tgtgcggaag tacctgtttg cccacgtggg taagtgcccg tccgttcatc ggccttccac 8220
   ttaccccgcc aaaaattcta tggctggaat aaatgggaac aggtttccaa ccaaggacat 8280
   tcagagcgtc cctgaaatcg acgttctgtg cgcacaggct gtgcgagaaa actggcaaac 8340
   tgttacccct tgtaccctta agaaacagta ctgcgggaag aagaagacta ggaccatact 8400
   cggcaccaac aacttcattg cgctggccca ccgggcagcg ttgagtggtg tcacccaagg 8460
   cttcatgaaa aaagcattta actcgcccat cgccctcggg aaaaacaaat ttaaagagct 8520
   acagactccg gtcctcggca ggtgccttga agctgatctt gcatcctgcg atcgatccac 8580
   acctgcaatt gtccgctggt ttgccgccaa tcttctttat gaactttcct gtgctgaaga 8640
   gcatctaccg tcgtacgtgc tgaactgctg ccacgaccta ctggtcacgc agtccggcgc 8700
   agtgactaag agaggtggcc tgtcgtctgg tgacccgatc acctctgtgt ccaacaccat 8760
   ttacagcttg gtgatctatg cacagcacat ggtgcttagt tacttcaaaa gtggtcatcc 8820
   ccatggcctt ctgtttttac aagaccagct aaagtttgag gacatgctca aggtccaacc 8880
   cctgatcgtc tattcggacg accttgtgct gtatgccgag tctcccacca tgccaaacta 8940
   ccattggtgg gttgaacatc tgaatctgat gttggggttt cagacggacc caaagaagac 9000
   aaccataaca gactcaccat catttctagg ctgtagaata gtaaatggac gccagctagt 9060
   ccccaaccgt gacaggattc tcgcggccct cgcctaccac atgaaggcga gtaatgtttc 9120
   tgaatactac gcctcagcgg ctgcaatact catggacagc tgtgcttgtt tagagtatga 9180
   tcctgaatgg tttgaagaac ttgtagttgg aatagcgcag tgcgcccgca aggacggcta 9240
   cagctttccc ggcacgccgt tcttcatgtc catgtgggaa aaactcaggt caaattatga 9300
   ggggaaaaag tcgagagtgt gcgggtactg cggggccccg gccccgtacg ctactgcctg 9360
   cggccttgac gtctgcattt accacaccca cttccaccag cattgtccag tcacaatctg 9420
   gtgcggccat ccagcgggtt ctggttcttg taatgagtgc aagtccccca tagggaaagg 9480
   cacaagcccc ctagacgagg tgctagaaca agtcccgtat aagcccccac ggaccgtaat 9540
   tatgcatgtg gagcagggtc ttacccccct tgacccaggt aggtaccaga ctcgccgcgg 9600
   attagtctcc gtcaggcgtg gaatcaaggg aaatgaagtt gaactaccag acggtgatta 9660
   tgctagtacc gccttgctcc ccacctgtaa agagatcaac atggtcgctg tcgcttctaa 9720
   tgtgttgcgc agcaggttca tcatcggtcc acccggtgct gggaaaacat actggctcct 9780
   tcaacaagtc caggatggtg atgttattta cacaccaact caccagacca tgcttgacat 9840
   gatcagagct ttggggacgt gccgattcaa tgtccctaca ggcacaacac tgcagttccc 9900
   tgtcccctcc cgtaccggtc cgtgggttcg catcctagcc ggtggttggt gtcctggcaa 9960
   gaattccttc ctggatgaag cagcgtatta caatcacctt gatgtcttga ggcttcttag 10020
   taaaactacc ctcacctgtc tgggagactt taaactactc cacccagtgg gttttgattc 10080
   ccattgctat gtttttgaca tcatgcctca gactcaatta aagaccatct ggagatttgg 10140
   acagaatatc tgtgatgcca ttcaaccaga ttacagggac aaactcatgt ccatggtcaa 10200
   cacaacccgt gtaacttacg tggaaaaacc cgtcaggtat gggcaagtcc ttacccccta 10260
   ccataaggac cgagaggacg gcgccatcac cattgactcc agtcaaggtg ccacgtttga 10320
   tgtggttaca ttgcatttgc ccactaaaga ttcactcaac aggcaaagag cccttgttgc 10380
   tatcactagg gcaagacatg caatttttgt gtatgaccca cacaagcaac tgcagagcct 10440
   gtttgatctc cctgcaaaag gcacacccgt caacctcgct gtgcaccgcg acgggcagct 10500
   tattgtgctg gatagaaata acaaggaatg cacggttgct caggctctag gcaatggaga 10560
   taaatttagg gccacagaca aacgcgttgt ggattctctc cgcgccattt gtgctgatct 10620
   agaagggtcg agctctccgc tccccaaggt cgcacacaac ttgggatttt atttctcacc 10680
   tgatttaacg cagtttgcta aactcccagt agaacttgca ccccactggc ccgtggtgac 10740
   aactcagaac aatgaaaagt ggccagatcg gctggttacc agccttcgcc ctatccataa 10800
   atatagccgc gcgtgcattg gtgccggcta tatggtgggt ccctcggtgt tcctgggcac 10860
   tcctggggtc gtgtcatact acctcacaaa atttgttaag ggcgaggctc aagtgcttcc 10920
   ggagacgatc ttcagcaccg gccgaattga ggtagattgc cgggaatatc ttgatgatcg 10980
   ggagcgagaa gttgctgcgt ccctcccaca tgccttcatt ggtgacgtca aaggcactac 11040
   cgttggggga tgtcaccatg tcacctccaa ataccttccg cgcttccttc ccaaggaaac 11100
   agttgcggta gtcggggttt caagccccgg aaaagccgcg aaagcagtgt gcacactgac 11160
   agatgtgtac ctcccagacc ttgaagccta tctccacccg gagactcagt ccaagtgctg 11220
   gaaattgatg ttggacttca aggaagttca ctgatggtct ggaaagacaa aacagcctat 11280
   ttccaacttg aaggtcgcta cttcacctgg tatcagcttg ctagctatgc ctcgtacatc 11340
   cgtgttcctg tcaactctac ggtgtacttg gacccctgca tgggccccgc cctttgcaac 11400
   aggagagtcg tcgggtccac ccactggggg gctgacctcg cagtcacccc ttatgattac 11460
   ggcgctaaaa tcatcctgtc tagcgcgtac catggtgaaa tgccccccgg atacaaaatt 11520
   ctggcgtgcg cggaattctc gttggatgac ccagtcaggt ataaacatac ctgggggttt 11580
   gaatcggata cagcgtatct atatgagttc accggaaacg gtgaggactg ggaggattac 11640
   aatgatgcgt tccgtgcgcg ccagaaaggg aaaatttaca aggccactgc caccagcatg 11700
   aagttttatt tccctccggg ccctgtcatt gaaccaactt taggcctgaa ttgagatgaa 11760
   atggggtcta tgcaaagcct ttttgacaaa attggccaac tttttgtgga tgctttcacg 11820
   gagttcttgg tgtccattgt tgatatcatt atatttttgg ccattttgtt tggcttcacc 11880
   atcgcaggtt ggctggtggt cttttgcatc agattggttt gctccgcgat actccgtgcg 11940
   cgccctgcca ttcactctga gcaattacag aagatcctat gaggcctttc tctctcagtg 12000
   ccaggtggac attcccacct ggggaactaa acatcctttg gggatgcttt ggcaccataa 12060
   ggtgtcaacc ctgattgatg aaatggtgtc gcgtcgaatg taccgcatca tggaaaaagc 12120
   aggacaggct gcctggaaac aggtagtgag cgaggctacg ctgtctcgca ttagtagttt 12180
   ggatgtggtg gctcattttc agcatcttgc cgccattgaa gccgagacct gtaaatatct 12240
   ggcctctcgg ctgcccatgc tacaccacct gcgcatgaca gggtcaaatg taaccatagt 12300
   gtataatagt actttgaatc aggtgtttgc tgttttccca acccctggtt cccggccaaa 12360
   gcttcatgat ttccagcaat ggctaatagc tgtacattcc tctatatttt cctctgttgc 12420
   agcttcttgt actctttttg ttgtgctgtg gttgcgggtt ccaatgctac gtactgtttt 12480
   tggtttccgc tggttagggg caatttttct ttcgaactca cggtgaatta cacggtgtgc 12540
   ccgccttgcc tcacccggca agcagccgca gaggcctacg aacccggcag gtccctttgg 12600
   tgcaggatag ggcatgatcg atgtggggag gacgatcatg atgaactagg gtttgtggtg 12660
   ccgtctggcc tctccagcga aggccacttg accagtgctt acccctggtt ggcgttcctg 12720
   tccttcagct atacggccca gttccatccc gagatattcg ggatagggaa tgtgagtcga 12780
   gtctatgttg acatcaagca ccaattcatt tgcgctgttc atgatgggca gaacaccacc 12840
   ttgccccacc atgacaacat ttcagccgtg tttcagacct attaccagca tcaggtcgac 12900
   gggggcaatt ggtttcacct agaatggctg cgtcccttct tttcctcttg gttggtttta 12960
   aatgtctctt ggtttctcag gcgttcgcct gcaagccatg tttcagttcg agtctttcag 13020
   acatcaagac caacaccacc gcagcggcag gctttgctgt cctccaagac atcagttgcc 13080
   ttaggcatcg caactcggcc tctgaggcga ttcgcaaagt ccctcagtgc cgcacggcga 13140
   tagggacacc cgtgtatatc actgtcacag ccaatgttac cgatgagaat tatttgcatt 13200
   cctctgatct tctcatgctt tcttcttgcc ttttctatgc ttctgagatg agtgaaaagg 13260
   gatttaaggt ggtatttggc aatgtgtcag gcatcgtggc agtgtgcgtc aacttcacca 13320
   gttacgtcca acatgtcaag gaatttaccc aacgttcctt ggtagttgac catgtgcggc 13380
   tgctccattt catgacgccc gagaccatga ggtgggcaac tgttttagcc tgtcttttta 13440
   ccattctgtt ggcaatttga atgtttaagt atgttgggga aatgcttgac cgcgggctgt 13500
   tgctcgcaat tgcttttttt atggtgtatc gtgccgtctt gttttgttgc gctcgtcagc 13560
   gccaacggga acagcggctc aaatttacag ctgatttaca acttgacgct atgtgagctg 13620
   aatggcacag attggctagc taataaattt gactgggcag tggagtgttt tgtcattttt 13680
   cctgtgttga ctcacattgt ctcttatggt gccctcacta ctagccattt ccttgacaca 13740
   gtcggtctgg tcactgtgtc taccgctggg tttgttcacg ggcggtatgt tctgagtagc 13800
   atgtacgcgg tctgtgccct ggctgcgttg atttgcttcg tcattaggct tgcgaagaat 13860
   tgcatgtcct ggcgctactc atgtaccaga tataccaact ttcttctgga cactaagggc 13920
   agactctatc gttggcggtc gcctgtcatc atagagaaaa ggggcaaagt tgaggtcgaa 13980
   ggtcacctga tcgacctcaa aagagttgtg cttgatggtt ccgcggctac ccctgtaacc 14040
   agagtttcag cggaacaatg gagtcgtcct tagatgactt ctgtcatgat agcacggctc 14100
   cacaaaaggt gctcttggcg ttttctatta cctacacgcc agtgatgata tatgccctaa 14160
   aggtgagtcg cggccactgc tagggcttct gcaccttttg gtcttcctga attgtgcttt 14220
   caccttcggg tacatgacat tcgtgcactt tcagagtaca aataaggtcg cgctcactat 14280
   gggagcagta gttgcactcc tttggggggt gtactcagcc atacaaacct ggaaattcat 14340
   cacctccaga tgccgtttgt gctgctaggc cgcaagtaca ttctggcccc tgcccaccac 14400
   gttgaaagtg ccgcaggctt tcatccgatt gcggcaaatg ataaccacgc atttgtcgtc 14460
   cggcgtcccg gctccactac ggtcaacggc acattggtgc ccgggttaaa aagcctcgtg 14520
   ttggtggcag aaaagctgtt aaacagggag tggtaaacct tgttaaatat gccaaataac 14580
   accggcaagc agcagaagag aaagaagggg gatggccagc cagtcaatca gctgtgccag 14640
   atgctgggta agatcatcgc tcaccaaaac cagtccagag gcaagggacc gggaaagaaa 14700
   aataagaaga aaaacccgga gaagccccat ttccctctag cgactgaaga tgatgtcaga 14760
   catcacttta cccctagtga gcgtcaattg tgtctgtcgt caatccagac cgcctttaat 14820
   caaggcgctg ggacttgcac cctgtcagat tcagggagga taagttacac tgtggagttt 14880
   agtttgccta cgcatcatac tgtgcgcctg atccgcgtca cagcatcacc ctcagcatga 14940
   tgggctggca ttcttgaggc atcccagtgt ttgaattgaa gaatgcgtgg tgaatggcac 15000
   tgattgacat tgtgcctcta agtcacctat tcaattaggg cgaccgtgtg ggggtaagat 15060
   ttaattggcg agaaccacac ggccgaaatt aaaaaaaaaa aaa 15103
<210> 56
   <211> 189
   <212> DNA
   <213> Porcine reproductive and respiratory syndrome virus
<400> 56
   nngacgtata ggtgttggct ctatgccttg acatttgtat tgtcaggagc tgtggccatt 60
   ggcacagccc aaaaacttgc tcacggaaac acccttctct gacagcctcc ttcaggggag 120
   cttggggtct gtccctagca ccttgcttcc ggagttgcac tgctttaccg tctctccacc 180
   cctttaacc 189
<210> 57
   <211> 221
   <212> DNA
   <213> Porcine reproductive and respiratory syndrome virus
<400> 57
   atgatgtgta gggtattccc cctacataca cgacacttct agtgtttgtg taccttggag 60
   gcgtgcgtac agccccgccc caccccttgg cccctgttct agcccaacag gtatccttct 120
   ctctcggggc gagtgcgccg cctgctgctc ccttgcagcg ggaaggacct cccgagtatt 180
   tccggagagc acctgcttta cgggatctcc accctttaac c 221
<210> 58
   <211> 1998
   <212> DNA
   <213> Porcine reproductive and respiratory syndrome virus
<400> 58.
   atgtctggga tgcttgatcg gtgcacgtgt acccccaatg ccagggtgtt tatggcggaa 60
   ggccaagtct actgcacacg atgcctcagt gcacggtctc tccttcccct gaatctccaa 120
   gcttctgagc ttggggtgct aggcctattc tacaggcccg aagagccact ccggtggacg 180
   ttgccacgtg cattccccac tgttgagtgc tcccccgccg gagcctgctg gctttctgca 240
   atctttccaa ttgcacggat gaccagtgga aacctgaact tccaacaaag aatggtacgg 300
   gtcgcagctg agtttaacag agccggccag ttcacccctg cagttttgaa gactctacaa 360
   gtttatgaac ggggttgccg ctggtacccc attgttggac ctgtccctgg agtggccgtt 420
   ttcgccaact ccctacatgt gagtgataaa cctttcccgg gagcaactca cgtgctaacc 480
   aacctgccgc tcccgcagag acccaagcct gaagactttt gcccctttga gtgtgctatg 540
   gctactgtct atgacattgg tcatgacgcc gtcatgtatg tggccgaggg gaaagtctcc 600
   tgggcccctc gtggcggaaa tgaagtgaaa tttgaaactg tccccgagga gttgaaattg 660
   attgcggacc ggctccgcac ctccatcccg ccccaccatg tagtggacat gtctaagttc 720
   gccttcacgg ctcctgggcg tggtgtttct atgcgggttg aacgccaaca cggctgcctc 780
   cccactgaca ctgtccctga aggcaactgc tggtggagct tgtttaactt gctcccactg 840
   gaagtccaga acaaagaaat ccgccatgct aaccaatttg gctaccagac caagcatggt 900
   gtttctggca agtacctaca gcggaggctg caagttaatg gtctccgagc agtaactgac 960
   ccaaatggac ctatcgtcgt acagtacttc tccgttaagg agagttggat ccgccacttg 1020
   aaactggcgg gagaacccag ctaccctggg tttgaggacc tcctcagaat aagggttgag 1080
   cccaatacgt cgccattggc tgacaaggat gaaaaaattt tccggtttgg cagtcacaag 1140
   tggtacggcg ctggaaagag agcaaggaaa gcacgctctt gtgcgactgc cacagtcgct 1200
   ggccgcgctt tgtccgttcg tgaaacccgg caggccaagg ggcacgaggt tgccggcgcc 1260
   aacaaggctg agcacctcaa acattattcc ccgcctgccg aagggaattg tggttggcac 1320
   tgcatttccg ccatcgccaa ccggatggtg aattccaaat ttgaaaccac ccttcccgaa 1380
   agagtgagac ctccagatga ctgggctact gacgaggatc ttgtgaatgc cattcaaatc 1440
   ctcagacttc ctgcggcctt ggacaggaac ggtgcttgtg ttagcgccaa gtacgtactt 1500
   aagctggaag gtgagcattg gactgtcact gtgacccctg ggatgtctcc ttctttgctc 1560
   cctcttgaat gtgttcaggg ctgttgtgag cacaagggtg gtcttggttc cccagatgca 1620
   gtcgaggtct tcggatttga ccctgcctgc cttgaccggc tggctgaggt gatgcacctg 1680
   cctagcagtg ttatcccagc cgccctggcc gaaatgtccg gcgattccga tcgttcggct 1740
   tccccggtca ccaccgtgtg gactgtttcg cagttctttg cccgtcacaa cggagggaat 1800
   caccctgacc aggcgcgctt agggaaaatt atcagccttt gtcaggtgat tgaggactgc 1860
   tgctgttccc agaacaaaac caaccgggtc accccggagg aggtcgcagc aaagattgac 1920
   ctgtacctcc gtggtgcaac aaatcttgaa gaatgcttgg ccaggcttga gaaagcgcgc 1980
   ccgccacgcg taatggac 1998
<210> 59
   <211> 1938
   <212> DNA
   <213> Porcine reproductive and respiratory syndrome virus
<400> 59
   atgtctggga cgttctcccg gtgcatgtgc accccggctg cccgggtatt ttggaacgcc 60
   ggccaagtct tttgcacacg gtgtctcagt gcgcggtctc ttctctctcc agagcttcag 120
   gacactgacc tcggtgcagt tggcttgttt tacaagccta gggacaagct tcactggaaa 180
   gtccctatcg gcatccctca ggtggaatgt actccatccg ggtgctgttg gctctcagct 240
   gttttccctt tggcgcgtat gacctccggc aatcacaact tcctccaacg acttgtgaag 300
   gttgctgatg ttttgtaccg tgacggttgc ttggcacctc gacaccttcg tgaactccaa 360
   gtttacgagc gcggctgcaa ctggtacccg atcacggggc ccgtgcccgg gatgggtttg 420
   tttgcgaact ccatgcacgt atccgaccag ccgttccctg gtgccaccca tgtgttgact 480
   aactcgcctt tgcctcaaca ggcttgtcgg cagccgttct gtccatttga ggaggctcat 540
   tctagcgtgt acaggtggaa gaaatttgtg gttttcacgg actcctccct caacggtcga 600
   tctcgcatga tgtggacgcc ggaatccgat gattcagccg ccctggaggt actaccgcct 660
   gagttagaac gtcaggtcga aatcctcatt cggagttttc ctgctcatca ccctgtcgac 720
   ctggccgact gggagctcac tgagtcccct gagaacggtt tttccttcaa cacgtctcat 780
   tcttgcggtc accttgtcca gaaccccgac gtgtttgatg gcaagtgctg gctctcctgc 840
   tttttgggcc agtcggtcga agtgcgctgc catgaggaac atctagctga cgccttcggt 900
   taccaaacca agtggggcgt gcatggtaag tacctccagc gcaggcttca agttcgcggc 960
   attcgtgctg tagtcgatcc tgatggtccc attcacgttg aagcgctgtc ttgcccccag 1020
   tcttggatca ggcacctgac tctggatgat gatgtcaccc caggattcgt tcgcctgaca 1080
   tcccttcgca ttgtgccgaa cacagagcct accacttccc ggatctttcg gtttggagcg 1140
   cataagtggt atggcgctgc cggcaaacgg gctcgtgcta agcgtgccgc taaaagtgag 1200
   aaggattcgg ctcccacccc caaggttgcc ctgccggtcc ccacctgtgg aattaccacc 1260
   tactctccac cgacagacgg gtcttgtggt tggcatgtcc ttgccgccat aatgaaccgg 1320
   atgataaatg gtgacttcac gtcccctctg actcagtaca acagaccaga ggatgattgg 1380
   gcttctgatt atgatcttgt tcaggcgatt caatgtctac gactgcctgc taccgtggtt 1440
   cggaatcgcg cctgtcctaa cgccaagtac cttataaaac ttaacggagt tcactgggag 1500
   gtagaggtga ggtctggaat ggctcctcgc tccctttctc gtgaatgtgt ggttggcgtt 1560
   tgctctgaag gctgtgtcgc accgccttat ccagcagacg ggctacctaa acgtgcactc 1620
   gaggccttgg cgtctgctta cagactaccc tccgattgtg ttagctctgg tattgctgac 1680
   tttcttgcta atccacctcc tcaggaattc tggaccctcg acaaaatgtt gacctccccg 1740
   tcaccagagc ggtccggctt ctctagtttg tataaattac tattagaggt tgttccgcaa 1800
   aaatgcggtg ccacggaagg ggctttcatc tatgctgttg agaggatgtt gaaggattgt 1860
   ccgagctcca aacaggccat ggcccttctg gcaaaaatta aagttccatc ctcaaaggcc 1920
   ccgtctgtgt ccctggac                                              1938
<210> 60
   <211> 652
   <212> DNA
   <213> Porcine reproductive and respiratory syndrome virus
<400> 60
   aaatttggcg acagcaacgc tgacgtacca ggacgagccc ctagatttgt ctgcatcctc 60
   acagactgaa tatgaggctt ttcctccagc accactgcag aacatgggta ttccggaggt 120
   ggaagggcaa gaagctgagg aagtcctgag tggaatctcg atatactgga tgacatcaat 180
   tctgccctgt atcatcaagc ggttccctgt caagcgtagc gatcacacgc ccaataggtg 240
   cggagagtga ccttaccatt ggctcagtcg ccactgaaga tattccacgc atcctcggga 300
   aaatagaaga tgccggtgag atgtccaacc agggaccctt ggcattctcc gaggaaaaac 360
   cggtagatga ccaacctacc aaagaccccc ggatgtcgtc gcggaggtca gacaagagcg 420
   caccagctcg gtccgcaggc acaggtggcg tcggcttgtt tactgatttg ccccttcaga 480
   cggtgtggat gcggacgggg ggggcccgtt acggacggta aaaacaaaaa ctgaaaggtt 540
   ctttgaccag ctgagccgtc aggtttttaa cctcgtctcc catctccctg ttttcttctc 600
   ataccttttc aaacctggca gtggttattc tccgggtgat tggggttttg ca         652
<210> 61
   <211> 660
   <212> DNA
   <213> Porcine reproductive and respiratory syndrome virus
<400> 61
   gaatttgccg aactcaagcg cccgcgtttc tccgcacaag ccttaattga ccgaggcggt 60
   ccacttgccg atgtccatgc aaaaataaag aaccgggtat atgaacagtg cctccaagct 120
   tgtgagcccg gtagtcgtgc aaccccagcc accagggagt ggctcgacaa aatgtgggat 180
   agggtggaca tgaaaacttg gcgctgcacc tcgcagttcc aagctggtcg cattcttgcg 240
   tccctcaaat tcctccctga catgattcaa gacacaccgc ctcctgttcc caggaagaac 300
   cgagctagtg acaatgccgg cctgaagcaa ctggtggcac agtgggatag gaaattgagt 360
   gtgacccccc ccccaaaacc ggttgggcca gtgcttgacc agatcgtccc tccgcctacg 420
   gatatccagc aagaagatgt caccccctcc gatgggccac cccatgcgcc ggattttcct 480
   agtcgagtga gcacgggcgg gagttggaaa ggccttatgc tttccggcac ccgtctcgcg 540
   gggtctatca gccagcgcct tatgacatgg gtttttgaag ttttctccca cctcccagct 600
   tttatgctca cacttttctc gccgcggggc tctatggctc caggtgattg gttgtttgca 660
<210> 62
   <211> 3198
   <212> DNA
   <213> Porcine reproductive and respiratory syndrome virus
<400> 62
   gaccccattt ttctcgccac tgggtggcgc gggtgctggg ccggccgaag ccccattgag 60
   caaccctctg aaaaacctat cgcgtttgcc cagttggatg aaaagaaaat tacggctagg 120
   actgtggtcg cccagcctta tgaccccaac caagccgtaa agtgcttgcg ggtattgcag 180
   gcgggtgggg tgatggtggc tgaggcggtc ccaaaagtgg tcaaggtttc cgctgttcca 240
   ttccgagccc ccttttttcc taccggagtg aaagttgacc ctgaatgtag ggtcgtggtt 300
   gaccctgaca ctttcactgc agctctccgg tctggctact ccaccacaaa ccttgtcctt 360
   ggtgtagggg actttgccca gctgaatgga ttaaaaatca ggcaaatttc caagccttca 420
   ggaggaggcc cacatctcat ggctgccctg catgttgcct gctcgatggt tttggacatg 480
   cttgctggga tttatgtgac tgcggtgggt tcttgcggca ccggcaccaa cgatccgtgg 540
   tgcgctaacc cgtttggcgt ccctggctac ggacctgcct ccctctgcac gtccagattg 600
   tgcatttccc agcatgccct taccctgccc ttgacagcac ttgtggcggg attcggtatc 660
   caagaaattg ccttagtcgt tttgattttt gtttccatcg gaggcatggc tcataggttg 720
   agttgtaaag ctgatatgct gtgtattttg cttgcaattg ccagcaatgt ttgggtacct 780
   cttacctggt tgctttgtgt gtttccttgc tggttgcgct gtttttcttt gcaccccctt 840
   accatcctat ggttggtgtt tttcttgatt tctgtgaata tgccttcagg aatcttggcc 900
   atggtgttgt tggtttctct ttggcttctt ggtcgtaata ctaatgttgc tggtcttgtc 960
   accccctacg acattcatca ttacaccagt ggcccccgcg gtgttgccgc cttggctacc 1020
   gcaccagatg ggacttactt agccgctgtc cgccgtgctg cgttgactgg ccgcaccatg 1080
   ctgttcaccc cgtcccagct tgggtctctt cttgagggtg ctttcagaac tcgaaagccc 1140
   tcactgaaca ccgtcaatgt ggtcgggtcc tccatgggct ctggcggggt gtttaccatc 1200
   gacgggaaag tcaagtgcgt aactgccgca catgtcctta cgggtaactc agctagggtt 1260
   tccggggtcg gcttcaatca aatgcttgac tttgacgtaa agggggattt cgccatagcc 1320
   gattgcccga attggcaagg ggctgccccc aagacccaat tctgcgagga tggatggact 1380
   ggccgtgcct attggctaac atcctctggc gtcgaacccg gcgtcattgg aaaaggattc 1440
   gccttctgct tcaccgcgtg cggcgattcc gggtccccag tgatcaccga ggccggtgag 1500
   cttgtcggcg ttcacacggg atcaaataaa caagggggag gcatcgtcac gcgcccctca 1560
   ggccagtttt gtaatgtggc acccatcaag ctaagcgaat taagtgaatt ctttgctggg 1620
   cccaaggtcc cgctcggtga tgtggaggtt ggcaaccata taattaaaga cataggcgaa 1680
   gtgccttcag atctttgtgc cttgctcgct gccaaacctg aactggaagg aggcctctcc 1740
   accgtccaac ttctttgtgt gttttttctc ctgtggagaa tgatgggaca tgcctggacg 1800
   cccttggttg ctgtgggttt ctttatcttg aatgaggttc tcccagccgt cctggtccgg 1860
   agtattttct cctttggaat gtttgtgcta tcctggctca ctccatggtc tgcgcaagtt 1920
   ctaatgatca ggcttctaac agcagctctt aacaggaaca gatggtcact tgcctttttc 1980
   agccttggtg cggtgaccgg ttttgtcgca gatcttgcgg ccactcaggg gcatccgttg 2040
   cagacagtga tgaatttgag tacctatgca ttcctgcctc ggatgatggt tgtgacctca 2100
   ccagtcccag tgatcgcgtg cggtgtcgtg cacctacttg ccatcatttt gtacttgttt 2160
   aagtaccgtg gcctgcacta tatccttgtt ggcgatggag tgttctctgc ggctttcttc 2220
   ctgcggtact ttgccgaggg aaagttgagg gaagggttgt cccaatcctg cggaatgaat 2280
   catgagtccc taactgttgc ccttgctatg agactcaatg acgaggactt ggatttcctt 2340
   acgaaatgga ctgattttaa gtgctttgtt tctgcgtcca acatgaggaa tgcagcgggt 2400
   caatttatcg aggctgccta tgctaaagca cttagagtag aacttgccca gttggtgcag 2460
   gttgataaag ttcgaggtac tttggccaaa cttgaagctt ttgctgatac cgtggcaccc 2520
   caactctcgc ccggtgacat tgttgtcgct ctcggccata cgcctgttgg cagtatcttc 2580
   gacctaaagg ttggtagcac caagcatacc ctccaagcca ttgaaaccag agtccttgca 2640
   gggtccaaaa tgaccgtggc gcgcgtcgtc gacccgaccc ctacgccccc acccgcaccc 2700
   ctgtccatcc ccctcccacc gaaagtcctg gagaatgccc ccaacgcttg gggggatgag 2760
   gaccgtttga ataagaagaa gaggcccagg atggaagccc tcggcatcta tgttatgggt 2820
   gggaaaaagt accagaaatt ttgggacaag aattccggtg atgtgtttta tgaggaggtc 2880
   catgacaaca cagatgagtg ggagtgtctc agagtcggcg accctgccga ctttgaccct 2940
   gagaagggaa ctctgtgtgg acatgtcacc attgaagata aggcttacca tgtttacacc 3000
   tcctcatctg gtaagaagtt cttggtcccc gtcaacccag agaatggaag agtccagtgg 3060
   gaagctgcca agctttccgt ggagcagccc cttggcatga tgaacgtcga cggtgaactg 3120
   actgccaaag aactggagaa actgaaaaga ataattgata aactccaggg cctgactaag 3180
   gagcagtgtt taaactgc 3198
<210> 63
   <211> 3228
   <212> DNA
   <213> Porcine reproductive and respiratory syndrome virus
<400> 63
   gatcctgtgc acttggcaac gggttggcgc gggtgctggc gtggtgagag ccccatccat 60
   caaccacacc aaaagcccat agcttatgcc aatttggatg aaaagaaaat gtctgcccaa 120
   acggtggttg ctgtcccata cgatcccagt caggctatca aatgcctgaa agttctgcag 180
   gcgggagggg ccatcgtgga ccagcctaca cctgaggtcg ttcgtgtgtc cgagatcccc 240
   ttctcagccc catttttccc aaaagttcca gtcaacccag attgcagggt tgtggtagat 300
   tcggacactt ttgtggctgc ggttcgctgc ggttactcga cagcacaact ggttctgggc 360
   cggggcaact ttgccaagtt aaatcagacc ccccccagga actctatctc caccaaaacg 420
   actggtgggg cctcttacac ccttgctgtg gctcaagtgt ctgcgtggac tcttgttcat 480
   ttcatcctcg gtctttggtt cacatcacct caagtgtgtg gccgaggaac cgctgaccca 540
   tggtgttcaa atcctttttc atatcctacc tatggccccg gagttgtgtg ctcctctcga 600
   ctttgtgtgt ctgccgacgg ggtcaccctg ccattgttct cagccgtggc acaactctcc 660
   ggtagagagg tggggatttt tattttggtg ctcgtctcct tgactgcttt ggcccaccgc 720
   atggctctta aggcagacat gttagtggtc ttttcggctt tttgtgctta cgcctggccc 780
   atgagctcct ggttaatctg cttctttcct atactcttga agtgggttac ccttcaccct 840
   cttactatgc tttgggtgca ctcattcttg gtgttttgtc tgccagcagc cggcatcctc 900
   tcactaggga taactggcct tctttgggca attggccgct ttacccaggt tgccggaatt 960
   attacacctt atgacatcca ccagtacacc tctgggccac gtggtgcagc tgctgtggcc 1020
   acagccccag aaggcactta tatggccgcc gtccggagag ctgctttaac tgggcgaact 1080
   ttaatcttca ccccgtctgc agttggatcc cttctcgaag gtgctttcag gactcataaa 1140
   ccctgcctta acaccgtgaa tgttgtaggc tcttcccttg gttccggagg ggttttcacc 1200
   attgatggca gaagaactgt cgtcactgct gcccatgtgt tgaacggcga cacagctaga 1260
   gtcaccggcg actcctacaa ccgcatgcac actttcaaga ccaatggtga ttatgcctgg 1320
   tcccatgctg atgactggca gggcgttgcc cctgtggtca aggttgcgaa ggggtaccgc 1380
   ggtcgtgcct actggcaaac atcaactggt gtcgaacccg gtatcattgg ggaagggttc 1440
   gccttctgtt ttactaactg cggcgattcg gggtcacccg tcatctcaga atctggtgat 1500
   cttattggaa tccacaccgg ttcaaacaaa cttggttctg gtcttgtgac aacccctgaa 1560
   ggggagacct gcaccatcaa agaaaccaag ctctctgacc tttccagaca ttttgcaggc 1620
   ccaagcgttc ctcttgggga cattaaattg agtccggcca tcatccctga tgtaacatcc 1680
   attccgagtg acttggcatc gctcctagcc tccgtccctg tagtggaagg cggcctctcg 1740
   accgttcaac ttttgtgtgt ctttttcctt ctctggcgca tgatgggcca tgcctggaca 1800
   cccattgttg ccgtgggctt ctttttgctg aatgaaattc ttccagcagt tttggtccga 1860
   gccgtgtttt cttttgcact ctttgtgctt gcatgggcca ccccctggtc tgcacaggtg 1920
   ttgatgatta gactcctcac ggcatctctc aaccgcaaca agctttctct ggcgttctac 1980
   gcactcgggg gtgtcgtcgg tttggcagct gaaatcggga cttttgctgg cagattgtct 2040
   gaattgtctc aagctctttc gacatactgc ttcttaccta gggtccttgc tatgaccagt 2100
   tgtgttccca ccatcatcat tggtggactc cataccctcg gtgtgattct gtggttattc 2160
   aaataccggt gcctccacaa catgctggtt ggtgatggga gtttttcaag cgccttcttc 2220
   ctacggtatt ttgcagaggg taatctcaga aaaggtgttt cacagtcctg tggcatgaat 2280
   aacgagtccc taacggctgc tttagcttgc aagttgtcac aggctgacct tgattttttg 2340
   tccagcttaa cgaacttcaa gtgctttgta tctgcttcaa acatgaaaaa tgctgccggc 2400
   cagtacattg aagcagcgta tgccaaggcc ctgcgccaag agttggcctc tctagttcag 2460
   attgacaaaa tgaaaggagt tttgtccaag ctcgaggcct ttgctgaaac agccaccccg 2520
   tcccttgaca taggtgacgt gattgttctg cttgggcaac atcctcacgg atccatcctc 2580
   gatattaatg tggggactga aaggaaaact gtgtccgtgc aagagacccg gagcctaggc 2640
   ggctccaaat tcagtgtttg tactgtcgtg tccaacacac ccgtggacgc cttgaccggc 2700
   atcccactcc agacaccaac ccctcttttt gagaatggtc cgcgtcatcg cagcgaggaa 2760
   gacgatctta aagtcgagag gatgaagaaa cactgtgtat ccctcggctt ccacaacatc 2820
   aatggcaaag tttactgcaa aatttgggac aagtctaccg gtgacacctt ttacacggat 2880
   gattcccggt acacccaaga ccatgctttt caggacaggt cagccgacta cagagacagg 2940
   gactatgagg gtgtgcaaac caccccccaa cagggatttg atccaaagtc tgaaacccct 3000
   gttggcactg ttgtgatcgg cggtattacg tataacaggt atctgatcaa aggtaaggag 3060
   gttctggtcc ccaagcctga caactgcctt gaagctgcca agctgtccct tgagcaagct 3120
   ctcgctggga tgggccaaac ttgcgacctt acagctgccg aggtggaaaa gctaaagcgc 3180
   atcattagtc aactccaagg tttgaccact gaacaggctt taaactgt 3228
<210> 64
   <211> 2696
   <212> DNA
   <213> Porcine reproductive and respiratory syndrome virus
<400> 64
   tgtataatag tactttgaat caggtgcttg ctattttccc aacccctggt tcccggccaa 60
   agcttcatga ttttcagcaa tggctaatag ctgtacattc ctctatattt tcctctgttg 120
   cagcttcttg tactcttttt gttgtgctgt ggttgcgggt tccaatgcta cgtattgctt 180
   ttggtttccg ctggttaggg gcaatttttc tttcgaactc acagtgaact acacggtgtg 240
   tccaccttgc ctcacccggc aagcagccac agaggcctac gaacctggca ggtctctttg 300
   gtgcaggata gggtatgatc gctgtgggga ggacgatcat gacgaactag ggtttgtggt 360
   gccgtctggc ctctccagcg aaggccactt gaccagtgtt tacgcctggt tggcgttcct 420
   gtctttcagt tacacagccc agttccatcc tgagatattc gggataggga atgtgagtca 480
   agtttatgtt gacatcaggc atcaattcat ttgcgccgtt cacgacgggc agaacgccac 540
   tttgcctcgc catgacaata tttcagccgt gttccagact tattaccaac atcaagtcga 600
   cggcggcaat tggtttcacc tagaatggct gcgtcccttc ttttcctctt ggttggtttt 660
   aaatgtctct tggtttctca ggcgttcgcc tgcaagccat gtttcagttc gagtcttgca 720
   gacattaaga ccaacaccac cgcagcggca ggctttgctg tcctccaaga catcagttgc 780
   cttaggtatc gcaactcggc ctctgaggcg tttcgcaaaa tccctcagtg tcgtacggcg 840
   atagggacac ccatgtatat tactgtcaca gccaatgtaa ccgatgagaa ttatttgcat 900
   tcctctgacc ttctcatgct ttcttcttgc cttttctacg cttctgagat gagtgaaaag 960
   ggatttaaag tggtatttgg caatgtgtca ggcatcgtgg ctgtgtgcgt caactttacc 1020
   agctacgtcc aacatgtcaa ggaatttacc caacgctcct tggtagtcga ccatgtgcgg 1080
   ctgctccatt tcatgacacc tgagaccatg aggtgggcaa ctgttttagc ctgtcttttt 1140
   gccattctgt tggccatttg aatgtttaag tatgttgggg aaatgcttga ccgcgggcta 1200
   ttgctcgtca ttgctttttt tgtggtgtat cgtgccgtct tggtttgttg cgctcgccag 1260
   cgccaacagc agcaacagct ctcatttaca gttgatttat aacttgacgc tatgtgagct 1320
   gaatggcaca gattggttag ctggtgaatt tgactgggca gtggagtgtt ttgtcatttt 1380
   tcctgtgttg actcacattg tctcctatgg tgccctcacc accagccatt tccttgacac 1440
   agtcggtctg gtcactgtgt ctaccgccgg cttttcccac gggcggtatg ttctgagtag 1500
   catctacgcg gtctgtgccc tggctgcgtt gatttgcttc gtcattaggt ttacgaagaa 1560
   ttgcatgtcc tggcgctact catgtaccag atataccaac tttcttctgg acactaaggg 1620
   cagactctat cgttggcggt cgcctgtcat catagagaaa aggggtaaag ttgaggtcga 1680
   aggtcatctg atcgacctca agagagttgt gcttgatggt tccgcggcaa cccctataac 1740
   caaagtttca gccgagcaat ggggtcgtcc ttagatgact tctgccatga tagcacggct 1800
   ccacaaaagg tgcttttggc gttctctatt acctacacgc cagtgatgat atatgcccta 1860
   aaagtaagtc gcggccgact gctagggctt ctgcaccttt tgatcttcct aaattgtgct 1920
   ttcaccttcg ggtacatgac attcgtgcac tttcagagca caaacaaggt cgcgctcact 1980
   atgggagcag tagttgcact cctttggggg gtgtactcag ccatagaaac ctggaaattc 2040
   atcacctcca gatgccgttt gtgcttgcta ggccgcaagt acattttggc ccctgcccac 2100
   cacgttgaaa gtgccgcagg ctttcatccg atagcggcaa atgataacca cgcatttgtc 2160
   gtccggcgtc ccggctccac tacggttaac ggcacattgg tgcccgggtt gaaaagcctc 2220
   gtgttgggtg gcagaaaagc tgtcaaacag ggagtggtaa accttgttaa atatgccaaa 2280
   taacaacggc aagcagcaga agaaaaagaa gggggatggc cagccagtca atcagctgtg 2340
   ccagatgctg ggtaagatca tcgctcagca aaaccagtcc agaggcaagg gaccgggaaa 2400
   gaaaaacaag aagaaaaacc cggagaagcc ccattttcct ctagcgactg aagatgatgt 2460
   cagacatcac ttcacctctg gtgagcggca attgtgtctg tcgtcaatcc agacagcctt 2520
   taatcaaggc gctggaactt gtaccctgtc agattcaggg aggataagtt acactgtgga 2580
   gtttagtttg ccgacgcatc atactgtgcg cctgatccgc gtcacagcgt caccctcagc 2640
   atgatgagct ggcattcttg aggcatccca gtgtttgaat tggaagaatg cgtggt 2696
<210> 65
   <211> 2696
   <212> DNA
   <213> Porcine reproductive and respiratory syndrome virus
<400> 65
   tgtataatag tactttgaat caggtgcttg ctatttttcc aacccctggt tcccggccaa 60
   agcttcatga ttttcagcaa tggctaatag ctgtacattc ctctatattt tcctctgttg 120
   cagcttcttg tactcttttt gttgtgctgt ggttgcgggt tccaatgcta cgtattgctt 180
   ttggtttccg ctggttaggg gcaatttttc cttcgaactc acagtgaact acacggtgtg 240
   tccaccttgc ctcacccggc aagcagccat agaggcctac gaacctggca ggtctctttg 300
   gtgcaggata gggtatgatc gctgtgggga ggacgatcat gacgaactag ggtttgtggt 360
   gccgtctggc ctctccagcg aaggccactt gaccagtgtt tacgcctggt tggcgttcct 420
   gtctttcagt tacacagccc agttccatcc tgagatattc gggataggga atgtgagtca 480
   agtttatgtt gacatcaggc atcaatccat ttgcgccgtt cacgacgggc agaacgccac 540
   tttgcctcgc catgacaata tttcagccgt gttccagact tattaccaac atcaagtcga 600
   cggcggcaat tggtttcacc tagaatggct gcgtcccttc ttttcctctt ggttggtttt 660
   aaatgtctct tggtttctca ggcgttcgct tgcaagccat gtttcagttc gagtcttgca 720
   gacattaaga ccaacaccac cgcagcggca ggctttgctg tcctccaaga catcagttgc 780
   cttaggtatc gcaactcggc ctctgaggcg tttcgcaaaa tccctcagtg tcgtacggcg 840
   atagggacac ccatgtatat tactgtcaca gccaatgtaa ccgatgagaa ttatttgcat 900
   tcctctgacc ttctcatgct ttcttcttgc cttttctacg cttctgagat gagtgaaaag 960
   ggatttaaag tggtatttgg caatgtgtca ggcatcgtgg ctgtgtgcgt caactttacc 1020
   agctacgtcc aacatgtcaa ggaatttacc caacgctcct tggtagtcga ccatgtgcgg 1080
   ctgctccatt tcatgacacc tgagaccatg aggtgggcaa ctgttttagc ctgtcttttt 1140
   gccattctgt tggccattta aatgtttgag tatgttgggg aaatgcttga ccgcgggcta 1200
   ttgctcgtca ttgctttttt tgtggtgtat cgtgccgtct tggtttgttg cgctcgccag 1260
   cgccaacagc atcaacagcc ctcatttaca gttgatttat aacttgacgc tatgtgagct 1320
   gaatggcaca gattggttag ctggtgaatt tgactgggca gtggagtgtt ttgtcatttt 1380
   tcctgtgttg actcacattg tctcctatgg tgccctcacc accagccatt tccttgacac 1440
   agtcggtctg gtcactgtgt ctaccgccgg cttttcccac gggcggtatg ttctgagtag 1500
   catctacgcg gtctgtgccc tggctgcgtt gatttgcttc gtcattaggt ttacgaagaa 1560
   ttgcatgtcc tggcgctact catgtaccag atataccaac tttcttctgg acactaaggg 1620
   cagactctat cgttggcggt cgcctgtcat catagagaaa aggggtaaag ttgaggtcga 1680
   aggtcatctg atcgacctca agagagttgt gcttgatggt tccgcggcaa cccctataac 1740
   caaaatttca gccgagcaat ggggtcgtcc ttagatgact tctgccatga tagcacggct 1800
   ccactaaagg tgcttttggc gttctctatt acctacacgc cagtgatgat atatgcccta 1860
   aaagtaagtc gcggccgact gttagggctt ctgcaccttt tgatcttcct aaattgtgct 1920
   ttcaccttcg ggtacatgac attcgtgcac tttcagagca caaacaaggt cgcgctcact 1980
   atgggagcag tagttgcact cctttggggg gtgtactcag ccatagaaac ctggaaattc 2040
   atcacctcca gatgccgttt gtgcttgcta ggccgcaagt acattttggc ccctgcccac 2100
   cacgttgaaa gtgccgcagg ctttcatccg atagcggcaa atgataacca cgcatttgtc 2160
   gtccggcgtc ccggctccac tacggttaac ggcacattgg tgcccgggtt gaaaagcctc 2220
   gtgttgggtg gcagaaaagc tgtcaaacag ggagtggtaa accttgttaa atatgccaaa 2280
   taacaacggc aagcagcaga agaaaaagaa gggggatggc cagccagtca atcagctgtg 2340
   ccagatgctg ggtaagatca tcgctcagca aaaccagtcc agaggcaagg gaccgggaaa 2400
   gaaaaacaag aagaaaaacc cggagaagcc ccattttcct ctagcgactg aagatgatgt 2460
   cagacatcac ttcacctctg gtgagcggct attgtgtctg tcgtcaatcc agacagcctt 2520
   taatcaaggc gctggaattt gtaccctgtc agattcaggg aggataagtt acactgtgga 2580
   gtttagtttg ccgacgcatc atactgtgcg cctgatccgc gtcacagcgt caccctcagc 2640
   atgatgagct ggcattcttg aggcatccca gtgtttgaat tggaagaatg tgtggt     2696
<210> 66
   <211> 1098
   <212> DNA
   <213> Porcine reproductive and respiratory syndrome virus
<400> 66
   attccacgca tcctcgggaa aatagaagat gccggtgaga tgtccaacca gggacccttg 60
   gcattctccg aggaaaaacc ggtagatgac caacctacca aagacccccg gatgtcgtcg 120
   cggaggtcag acaagagcgc accagctcgg tccgcaggca caggtggcgt cggcttgttt 180
   actgatttgc cccttcagac ggtgtggatg cggacggggg gggcccgtta cggacggtaa 240
   aaacaaaaac tgaaaggttc tttgaccagc tgagccgtca ggtttttaac ctcgtctccc 300
   atctccctgt tttcttctca taccttttca aacctggcag tggttattct ccgggtgatt 360
   ggggttttgc agcttttact ctattgtgcc tctttttatg ttacagttat ccagcctttg 420
   gtattgctcc cctcttgggt gtattttctg ggtcttctcg gcgcgtccga atgggggttt 480
   ttggttgctg gttggctttt gctgttggtc tgttcaaatc tgtgcccgac ccagtcggca 540
   ctgcttgtga atttgactcg ccagagtgca gaaacatcct tcattctttt gagcttctca 600
   aaccttggga ccctgttcgc agccttgttg tgggccccgt cggtctcggc cttgccattc 660
   ttggcaggtt actgggcggg gcacgctaca tctggcactt tttgcttagg cttggcattg 720
   ttgcagattg tatcttggct ggagcttatg tgctttctca aggtaggtgt aaaaagtgct 780
   ggggatcttg tataagaact gctcctaatg aggtcgcttt taacgtgttt cctttcacac 840
   gtgcgaccag gtcgtcactt gttgacctgt gtgatcggtt ttgcgcgcca aaaggcatgg 900
   accccatttt tctcgccact gggtggcgcg ggtgctgggc cggccgaagc cccattgagc 960
   aaccctctga aaaacctatc gcgtttgccc agttggatga aaagaaaatt acggctagga 1020
   ctgtggtcgc ccagccttat gaccccaacc aagccgtaaa gtgcttgcgg gtattgcagg 1080
   cgggtggggt gatggtgg 1098
<210> 67
   <211> 1108
   <212> DNA
   <213> Porcine reproductive and respiratory syndrome virus
<400> 67
   cctcctgttc ccaggaagaa ccgagctagt gacaatgccg gcctgaagca actggtggca 60
   cagtgggata ggaaattgag tgtgaccccc cccccaaaac cggttgggcc agtgcttgac 120
   cagatcgtcc ctccgcctac ggatatccag caagaagatg tcaccccctc cgatgggcca 180
   ccccatgcgc cggattttcc tagtcgagtg agcacgggcg ggagttggaa aggccttatg 240
   ctttccggca cccgtctcgc ggggtctatc agccagcgcc ttatgacatg ggtttttgaa 300
   gttttctccc acctcccagc ttttatgctc acacttttct cgccgcgggg ctctatggct 360
   ccaggtgatt ggttgtttgc aggtgtcgtt ttacttgctc tcttgctctg tcgttcttac 420
   ccgatactcg gatgccttcc cttattgggt gtcttttctg gttctttgcg gcgtgttcgt 480
   ctgggtgttt ttggttcttg gatggctttt gctgtatttt tattctcgac tccatccaac 540
   ccagtcggtt cttcttgtga ccacgattcg ccggagtgtc atgctgagct tttggctctt 600
   gagcagcgcc aactttggga acctgtgcgc ggccttgtgg tcggcccctc aggcctctta 660
   tgtgtcattc ttggcaagtt actcggtggg tcacgttatc tctggcatgt tctcctacgt 720
   ttatgcatgc ttgcagattt ggccctttct cttgtttatg tggtgtccca ggggcgttgt 780
   cacaagtgtt ggggaaagtg tataaggaca gctcctgcgg aggtggctct taatgtattt 840
   cctttctcgc gcgccacccg tgtctctctt gtatccttgt gtgatcgatt ccaaacgcca 900
   aaaggggttg atcctgtgca cttggcaacg ggttggcgcg ggtgctggcg tggtgagagc 960
   cccatccatc aaccacacca aaagcccata gcttatgcca atttggatga aaagaaaatg 1020
   tctgcccaaa cggtggttgc tgtcccatac gatcccagtc aggctatcaa atgcctgaaa 1080
   gttctgcagg cgggaggggc catcgtgg 1108
<210> 68
   <211> 7193
   <212> DNA
   <213> Porcine reproductive and respiratory syndrome virus
<400> 68
   atgtctggga tgcttgatcg gtgcacgtgt acccccaatg ccagggtgtt tatggcggaa 60
   ggccaagtct actgcacacg atgcctcagt gcacggtctc tccttcccct gaatctccaa 120
   gcttctgagc ttggggtgct aggcctattc tacaggcccg aagagccact ccggtggacg 180
   ttgccacgtg cattccccac tgttgagtgc tcccccgccg gagcctgctg gctttctgca 240
   atctttccaa ttgcacggat gaccagtgga aacctgaact tccaacaaag aatggtacgg 300
   gtcgcagctg agtttaacag agccggccag ttcacccctg cagttttgaa gactctacaa 360
   gtttatgaac ggggttgccg ctggtacccc attgttggac ctgtccctgg agtggccgtt 420
   ttcgccaact ccctacatgt gagtgataaa cctttcccgg gagcaactca cgtgctaacc 480
   aacctgccgc tcccgcagag acccaagcct gaagactttt gcccctttga gtgtgctatg 540
   gctactgtct atgacattgg tcatgacgcc gtcatgtatg tggccgaggg gaaagtctcc 600
   tgggcccctc gtggcggaaa tgaagtgaaa tttgaaactg tccccgagga gttgaaattg 660
   attgcggacc ggctccgcac ctccatcccg ccccaccatg tagtggacat gtctaagttc 720
   gccttcacgg ctcctgggcg tggtgtttct atgcgggttg aacgccaaca cggctgcctc 780
   cccactgaca ctgtccctga aggcaactgc tggtggagct tgtttaactt gctcccactg 840
   gaagtccaga acaaagaaat ccgccatgct aaccaatttg gctaccagac caagcatggt 900
   gtttctggca agtacctaca gcggaggctg caagttaatg gtctccgagc agtaactgac 960
   ccaaatggac ctatcgtcgt acagtacttc tccgttaagg agagttggat ccgccacttg 1020
   aaactggcgg gagaacccag ctaccctggg tttgaggacc tcctcagaat aagggttgag 1080
   cccaatacgt cgccattggc tgacaaggat gaaaaaattt tccggtttgg cagtcacaag 1140
   tggtacggcg ctggaaagag agcaaggaaa gcacgctctt gtgcgactgc cacagtcgct 1200
   ggccgcgctt tgtccgttcg tgaaacccgg caggccaagg ggcacgaggt tgccggcgcc 1260
   aacaaggctg agcacctcaa acattattcc ccgcctgccg aagggaattg tggttggcac 1320
   tgcatttccg ccatcgccaa ccggatggtg aattccaaat ttgaaaccac ccttcccgaa 1380
   agagtgagac ctccagatga ctgggctact gacgaggatc ttgtgaatgc cattcaaatc 1440
   ctcagacttc ctgcggcctt ggacaggaac ggtgcttgtg ttagcgccaa gtacgtactt 1500
   aagctggaag gtgagcattg gactgtcact gtgacccctg ggatgtctcc ttctttgctc 1560
   cctcttgaat gtgttcaggg ctgttgtgag cacaagggtg gtcttggttc cccagatgca 1620
   gtcgaggtct tcggatttga ccctgcctgc cttgaccggc tggctgaggt gatgcacctg 1680
   cctagcagtg ttatcccagc cgccctggcc gaaatgtccg gcgattccga tcgttcggct 1740
   tccccggtca ccaccgtgtg gactgtttcg cagttctttg cccgtcacaa cggagggaat 1800
   caccctgacc aggcgcgctt agggaaaatt atcagccttt gtcaggtgat tgaggactgc 1860
   tgctgttccc agaacaaaac caaccgggtc accccggagg aggtcgcagc aaagattgac 1920
   ctgtacctcc gtggtgcaac aaatcttgaa gaatgcttgg ccaggcttga gaaagcgcgc 1980
   ccgccacgcg taatggacac ctcctttgat tgggatgttg tgctccctgg ggttgaggcg 2040
   gcaactcaga cgaccgaact gccccgggtc aaccagtgtc gcgctctggt ccctgttgtg 2100
   actcaaaagt ctctggacaa taactcggtt cctctgaccg ccttctcgct gtccaattac 2160
   tactaccgtg cacaaggtga cgagattcgt caccgtgaca ggctaaacgc cgtactctct 2220
   aagttggagg gggctgttcg agaagaatat gggctcatgc cgactggacc tggcccgcga 2280
   cccgcactgt cgagcgggct cgatgggctt aaagacagat ggagagatct gctgaaacta 2340
   gccaacgccc agacaacctc agaaatgatg gcctgggcag ccgagcaggt tgatctagaa 2400
   gcttgggtca aaagctaccc acggtggaca ccaccacccc ctccgccaag agttcagcct 2460
   cgaaaagcga agcctgtcag gagcttgcca gagagcaagc ctgtccctgc cccgcgcagg 2520
   aaggttagat ccgatcgtgg cagcccggtt ttgttgggcg acaatgttcc taacagttgg 2580
   gaagacttga ctgtcggtgg cccccttgat ctcctgaccc cacccgagtc agtgacacct 2640
   ccagtgagct tgcgcttacg tccgcgccgc aacacacttt taggccggtg acacctttgg 2700
   gtgaaccggc cccagttccc gcaccgcgca gaactgtgtc ccgaccggtg acatccttga 2760
   atgggccgat ccttatgtcc gcaccgcggc acaagtttca gcaggtggaa aaagcaaatt 2820
   tggcgacagc aacgctgacg taccaggacg agcccctaga tttgtctgca tcctcacaga 2880
   ctgaatatga ggcttttcct ccagcaccac tgcagaacat gggtattccg gaggtggaag 2940
   ggcaagaagc tgaggaagtc ctgagtggaa tctcgatata ctggatgaca tcaattctgc 3000
   cctgtatcat caagcggttc cctgtcaagc gtagcgatca cacgcccaat aggtgcggag 3060
   agtgacctta ccattggctc agtcgccact gaagatattc cacgcatcct cgggaaaata 3120
   gaagatgccg gtgagatgtc caaccaggga cccttggcat tctccgagga aaaaccggta 3180
   gatgaccaac ctaccaaaga cccccggatg tcgtcgcgga ggtcagacaa gagcgcacca 3240
   gctcggtccg caggcacagg tggcgtcggc ttgtttactg atttgcccct tcagacggtg 3300
   tggatgcgga cggggggggc ccgttacgga cggtaaaaac aaaaactgaa aggttctttg 3360
   accagctgag ccgtcaggtt tttaacctcg tctcccatct ccctgttttc ttctcatacc 3420
   ttttcaaacc tggcagtggt tattctccgg gtgattgggg ttttgcagct tttactctat 3480
   tgtgcctctt tttatgttac agttatccag cctttggtat tgctcccctc ttgggtgtat 3540
   tttctgggtc ttctcggcgc gtccgaatgg gggtttttgg ttgctggttg gcttttgctg 3600
   ttggtctgtt caaatctgtg cccgacccag tcggcactgc ttgtgaattt gactcgccag 3660
   agtgcagaaa catccttcat tcttttgagc ttctcaaacc ttgggaccct gttcgcagcc 3720
   ttgttgtggg ccccgtcggt ctcggccttg ccattcttgg caggttactg ggcggggcac 3780
   gctacatctg gcactttttg cttaggcttg gcattgttgc agattgtatc ttggctggag 3840
   cttatgtgct ttctcaaggt aggtgtaaaa agtgctgggg atcttgtata agaactgctc 3900
   ctaatgaggt cgcttttaac gtgtttcctt tcacacgtgc gaccaggtcg tcacttgttg 3960
   acctgtgtga tcggttttgc gcgccaaaag gcatggaccc catttttctc gccactgggt 4020
   ggcgcgggtg ctgggccggc cgaagcccca ttgagcaacc ctctgaaaaa cctatcgcgt 4080
   ttgcccagtt ggatgaaaag aaaattacgg ctaggactgt ggtcgcccag ccttatgacc 4140
   ccaaccaagc cgtaaagtgc ttgcgggtat tgcaggcggg tggggtgatg gtggctgagg 4200
   cggtcccaaa agtggtcaag gtttccgctg ttccattccg agcccccttt tttcctaccg 4260
   gagtgaaagt tgaccctgaa tgtagggtcg tggttgaccc tgacactttc actgcagctc 4320
   tccggtctgg ctactccacc acaaaccttg tccttggtgt aggggacttt gcccagctga 4380
   atggattaaa aatcaggcaa atttccaagc cttcaggagg aggcccacat ctcatggctg 4440
   ccctgcatgt tgcctgctcg atggttttgg acatgcttgc tgggatttat gtgactgcgg 4500
   tgggttcttg cggcaccggc accaacgatc cgtggtgcgc taacccgttt ggcgtccctg 4560
   gctacggacc tgcctccctc tgcacgtcca gattgtgcat ttcccagcat gcccttaccc 4620
   tgcccttgac agcacttgtg gcgggattcg gtatccaaga aattgcctta gtcgttttga 4680
   tttttgtttc catcggaggc atggctcata ggttgagttg taaagctgat atgctgtgta 4740
   ttttgcttgc aattgccagc aatgtttggg tacctcttac ctggttgctt tgtgtgtttc 4800
   cttgctggtt gcgctgtttt tctttgcacc cccttaccat cctatggttg gtgtttttct 4860
   tgatttctgt gaatatgcct tcaggaatct tggccatggt gttgttggtt tctctttggc 4920
   ttcttggtcg taatactaat gttgctggtc ttgtcacccc ctacgacatt catcattaca 4980
   ccagtggccc ccgcggtgtt gccgccttgg ctaccgcacc agatgggact tacttagccg 5040
   ctgtccgccg tgctgcgttg actggccgca ccatgctgtt caccccgtcc cagcttgggt 5100
   ctcttcttga gggtgctttc agaactcgaa agccctcact gaacaccgtc aatgtggtcg 5160
   ggtcctccat gggctctggc ggggtgttta ccatcgacgg gaaagtcaag tgcgtaactg 5220
   ccgcacatgt ccttacgggt aactcagcta gggtttccgg ggtcggcttc aatcaaatgc 5280
   ttgactttga cgtaaagggg gatttcgcca tagccgattg cccgaattgg caaggggctg 5340
   cccccaagac ccaattctgc gaggatggat ggactggccg tgcctattgg ctaacatcct 5400
   ctggcgtcga acccggcgtc attggaaaag gattcgcctt ctgcttcacc gcgtgcggcg 5460
   attccgggtc cccagtgatc accgaggccg gtgagcttgt cggcgttcac acgggatcaa 5520
   ataaacaagg gggaggcatc gtcacgcgcc cctcaggcca gttttgtaat gtggcaccca 5580
   tcaagctaag cgaattaagt gaattctttg ctgggcccaa ggtcccgctc ggtgatgtgg 5640
   aggttggcaa ccatataatt aaagacatag gcgaagtgcc ttcagatctt tgtgccttgc 5700
   tcgctgccaa acctgaactg gaaggaggcc tctccaccgt ccaacttctt tgtgtgtttt 5760
   ttctcctgtg gagaatgatg ggacatgcct ggacgccctt ggttgctgtg ggtttcttta 5820
   tcttgaatga ggttctccca gccgtcctgg tccggagtat tttctccttt ggaatgtttg 5880
   tgctatcctg gctcactcca tggtctgcgc aagttctaat gatcaggctt ctaacagcag 5940
   ctcttaacag gaacagatgg tcacttgcct ttttcagcct tggtgcggtg accggttttg 6000
   tcgcagatct tgcggccact caggggcatc cgttgcagac agtgatgaat ttgagtacct 6060
   atgcattcct gcctcggatg atggttgtga cctcaccagt cccagtgatc gcgtgcggtg 6120
   tcgtgcacct acttgccatc attttgtact tgtttaagta ccgtggcctg cactatatcc 6180
   ttgttggcga tggagtgttc tctgcggctt tcttcctgcg gtactttgcc gagggaaagt 6240
   tgagggaagg gttgtcccaa tcctgcggaa tgaatcatga gtccctaact gttgcccttg 6300
   ctatgagact caatgacgag gacttggatt tccttacgaa atggactgat tttaagtgct 6360
   ttgtttctgc gtccaacatg aggaatgcag cgggtcaatt tatcgaggct gcctatgcta 6420
   aagcacttag agtagaactt gcccagttgg tgcaggttga taaagttcga ggtactttgg 6480
   ccaaacttga agcttttgct gataccgtgg caccccaact ctcgcccggt gacattgttg 6540
   tcgctctcgg ccatacgcct gttggcagta tcttcgacct aaaggttggt agcaccaagc 6600
   ataccctcca agccattgaa accagagtcc ttgcagggtc caaaatgacc gtggcgcgcg 6660
   tcgtcgaccc gacccctacg cccccacccg cacccctgtc catccccctc ccaccgaaag 6720
   tcctggagaa tgcccccaac gcttgggggg atgaggaccg tttgaataag aagaagaggc 6780
   ccaggatgga agccctcggc atctatgtta tgggtgggaa aaagtaccag aaatttgggg 6840
   acaagaattc cggtgatgtg ttttatgagg aggtccatga caacacagat gagtgggagt 6900
   gtctcagagt cggcgaccct gccgactttg accctgagaa gggaactctg tgtggacatg 6960
   tcaccattga agataaggct taccatgttt acacctcctc atctggtaag aagttcttgg 7020
   tccccgtcaa cccagagaat ggaagagtcc agtgggaagc tgccaagctt tccgtggagc 7080
   agccccttgg catgatgaac gtcgacggtg aactgactgc caaagaactg gagaaactga 7140
   aaagaataat tgataaactc cagggcctga ctaaggagca gtgtttaaac tgc 7193
<210> 69
   <211> 4382
   <212> DNA
   <213> Porcine reproductive and respiratory syndrome virus
<400> 69
   gtgtttaaac tgctagccgc cagcggcttg acccgctgtg gtcgcggcgg cttagttgtt 60
   actgagacag cggtgaagat cgtcaaattt cacaaccgga ccttcacctt gggacctgtg 120
   aatttaaaag tggccagtga ggttgagctg aaagacgcgg ttgagcacaa ccagcacccg 180
   gttgcaagac cggttgatgg tggtgttgtg ctcctgcgtt ctgcagttcc ttcgcttgtc 240
   gacgtcttaa tctccggtgc tgatgcatct cccaagttac ttgcccatca cgggccggga 300
   aacactggga tcgatggcac gctctgggat tttgagtccg aagccattaa agaggaagtc 360
   gcacttagtg cgcaaataat acaggcttgt gacattaggc gcggtgacgc acctgaaatt 420
   ggtctccctt acaaactata ccctgttagg ggcaaccctg agcgggtaaa aggagttttg 480
   cagaatacaa ggtttggaga cataccttac aaaaccccca gtgacaccgg aagcccagtg 540
   cacgcggctg cctgccttac gcccaacgcc accccggtga ctgatgggcg ctctgtcttg 600
   gccacgacca tgccctccgg gttcgagttg tatgtaccca ccattccggc gtctgttctt 660
   gattatcttg attctaggcc tgactgccct aaacagttga cagagcacgg ctgtgaagat 720
   gccgcattga gagatctctc caagtatgac ttgtccaccc aaggctttgt tttgcctgga 780
   gttcttcgcc ttgtgcggaa gtacctgttt gcccacgtgg gtaagtgccc gtccgttcat 840
   cggccttcca cttaccccgc caaaaattct atggctggaa taaatgggaa caggtttcca 900
   accaaggaca ttcagagcgt ccctgaaatc gacgttctgt gcgcacaggc tgtgcgagaa 960
   aactggcaaa ctgttacccc ttgtaccctt aagaaacagt actgcgggaa gaagaagact 1020
   aggaccatac tcggcaccaa caacttcatt gcgctggccc accgggcagc gttgagtggt 1080
   gtcacccaag gcttcatgaa aaaagcattt aactcgccca tcgccctcgg gaaaaacaaa 1140
   tttaaagagc tacagactcc ggtcctcggc aggtgccttg aagctgatct tgcatcctgc 1200
   gatcgatcca cacctgcaat tgtccgctgg tttgccgcca atcttcttta tgaactttcc 1260
   tgtgctgaag agcatctacc gtcgtacgtg ctgaactgct gccacgacct actggtcacg 1320
   cagtccggcg cagtgactaa gagaggtggc ctgtcgtctg gtgacccgat cacctctgtg 1380
   tccaacacca tttacagctt ggtgatctat gcacagcaca tggtgcttag ttacttcaaa 1440
   agtggtcatc cccatggcct tctgttttta caagaccagc taaagtttga ggacatgctc 1500
   aaggtccaac ccctgatcgt ctattcggac gaccttgtgc tgtatgccga gtctcccacc 1560
   atgccaaact accattggtg ggttgaacat ctgaatctga tgttggggtt tcagacggac 1620
   ccaaagaaga caaccataac agactcacca tcatttctag gctgtagaat agtaaatgga 1680
   cgccagctag tccccaaccg tgacaggatt ctcgcggccc tcgcctacca catgaaggcg 1740
   agtaatgttt ctgaatacta cgcctcagcg gctgcaatac tcatggacag ctgtgcttgt 1800
   ttagagtatg atcctgaatg gtttgaagaa cttgtagttg gaatagcgca gtgcgcccgc 1860
   aaggacggct acagctttcc cggcacgccg ttcttcatgt ccatgtggga aaaactcagg 1920
   tcaaattatg aggggaaaaa gtcgagagtg tgcgggtact gcggggcccc ggccccgtac 1980
   gctactgcct gcggccttga cgtctgcatt taccacaccc acttccacca gcattgtcca 2040
   gtcacaatct ggtgcggcca tccagcgggt tctggttctt gtaatgagtg caagtccccc 2100
   atagggaaag gcacaagccc cctagacgag gtgctagaac aagtcccgta taagccccca 2160
   cggaccgtaa ttatgcatgt ggagcagggt cttacccccc ttgacccagg taggtaccag 2220
   actcgccgcg gattagtctc cgtcaggcgt ggaatcaagg gaaatgaagt tgaactacca 2280
   gacggtgatt atgctagtac cgccttgctc cccacctgta aagagatcaa catggtcgct 2340
   gtcgcttcta atgtgttgcg cagcaggttc atcatcggtc cacccggtgc tgggaaaaca 2400
   tactggctcc ttcaacaagt ccaggatggt gatgttattt acacaccaac tcaccagacc 2460
   atgcttgaca tgatcagagc tttggggacg tgccgattca atgtccctac aggcacaaca 2520
   ctgcagttcc ctgtcccctc ccgtaccggt ccgtgggttc gcatcctagc cggtggttgg 2580
   tgtcctggca agaattcctt cctggatgaa gcagcgtatt acaatcacct tgatgtcttg 2640
   aggcttctta gtaaaactac cctcacctgt ctgggagact ttaaactact ccacccagtg 2700
   ggttttgatt cccattgcta tgtttttgac atcatgcctc agactcaatt aaagaccatc 2760
   tggagatttg gacagaatat ctgtgatgcc attcaaccag attacaggga caaactcatg 2820
   tccatggtca acacaacccg tgtaacttac gtggaaaaac ccgtcaggta tgggcaagtc 2880
   cttaccccct accataagga ccgagaggac ggcgccatca ccattgactc cagtcaaggt 2940
   gccacgtttg atgtggttac attgcatttg cccactaaag attcactcaa caggcaaaga 3000
   gcccttgttg ctatcactag ggcaagacat gcaatttttg tgtatgaccc acacaagcaa 3060
   ctgcagagcc tgtttgatct ccctgcaaaa ggcacacccg tcaacctcgc tgtgcaccgc 3120
   gacgggcagc ttattgtgct ggatagaaat aacaaggaat gcacggttgc tcaggctcta 3180
   ggcaatggag ataaatttag ggccacagac aaacgcgttg tggattctct ccgcgccatt 3240
   tgtgctgatc tagaagggtc gagctctccg ctccccaagg tcgcacacaa cttgggattt 3300
   tatttctcac ctgatttaac gcagtttgct aaactcccag tagaacttgc accccactgg 3360
   cccgtggtga caactcagaa caatgaaaag tggccagatc ggctggttac cagccttcgc 3420
   cctatccata aatatagccg cgcgtgcatt ggtgccggct atatggtggg tccctcggtg 3480
   ttcctgggca ctcctggggt cgtgtcatac tacctcacaa aatttgttaa gggcgaggct 3540
   caagtgcttc cggagacgat cttcagcacc ggccgaattg aggtagattg ccgggaatat 3600
   cttgatgatc gggagcgaga agttgctgcg tccctcccac atgccttcat tggtgacgtc 3660
   aaaggcacta ccgttggggg atgtcaccat gtcacctcca aataccttcc gcgcttcctt 3720
   cccaaggaaa cagttgcggt agtcggggtt tcaagccccg gaaaagccgc gaaagcagtg 3780
   tgcacactga cagatgtgta cctcccagac cttgaagcct atctccaccc ggagactcag 3840
   tccaagtgct ggaaattgat gttggacttc aaggaagttc actgatggtc tggaaagaca 3900
   aaacagccta tttccaactt gaaggtcgct acttcacctg gtatcagctt gctagctatg 3960
   cctcgtacat ccgtgttcct gtcaactcta cggtgtactt ggacccctgc atgggccccg 4020
   ccctttgcaa caggagagtc gtcgggtcca cccactgggg ggctgacctc gcagtcaccc 4080
   cttatgatta cggcgctaaa atcatcctgt ctagcgcgta ccatggtgaa atgccccccg 4140
   gatacaaaat tctggcgtgc gcggaattct cgttggatga cccagtcagg tataaacata 4200
   cctgggggtt tgaatcggat acagcgtatc tatatgagtt caccggaaac ggtgaggact 4260
   gggaggatta caatgatgcg ttccgtgcgc gccagaaagg gaaaatttac aaggccactg 4320
   ccaccagcat gaagttttat ttccctccgg gccctgtcat tgaaccaact ttaggcctga 4380
   at 4382
<210> 70
   <211> 768
   <212> DNA
   <213> Porcine reproductive and respiratory syndrome virus
<400> 70
   atgaaatggg gtctatgcaa agcctttttg acaaaattgg ccaacttttt gtggatgctt 60
   tcacggagtt cttggtgtcc attgttgata tcattatatt tttggccatt ttgtttggct 120
   tcaccatcgc aggttggctg gtggtctttt gcatcagatt ggtttgctcc gcgatactcc 180
   gtgcgcgccc tgccattcac tctgagcaat tacagaagat cctatgaggc ctttctctct 240
   cagtgccagg tggacattcc cacctgggga actaaacatc ctttggggat gctttggcac 300
   cataaggtgt caaccctgat tgatgaaatg gtgtcgcgtc gaatgtaccg catcatggaa 360
   aaagcaggac aggctgcctg gaaacaggta gtgagcgagg ctacgctgtc tcgcattagt 420
   agtttggatg tggtggctca ttttcagcat cttgccgcca ttgaagccga gacctgtaaa 480
   tatctggcct ctcggctgcc catgctacac cacctgcgca tgacagggtc aaatgtaacc 540
   atagtgtata atagtacttt gaatcaggtg tttgctgttt tcccaacccc tggttcccgg 600
   ccaaagcttc atgatttcca gcaatggcta atagctgtac attcctctat attttcctct 660
   gttgcagctt cttgtactct ttttgttgtg ctgtggttgc gggttccaat gctacgtact 720
   gtttttggtt tccgctggtt aggggcaatt tttctttcga actcacgg 768
<210> 71
   <211> 732
   <212> DNA
   <213> Porcine reproductive and respiratory syndrome virus
<400> 71
   atggctaata gctgtacatt cctctatatt ttcctctgtt gcagcttctt gtactctttt 60
   tgttgtgctg tggttgcggg ttccaatgct acgtactgtt tttggtttcc gctggttagg 120
   ggcaattttt ctttcgaact cacggtgaat tacacggtgt gcccgccttg cctcacccgg 180
   caagcagccg cagaggccta cgaacccggc aggtcccttt ggtgcaggat agggcatgat 240
   cgatgtgggg aggacgatca tgatgaacta gggtttgtgg tgccgtctgg cctctccagc 300
   gaaggccact tgaccagtgc ttacccctgg ttggcgttcc tgtccttcag ctatacggcc 360
   cagttccatc ccgagatatt cgggataggg aatgtgagtc gagtctatgt tgacatcaag 420
   caccaattca tttgcgctgt tcatgatggg cagaacacca ccttgcccca ccatgacaac 480
   atttcagccg tgtttcagac ctattaccag catcaggtcg acgggggcaa ttggtttcac 540
   ctagaatggc tgcgtccctt cttttcctct tggttggttt taaatgtctc ttggtttctc 600
   aggcgttcgc ctgcaagcca tgtttcagtt cgagtctttc agacatcaag accaacacca 660
   ccgcagcggc aggctttgct gtcctccaag acatcagttg ccttaggcat cgcaactcgg 720
   cctctgaggc ga 732
<210> 72
   <211> 534
   <212> DNA
   <213> Porcine reproductive and respiratory syndrome virus
<400> 72
   atggctgcgt cccttctttt cctcttggtt ggttttaaat gtctcttggt ttctcaggcg 60
   ttcgcctgca agccatgttt cagttcgagt ctttcagaca tcaagaccaa caccaccgca 120
   gcggcaggct ttgctgtcct ccaagacatc agttgcctta ggcatcgcaa ctcggcctct 180
   gaggcgattc gcaaagtccc tcagtgccgc acggcgatag ggacacccgt gtatatcact 240
   gtcacagcca atgttaccga tgagaattat ttgcattcct ctgatcttct catgctttct 300
   tcttgccttt tctatgcttc tgagatgagt gaaaagggat ttaaggtggt atttggcaat 360
   gtgtcaggca tcgtggcagt gtgcgtcaac ttcaccagtt acgtccaaca tgtcaaggaa 420
   tttacccaac gttccttggt agttgaccat gtgcggctgc tccatttcat gacgcccgag 480
   accatgaggt gggcaactgt tttagcctgt ctttttacca ttctgttggc aatt 534
<210> 73
   <211> 600
   <212> DNA
   <213> Porcine reproductive and respiratory syndrome virus
<400> 73
   atgttgggga aatgcttgac cgcgggctgt tgctcgcaat tgcttttttt atggtgtatc 60
   gtgccgtctt gttttgttgc gctcgtcagc gccaacggga acagcggctc aaatttacag 120
   ctgatttaca acttgacgct atgtgagctg aatggcacag attggctagc taataaattt 180
   gactgggcag tggagtgttt tgtcattttt cctgtgttga ctcacattgt ctcttatggt 240
   gccctcacta ctagccattt ccttgacaca gtcggtctgg tcactgtgtc taccgctggg 300
   tttgttcacg ggcggtatgt tctgagtagc atgtacgcgg tctgtgccct ggctgcgttg 360
   atttgcttcg tcattaggct tgcgaagaat tgcatgtcct ggcgctactc atgtaccaga 420
   tataccaact ttcttctgga cactaagggc agactctatc gttggcggtc gcctgtcatc 480
   atagagaaaa ggggcaaagt tgaggtcgaa ggtcacctga tcgacctcaa aagagttgtg 540
   cttgatggtt ccgcggctac ccctgtaacc agagtttcag cggaacaatg gagtcgtcct 600
<210> 74
   <211> 519
   <212> DNA
   <213> Porcine reproductive and respiratory syndrome virus
<400> 74
   atggagtcgt ccttagatga cttctgtcat gatagcacgg ctccacaaaa ggtgctcttg 60
   gcgttttcta ttacctacac gccagtgatg atatatgccc taaaggtgag tcgcggccac 120
   tgctagggct tctgcacctt ttggtcttcc tgaattgtgc tttcaccttc gggtacatga 180
   cattcgtgca ctttcagagt acaaataagg tcgcgctcac tatgggagca gtagttgcac 240
   tcctttgggg ggtgtactca gccatacaaa cctggaaatt catcacctcc agatgccgtt 300
   tgtgctgcta ggccgcaagt acattctggc ccctgcccac cacgttgaaa gtgccgcagg 360
   ctttcatccg attgcggcaa atgataacca cgcatttgtc gtccggcgtc ccggctccac 420
   tacggtcaac ggcacattgg tgcccgggtt aaaaagcctc gtgttggtgg cagaaaagct 480
   gttaaacagg gagtggtaaa ccttgttaaa tatgccaaa                       519
<210> 75
   <211> 368
   <212> DNA
   <213> Porcine reproductive and respiratory syndrome virus
<400> 75
   atgccaaata acaccggcaa gcagcagaag agaaagaagg gggatggcca gccagtcaat 60
   cagctgtgcc agatgctggg taagatcatc gctcaccaaa accagtccag aggcaaggga 120
   ccgggaaaga aaaataagaa gaaaaacccg gagaagcccc atttccctct agcgactgaa 180
   gatgatgtca gacatcactt tacccctagt gagcgtcaat tgtgtctgtc gtcaatccag 240
   accgccttta atcaaggcgc tgggacttgc accctgtcag attcagggag gataagttac 300
   actgtggagt ttagtttgcc tacgcatcat actgtgcgcc tgatccgcgt cacagcatca 360
   ccctcagc                                                         368
<210> 76
   <211> 7194
   <212> DNA
   <213> Porcine reproductive and respiratory syndrome virus
<400> 76
   atgtctggga tacttgatcg gtgtacgtgc acccccaatg ccagggtgtt tatggcggag 60
   ggccaggtct actgcacacg atgtctcagt gcacggtctc tccttcctct gaatctccag 120
   actcccgagc ttggggtgtt gggtctattc tacaggcccg aagaaccact ccggtggacg 180
   ttgccacgtg cattccccac tgttgagtgt tcccccgctg gggcctgctg gctttctgca 240
   atctttccaa ttgcgcgaat gaccagtgga aacctgaact tccaacaaag aatggtacgg 300
   gtcgcagctg agctttacag agccggccag ctcacccctg tcgtcttgaa gactctgcaa 360
   gtttacgaac ggggttgccg ctggtacccc attgttggac ctgtccctgg agtggccgtt 420
   ttcgccaact ccctacatgt gagtgataaa cctttcccag gggcaactca cgtgttaacc 480
   aacctgccgc tcccgcagag acccaagccc gaagacttct gcccctttga atgcgccatg 540
   gccaccgtct atgacattgg tcatgacgct gtcatgtaca tggccggagg gaaagtctcc 600
   tgggcccctc gtggcgggga tggagtgaaa tttgaaactg tccccaaggg gttggagtta 660
   actgcggacc gactccgctc ctccttcccg ccccaccacg tagtggacat gtccaggttt 720
   gctttcacaa cccctgagtg tggtgcctct atgcgggtcg gacgccaacg tggctgcctc 780
   cccgctggta ctgtccctga aggcaactgt tggtggagct tgtttggctc gctcccactg 840
   gaagttctga acaaagaaat tcgctatgcc aaccgatttg gctaccaaac taagcatggt 900
   gtctctggca agtacctaca gcggaggctg caagttaatg gtctccgggc agtaactgac 960
   acacatggac ctatcgtcat acaatacttc tccgttaagg agagttggat ccgccacttg 1020
   agactggcgg aagaacccag cctccctggg tttgaggatc tcctcagaat aagggttgag 1080
   cccaacacat cgccattgct tggcaagggt gaaaaaatct tccgttttgg caatcacaaa 1140
   tggtacggcg ctggaaagag agcaaggaaa gcacgctcta gtgcgactgc tacggtcgct 1200
   gaccgcgctt tgtccgctcg tgaaacccgg ctggccaagg agcacgaggt tgccggcgcc 1260
   aataaggctg agcacctcaa gcactactcc ccgcctgccg aagggaattg tggttggcac 1320
   tgtatttccg ccatcgtcaa ccggatggtg aactccaaat ttgaaaccac cctccccgag 1380
   agagtgagac ctccagatga ctgggctact gacgaggatc ttgcgaacac catccaaatc 1440
   ctcaggcttc ctgcggcctt ggacaggggc ggtgcttgtg ttagcgccaa gtatgtactt 1500
   aagctggaag gtgaacattg gactgtctct gtgacccctg ggatgtctcc ctctttgctc 1560
   ccccttgaat gcgtccaggg ctgttgtgat cataagagcg gtcttggttc cccagatacg 1620
   gtcgaagttt ccggatttga ccctgcctgc cttgaccggc tggctgaggt gatgcacctg 1680
   cctagcagtg ccatcccagc cgctctggcc gaaatgtccg gcgattccga tcgtccggct 1740
   tccccggtca ccactgtgtg gacggtttcg cagttctttg cccgccacac aggagggaat 1800
   caccctgacc aggtgtgctt aggaaaaatc attagccttt gtcaagtgct tgagagttgc 1860
   tgctgtttcc agaacaaaac caaccgggcc accccggaag aggtcgcggc aaaaattgac 1920
   ctgtacctcc gcggagcaac aggtcttgaa gaatgcttgg ccaggcttga gagggctcgc 1980
   ccaccgagtg taatggacac ctcctttgat tggaatgttg tgcttcctgg gtttgaggcg 2040
   gcaactcaga caaccaaacc gccccaggtc aaccagtgtc gcgctctggt ccctgttgtg 2100
   actcaagagt ctttggacaa tggctcggtt cctctgaccg ccttctcgct gtccaattac 2160
   tactaccgcg cgcaaggaga cgaggttcgt caccgtgata ggttaaacgc cgtactctcc 2220
   aagttggagg gtgctgttcg agaagaatac gggctcatgc caactggacc tggcccgcga 2280
   cccgcactgc cgagtgggct tgacgagctt aaagaccaga tggaggagga tctgctgaaa 2340
   ctagccaatg cccagacaac ttcagaaatg atggcctggg cagccgagca ggttgatcta 2400
   aaagcttggg ttaaaaacta cccacggtgg acaccaccgc cccctccacc aagagtccag 2460
   cctcgaaaaa caaagcctgt caagagtttg ccagagagca agcctgtccc cgccccgcgc 2520
   aggaaggtta ggtccgattg tggcagcccg attttattgg gcgacaatgt tcctaacagt 2580
   tgggaagatt tgactgttgg tggccccctt gatctctcga cctcacccga gccggtgaca 2640
   cctccgagtg agcttgcgct catgtccgca ccgcaacaca cttttaggtc ggtgataccc 2700
   ttgggtgaac cggccccagt tcccgcattg cgcaaaactg tgccccgacc ggtaacaccc 2760
   ttgagcgagc cgatccctgt gtccgcaccg caatgcaagt ttcagcaggt ggaaaaagcg 2820
   gatctggcgg cagcagcgct ggcgtaccag gacgagcccc tagatttgtc tgcatcctca 2880
   caaactgaat atgaggcttc tcccctagaa ccactgcaga gcatgggcgt tctaaaggtg 2940
   gaaggacaag aagctgagga agtcctgagt ggaatctcgg acatactgga tgacatcaac 3000
   ccggtgcctg tatcatcaaa cggctccctg tcaagcgtga ggatcacacg cccaaaatac 3060
   tcagctcaag ccatcatcga ctcgggcggg ccctgcagtg ggcacctcca agggataaag 3120
   gaaacatgcc tcagtatcat gcgtgaggca tgtgatgcga ctaagcttga tgaccctact 3180
   acgcaggaat ggctttctcg catgtgggat agggtggaca tgctgacttg gcgcaacacg 3240
   tctgcttacc aggcgcttcg caccttagat agcaggtttg agtttctccc aaaaatgata 3300
   ctcgagacac cgccgcccta tccgtgtgag tttgtgatga tgcctcacac gcctgcacct 3360
   tctgtaagtg cggagagtga tcttaccatt ggctcagtcg ccactgaaga tgttccacgc 3420
   atcctcggga aaatagaaga tgtcggcgag atgaccaacc aggacccctt ggcattctcc 3480
   gaggaagaac cggtggatca ccaacctgcc aagggctccc ggtcattgtc gcggaggcct 3540
   gacgagagta caccaactct gtccgcaagc gcaggtggca ccgacttacc caccgatttg 3600
   ccgctttcag acggtgtgga tgcggacggg ggggggccgt tacggacggt aaaaaacaaa 3660
   actcaaaggc tctttgacca actgagccgt caggttttta acctcgtctc ccatctccct 3720
   gtttcttcct cacgccttct cctacctggc ggtggttatt ctccgggtga ttggggcttt 3780
   gcagctttta ctctattgtg cctctttttg tgttatagct acccagcctt tggtattgct 3840
   ccccttttgg gtgtattttc tgggtcttct cggcgcgttc gaatgggggt ttttggctgc 3900
   tggttggctt ttgctgttgg cctgttcaag cctgtgtccg acccagtcgg cactgcttgt 3960
   gagtttgact cgccagagtg tagaaacatc cttctttctt ttgagcttct caaaccttgg 4020
   gaccctgttc gcagccttgt tgtgggcccc gtcggtctcg gtcttgccat tcttggcagg 4080
   ttactgggcg gggcacgctg tatctggcac tttttgctta ggcttggcat tgttacagat 4140
   tgtatcctgg ctggagctta tgtgctttct caaggtaggt gtaaaaagtg ctggggatct 4200
   tgtataagaa ctgctcctag tgaggtcgcc tttaacgtgt ttccttttac acgtgcgacc 4260
   aggtcgtcac ttaccaactt gtgcgatcgg ttttgtgcgc caaaaggcat ggaccccatt 4320
   ttcctcgcca ctgggtggcg cgggtgctgg accggccgaa gccccattga gcaaccctct 4380
   gaaaaaccca tcgcgtttgc ccagttggat gaaaagaaga ttacggctaa gactgtggtc 4440
   gcccagcctt atgaccccaa ccaagccgta aagtgtttgc gggtgttaca ggcgggcggg 4500
   gtgatggtgg ctgaggcagt tccaaaagtg gtcaaggttt ccgctgtccc attccgagcc 4560
   cccttctttc ccactggggt gaaagttgat cctgggtgca ggatcgtggt tgaccccgac 4620
   accttcactg cagctctccg gtctggttac tccaccacaa acctcgtcct tggtgtaggg 4680
   gactttgccc agctgaatgg attaaaaatt aggcaaattt ccaagccttc tggaggaggc 4740
   ccacacctca tggctgccct gcatgttgct tgctcgatga ccttgcacat gcttgctggg 4800
   atttacgtga ctgcggtggg ttcttgcggc accggcacca acgatccgtg gtgcgctaac 4860
   ccgtttgccg tccctggcta tggacctgga tctctctgca cgtccaaatt gtgcatctcc 4920
   caacatggcc tcaccctgcc cttaacagca cttgttgcgg gattcggtat tcaggaaatt 4980
   gccttggtcg ttttgatttt tgtttccatc gggggcatgg ctcataggtt gagttgtaag 5040
   gctgatatgc tgtgtgtttt gcttgcaatc gccagctatg tttgggtacc tctaacctgg 5100
   ttgctttgtg tgtttccctg ctggttgcgc tgtttttctt tgcacccact caccatccta 5160
   tggttggtgt ttttcttgat ttctgtaaat atgccttcag gaatcttggc catggtgttg 5220
   ttggtttctc tttggcttct tggacgttat actaatgtcg ctggtcttgt caccccttat 5280
   gatattcacc attacaccag tggcccccgc ggtgttgccg ccttggctac agcaccagat 5340
   gggacctact tggccgctgt ccgccgcgct gcgttgactg gccgcaccat gctgtttacc 5400
   ccgtctcagc ttgggtccct tcttgagggc gcttttagaa ctcaaaagcc ctcgttgaac 5460
   accgtcaatg tggtcggtcc tccatgggct ctggcggggt gttcaccatc gacgggaaaa 5520
   tcaagtgcgt aactgccgca catgtcctta cgggcaattc agctagggtt tccggggtcg 5580
   gtttcaacca aatgcttgac tttgatgtaa aaggagactt cgccatggcc gattgcccgg 5640
   attggcaagg ggctgctccc aagacccaat tctgcaagga tggatggact ggccgtgcct 5700
   actggctaac atcctctggc gtcgaacccg gtgtcattgg aaaaggattc gccttctgct 5760
   tcaccgcgtg cggcattccg ggtccccagt gatcaccgag gccggtgagc ttgtcggtgt 5820
   ccacacggga tcaaataaac aaggaggagg catcgtcacg cgcccctcag gccagttttg 5880
   taatgtgtca cccgtcaagc taagcgaatt aagtgaattc tttgctgggc ctaaggtccc 5940
   gctcggtgat gtgaaggttg gcagccatat aatcaaagat ataggcgagg taccttcaga 6000
   tctttgcgcc ttgcttgctg ccaaacctga actggaagga ggcctctcca ccgtccaact 6060
   tctgtgtgtg ttttttctcc tgtggaggat gatgggacat gcctggacgc ccttggttgc 6120
   tgtggggttc tttatcttga atgaggttct tccagctgtc ctggtccgga gtgtcttctc 6180
   ctttggaatg tttgtgctat cctggctcac accatggtct gcgcaagttc tgatgatcag 6240
   gcttctaaca gcagctctta acaggaacag aggttcactt gccttttaca ccctcggtgc 6300
   aataaccggc tttgtccaga tcttgcggtt actcagggac atccgttgca ggcagtgatg 6360
   aatttgagca cctatgcatt cctgcctcgg atgatggttg tgacctcacc agtcccagtg 6420
   atcgcgtgtg gtgttgcgca cctgcttgcc atcattttgt acttgtttaa gtaccgcggc 6480
   ctgcacaaga tccttgttgg cgatggagcg ttctctgcgg ctttcttcct gcgatacttt 6540
   gccgagggaa agttgaggga aggggtgtcg caatcctgcg gaatgaatca tgagtcactg 6600
   actggtgccc tcgccatgaa actcaatgac gaggacttgg atttccttac gaaatggact 6660
   gattttaagt gctttgtttc tgcatccaac atgaggaatg cagcgggcca atttatcgag 6720
   gctgcctatg ctaaagcact tagagtagaa cttgcccagt tggtacaggt tgataaggtt 6780
   cgaggcacta tggccaaact agaagctttt gctgacaccg tggcacccca actctcgccc 6840
   ggtgacattg ttgtcgctct tggccatacg cctgttggca gtatcttcga cctaaaggtt 6900
   ggtagcacta agcacaccct ccaagccatt gagaccagat ttcttgctgg gtccaaaatg 6960
   accgtggcgc gtgtcgtcga cccgaccccc acgcccccac ccgcacccgt gcccatcccc 7020
   ctcccaccga aagttctgga gaatggtccc aacgcttggg gggatgagga tcgtttgaat 7080
   aaaaaaaaaa ggcgcaggat ggaagccctc ggcatctatg ttatgggtgg gaaaaagtac 7140
   cagaaatttt gggataagaa ctccggtgat gtgttttatg aggaggtcca taat       7194
<210> 77
   <211> 4352
   <212> DNA
   <213> Porcine reproductive and respiratory syndrome virus
<400> 77
   tgataaactc cagtgcctga ctaaggagca gtgtttaaac tgctagccgc cagcggcttg 60
   acccgctgtg gtcgcggcgg cttggttgtc actgagacag cggtaaaaat agtcaaattt 120
   cacaaccgga ccttcaccct gggacctgtg aatttaaaag tggccagtga ggttgagtta 180
   aaagacgcgg tcgagcacaa ccaacacccg gttgcaagac cggttgatgg tggtgttgtg 240
   ctcctgcgtt ctgcagttcc ttcacttata gacgtcctga tctccggtgc cgacgcatct 300
   cctaagttgc tcgcccatca cgggccgggg aacactggga tcgatggcac gctttgggat 360
   ttcgagtctg aggccactaa agaggaagtc gcacttagtg cgcaaataat acaggcttgt 420
   gacatcaggc gcggggacgc acccaaaatt gatctcccct acaagctgta ccctgttagg 480
   ggcaaccctg agcgggtgaa aggagttctg aggaatacaa ggtttggaga cataccttac 540
   aagaccccca gtgacactgg gagcccggtg cacgcggccg cctgccttac gcctaacgcc 600
   actccggtga ctgacgggcg ctccatcttg gccacgacca tgccctctgg gtttgagttg 660
   tatgtaccga ccattccagc gtctgtcctt gattaccttg attctaggcc tgactgccct 720
   aaacagttga cagagcacgg ctgtgaagat gccgcactga gagacctctc caaatatgac 780
   ttgtccaccc aaggctttgt tttacctgga gttcttcgcc tcgtgcggaa atacctgttt 840
   gcccatgtag gtaagtgccc acctgttcac cggccttcta cttatcctgc taagaattct 900
   atggctggac taaatgggaa caggttcccg accaaggata ttcagagcgt ccctgaaatc 960
   gacgttctgt gcgcgcaggc tgtgcggaaa actggcagac tgttacccct tgtaccctta 1020
   agaagcagta ttgcgggaag aagaaaacta ggacaatact cggcaccaat aacttcatcg 1080
   cgctggctca tcgggcagcg ttgagtggtg tcacccaggg cttcatgaaa aaggcattta 1140
   actcgcccat cgccctcgga aaaaacaaat ttaaggagct acaaactccg gtcctaggca 1200
   gatgccttga agctgatctt gcatcctgcg accgatccac acctgcaatt gtccgttggt 1260
   ttgccgccaa tcttctttat gaacttgcct gtgctgaaga tcacctgcca tcttatgtgc 1320
   tgaactgttg ccacgactta ttggtcacgc agtctggcgc agtgactaag agaggtggcc 1380
   tgtcatctgg cgacccgatc acctctgtgt ctaacaccat ttacagcttg gtgatctatg 1440
   cacagcacat ggtgctcagt tacttcaaaa gtggtcaccc ccacggcctt ctgttcttac 1500
   aagaccagct aaagtttgag gacatgctca aggttcaacc cctgatcgtc tattcggacg 1560
   acctcgtgct gtatgccgag tctcccacca tgccaaacta ccactggtgg gttgaacatc 1620
   tgaatttaat gctggggttt cagacggacc caaagaagac agctataaca gactcgccat 1680
   catttctagg ctgcaggata ataaatggac gccagctagt ccctaaccgt gacaggattc 1740
   tcgcggccct cgcctaccat atgaaggcga gtaatgtttc tcaatactac gcttcggcgg 1800
   ctgcaatact catggacagc tgtgcttgtt tagagtatga tcctgaatgg tttgaagaac 1860
   ttatagttgg aatatcgcag tgcgcccgca aggacggcta tagctttccc ggtccgccgt 1920
   tcttcttgtc tatgtgggaa aaactcaggt ctaattatga ggggaagaag tcgagagtgt 1980
   gcgggtactg cggggccccg gccccgtacg ctactgcctg tggcctcgat gtctgcattt 2040
   accacaccca cttccaccag cattgtccgg ttataatttg gtgtggccac ccagcgggtt 2100
   ctggttcttg tagtgagtgc aaatcccccg tggggaaagg cacaagccct ctggacgagg 2160
   tgttaaaaca agtcccgtat aaacccccac ggaccataat catgcatgtg gaacagggtc 2220
   ttacccccct tgacccaggc agataccaga ctcgccgcgg attggtctcc gttaggcgcg 2280
   gaatcagggg gaatgaagtt gaactaccag acggtgatta cgctagtacc gccttgctcc 2340
   ccacctgtaa agagatcaac atggtcgctg tcgcttctaa tgtgttgcgc agcaggttca 2400
   tcatcggtcc gcccggtgct gggaagacat actggcttct acaacaggtc caggatggtg 2460
   atgtcattta cacaccaact caccagacca tgcttgacat gattagagct ttggggacgt 2520
   gccggttcaa cgtcccagca ggcacaacgc tgcaattccc tgtcccctcc cgtaccggtc 2580
   cgtgggttcg catcctagcc ggcggttggt gtcctggcaa gaattccttc ctggatgaag 2640
   cagcgtattg caatcacctt gatgtcttga ggcttcttag caaaactacc ctcacctgtc 2700
   tgggagattt caaacaactc cacccagtgg gttttgattc tcattgctat gtttttgaca 2760
   ctatgcctca gactcaactg aagaccatct ggagattcgg acagaatatt tgtgatgcca 2820
   tccaaccaga ttacagagac aaactcatgt ccatggtcaa cacaacccgt gtaacctacg 2880
   tggagagacc tgtcaggcat gggcaagtcc tcacccccta ccacagggac cgagaggacg 2940
   acgccatcac cattgactcc agccaaggcg ccacatttga tgtggttaca ttgcatttgc 3000
   ccactaaaga ttcactcaac aggcaaagag cccttgttgc tatcaccagg gcaagacatg 3060
   ctatctttgt gtatgaccca cacaggcaac tgcagagcct atttgatctt cctgcgaaaa 3120
   gcacccctgt caacctcgca gtgcaccgcg acgggcagct gatcgtgcta gatagaaata 3180
   acaaagaatg cacggttgct caggctcttg gcaacggaga taaatttagg gccacagaca 3240
   agcgcgttgt agactctctc cgcgccattt gtgctgatct agaagggtct agctctccgc 3300
   tccccaaggt cgcccacaac ttgggatttc atttctcacc tgatttgaca cagtttgcca 3360
   aactcccagt agaacttgca cctcactggc ccgtggtgac aacccagaac aatgaaaagt 3420
   ggccagatcg gctggttgct agccttcgcc ctattcataa atatagccgc gcgtgcattg 3480
   gtgccggcta tatggtgggc ccctcggtgt ttctaggcac ccctggggtc gtgtcatact 3540
   acctcacaaa atttattaag ggcgaggctc aagtgcttcc ggagacggtc ttcagcaccg 3600
   gtcgaattga ggtagattgc cgggaatacc ttgatgatcg ggagccagaa gttgctgcgt 3660
   ccctcccaca tgccttcatt ggcgacgtca aaggcactac cgttggggga tgtcaccatg 3720
   tcacttccaa ataccttccg cgcttccttc ctaaggaatc agttgcggta gtcggggttt 3780
   cgagccccgg aaaagccgcg aaagcagtgt gcacactgac agatgtgtac ctcccagacc 3840
   ttgaagccta cctccacccg gaaacccagt ccaagtgctg gaaattgatg ttggacttca 3900
   aggaagtccg actgatggtc tggaaagaca agacggccta tttccaactt gaaggccgct 3960
   atttcacctg gtatcagctt gctagctacg cctcgtacat ccgtgttcct gtcaactctg 4020
   cggtgtactt agacccctgc atgggccctg ccctttgcaa caggagagtt atcgggtcca 4080
   ctcattgggg agctgacctc gcagtcaccc cttatgatta cggtgccaaa attattttgt 4140
   ctagtgcgta ccatggtgaa atgcctcccg ggtacaagat tctggcgtgc gcagagttct 4200
   cgcttgacga cccagtcaag tacaagcaca cctgggggtt tgaatcggat acagcgtatc 4260
   tgtatgagtt caccggaaac ggtgaggact gggaggatta caatgatgcg tttcgtgcgc 4320
   gccaggaggg gaaagtctat aaggccactg cc                               4352
<210> 78
   <211> 768
   <212> DNA
   <213> Porcine reproductive and respiratory syndrome virus
<400> 78
   atgaaatggg gtctatgcaa agcctttttg acaaaattgg ccaacttttc gtggatgctt 60
   tcacggagtt cttggtgtcc attgttgata tcattatatt tttggccatt ttgtttggct 120
   tcaccatcgc cggttggctg gtggtctttt gcatcagatt ggtttgctcc gcgctactcc 180
   gtgcgcgccc tgccattcac tctgagcaat tacagaagat cctatgaggc ctttctttct 240
   cagtgccagg tggacattcc cacctgggga tttaaacatc ctttggggat gttttggcac 300
   cataaggtgt caaccctgat tgatgaaatg gtgtcgcgtc gaatgtaccg catcatggat 360
   aaagcaggac aggctgcctg gaaacaggtg gtgagcgagg ctacgctgtc tcgcattagt 420
   agtttggatg tggtggctca ctttcagcat cttgccgcca ttgaagccga gacctgtaaa 480
   tatttggcct ctcggctgcc catgctacac aacctgcgca tgacagggtc aaatgtaacc 540
   atagtgtata atagtacttt gaatcaggtg cttgctattt ttccaacccc tggttcccgg 600
   ccaaagcttc atgattttca gcaatggcta atagctgtac attcctctat attttcctct 660
   gttgcagctt cttgtactct ttttgttgtg ctgtggttgc gggttccaat gctacgtatt 720
   gcttttggtt tccgctggtt aggggcaatt tttccttcga actcacag               768
<210> 79
   <211> 662
   <212> DNA
   <213> Porcine reproductive and respiratory syndrome virus
<400> 79
   atggctaata gctgtacatt cctctatatt ttcctctgtt gcagcttctt gtactctttt 60
   tgttgtgctg tggttgcggg ttccaatgct acgtattgct tttggtttcc gctggttagg 120
   ggcaattttt ccttcgaact cacagtgaac tacacggtgt gtccaccttg cctcacccgg 180
   caagcagcca tagaggccta cgaacctggc aggtctcttt ggtgcaggat agggtatgat 240
   cgctgtgggg aggacgatca tgacgaacta gggtttgtgg tgccgtctgg cctctccagc 300
   gaaggccact tgaccagtgt ttacgcctgg ttggcgttcc tgtctttcag ttacacagcc 360
   cagttccatc ctgagatatt cgggataggg aatgtgagtc aagtttatgt tgacatcagg 420
   catcaatcca tttgcgccgt tcacgacggg cagaacgcca ctttgcctcg ccatgacaat 480
   atttcagccg tgttccagac ttattaccaa catcaagtcg acggcggcaa ttggtttcac 540
   ctagaatggc tgcgtccctt cttttcctct tggttggttt taaatgtctc ttggtttctc 600
   aggcgttcgc ttgcaagcca tgtttcagtt cgagtcttgc agacattaag accaacacca 660
   cc                                                                662
<210> 80
   <211> 535
   <212> DNA
   <213> Porcine reproductive and respiratory syndrome virus
<400> 80
   atggctgcgt cccttctttt cctcttggtt ggttttaaat gtctcttggt ttctcaggcg 60
   ttcgcttgca agccatgttt cagttcgagt cttgcagaca ttaagaccaa caccaccgca 120
   gcggcaggct ttgctgtcct ccaagacatc agttgcctta ggtatcgcaa ctcggcctct 180
   gaggcgtttc gcaaaatccc tcagtgtcgt acggcgatag ggacacccat gtatattact 240
   gtcacagcca atgtaaccga tgagaattat ttgcattcct ctgaccttct catgctttct 300
   tcttgccttt tctacgcttc tgagatgagt gaaaagggat ttaaagtggt atttggcaat 360
   gtgtcaggca tcgtggctgt gtgcgtcaac tttaccagct acgtccaaca tgtcaaggaa 420
   tttacccaac gctccttggt agtcgaccat gtgcggctgc tccatttcat gacacctgag 480
   accatgaggt gggcaactgt tttagcctgt ctttttgcca ttctgttggc cattt      535
<210> 81
   <211> 600
   <212> DNA
   <213> Porcine reproductive and respiratory syndrome virus
<400> 81
   atgttgggga aatgcttgac cgcgggctat tgctcgtcat tgcttttttt gtggtgtatc 60
   gtgccgtctt ggtttgttgc gctcgccagc gccaacagca tcaacagccc tcatttacag 120
   ttgatttata acttgacgct atgtgagctg aatggcacag attggttagc tggtgaattt 180
   gactgggcag tggagtgttt tgtcattttt cctgtgttga ctcacattgt ctcctatggt 240
   gccctcacca ccagccattt ccttgacaca gtcggtctgg tcactgtgtc taccgccggc 300
   ttttcccacg ggcggtatgt tctgagtagc atctacgcgg tctgtgccct ggctgcgttg 360
   atttgcttcg tcattaggtt tacgaagaat tgcatgtcct ggcgctactc atgtaccaga 420
   tataccaact ttcttctgga cactaagggc agactctatc gttggcggtc gcctgtcatc 480
   atagagaaaa ggggtaaagt tgaggtcgaa ggtcatctga tcgacctcaa gagagttgtg 540
   cttgatggtt ccgcggcaac ccctataacc aaaatttcag ccgagcaatg gggtcgtcct 600
<210> 82
   <211> 522
   <212> DNA
   <213> Porcine reproductive and respiratory syndrome virus
<400> 82
   atggggtcgt ccttagatga cttctgccat gatagcacgg ctccactaaa ggtgcttttg 60
   gcgttctcta ttacctacac gccagtgatg atatatgccc taaaagtaag tcgcggccga 120
   ctgttagggc ttctgcacct tttgatcttc ctaaattgtg ctttcacctt cgggtacatg 180
   acattcgtgc actttcagag cacaaacaag gtcgcgctca ctatgggagc agtagttgca 240
   ctcctttggg gggtgtactc agccatagaa acctggaaat tcatcacctc cagatgccgt 300
   ttgtgcttgc taggccgcaa gtacattttg gcccctgccc accacgttga aagtgccgca 360
   ggctttcatc cgatagcggc aaatgataac cacgcatttg tcgtccggcg tcccggctcc 420
   actacggtta acggcacatt ggtgcccggg ttgaaaagcc tcgtgttggg tggcagaaaa 480
   gctgtcaaac agggagtggt aaaccttgtt aaatatgcca aa                    522
<210> 83
   <211> 469
   <212> DNA
   <213> Porcine reproductive and respiratory syndrome virus
<400> 83
   atgccaaata acaacggcaa gcagcagaag aaaaagaagg gggatggcca gccagtcaat 60
   cagctgtgcc agatgctggg taagatcatc gctcagcaaa accagtccag aggcaaggga 120
   ccgggaaaga aaaacaagaa gaaaaacccg gagaagcccc attttcctct agcgactgaa 180
   gatgatgtca gacatcactt cacctctggt gagcggctat tgtgtctgtc gtcaatccag 240
   acagccttta atcaaggcgc tggaatttgt accctgtcag attcagggag gataagttac 300
   actgtggagt ttagtttgcc gacgcatcat actgtgcgcc tgatccgcgt cacagcgtca 360
   ccctcagcat gatgagctgg cattcttgag gcatcccagt gtttgaattg gaagaatgtg 420
   tggtgaatgg cactgattga cattgtgctt ctaagtcacc tattcaatt            469
<210> 84
   <211> 20
   <212> DNA ,
   <213> Porcine reproductive and respiratory syndrome virus
<220>
   <223> Description of Artificial Sequence:Synthetic DNA
<400> 84
   cgtacggcga tagggacacc 20
<210> 85
   <211> 20
   <212> DNA
   <213> Porcine reproductive and respiratory syndrome virus
<220>
   <223> Description of Artificial Sequence:Synthetic DNA
<400> 85
   ggcatatatc atcactggcg 20
<210> 86
   <211> 7
   <212> PRT
   <213> Porcine reproductive and respiratory syndrome virus
<400> 86
   Asn Gly Asn Ser Gly Ser Asn
     1               5
<210> 87
   <211> 7
   <212> PRT
   <213> Porcine reproductive and respiratory syndrome virus
<400> 87
   Ser Asn Asp Ser Ser Ser His
     1               5
<210> 88
   <211> 7
   <212> PRT
   <213> Porcine reproductive and respiratory syndrome virus
<400> 88
   Ser Ser Ser Asn Ser Ser His
   1 5
<210> 89
   <211> 7
   <212> PRT
   <213> Porcine reproductive and respiratory syndrome virus
<400> 89
   Ser Ala Asn Ser Ser Ser His
   1 5
<210> 90
   <211> 7
   <212> PRT
   <213> Porcine reproductive and respiratory syndrome virus
<400> 90
   His Ser Asn Ser Ser Ser His
   1 5
<210> 91
   <211> 7
   <212> PRT
   <213> Porcine reproductive and respiratory syndrome virus
<400> 91
   Ser Asn Ser Ser Ser Ser His
   1 5
<210> 92
   <211> 7
   <212> PRT
   <213> Porcine reproductive and respiratory syndrome virus
<400> 92
   Asn Asn Ser Ser Ser Ser His
   1 5
<210> 93
   <211> 8
   <212> PRT
   <213> Porcine reproductive and respiratory syndrome virus
<400> 93
   Asn Gly Gly Asp Ser Ser Thr Tyr
   1 5
<210> 94
   <211> 10
   <212> PRT
   <213> Porcine reproductive and respiratory syndrome virus
<400> 94
   Ala Asn Lys Phe Asp Trp Ala Val Glu Thr
   1 5 10
<210> 95
   <211> 10
   <212> PRT
   <213> Porcine reproductive and respiratory syndrome virus
<400> 95
   Ala Asn Lys Phe Asp Trp Ala Val Glu Pro
   1 5 10
<210> 96
   <211> 10
   <212> PRT
   <213> Porcine reproductive and respiratory syndrome virus
<400> 96
   Ala Gly Glu Phe Asp Trp Ala Val Glu Thr
   1 5 10
<210> 97
   <211> 10
   <212> PRT
   <213> Porcine reproductive and respiratory syndrome virus
<400> 97
   Ala Asp Lys Phe Asp Trp Ala Val Glu Pro
   1 5 10
<210> 98
   <211> 10
   <212> PRT
   <213> Porcine reproductive and respiratory syndrome virus
<400> 98
   Ala Asp Arg Phe Asp Trp Ala Val Glu Pro
   1 5 10
<210> 99
   <211> 10
   <212> PRT
   <213> Porcine reproductive and respiratory syndrome virus
<400> 99
   Ser Ser His Phe Gly Trp Ala Val Glu Thr
   1 5 10
<210> 100
   <211> 9
   <212> PRT
   <213> Porcine reproductive and respiratory syndrome virus
<400> 100
   Leu Ile Cys Phe Val Ile Arg Leu Ala
   1 5
<210> 101
   <211> 9
   <212> PRT
   <213> Porcine reproductive and respiratory syndrome virus
<400> 101
   Leu Thr Cys Phe Val Ile Arg Phe Ala
   1 5
<210> 102
   <211> 9
   <212> PRT
   <213> Porcine reproductive and respiratory syndrome virus
<400> 102
   Leu Ile Cys Phe Val Ile Arg Phe Thr
   1 5
<210> 103
   <211> 9
   <212> PRT
   <213> Porcine reproductive and respiratory syndrome virus
<400> 103
   Leu Ala Cys Phe Val Ile Arg Phe Ala
   1 5
<210> 104
   <211> 9
   <212> PRT
   <213> Artificial Sequence
<220>
   <223> Description of Artificial Sequence:Synthetic DNA
<400> 104
   Leu Thr Cys Phe Val Ile Arg Phe Val
   1 5
<210> 105
   <211> 9
   <212> PRT
   <213> Artificial Sequence
<220>
   <223> Description of Artificial Sequence:Synthetic DNA
<400> 105
   Leu Thr Cys Phe Ile Ile Arg Phe Ala
   1 5
<210> 106
   <211> 9
   <212> PRT
   <213> Artificial Sequence
<220>
   <223> Description of Artificial Sequence:Synthetic DNA
<400> 106
   Phe Ile Cys Phe Val Ile Arg Phe Ala
   1 5
<210> 107
   <211> 9
   <212> PRT
   <213> Artificial Sequence
<220>
   <223> Description of Artificial Sequence:Synthetic DNA
<400> 107
   Phe Val Cys Phe Val Ile Arg Ala Ala
   1 5
<210> 108
   <211> 18
   <212> PRT
   <213> Artificial Sequence
<220>
   <223> Description of Artificial Sequence:Synthetic DNA
<400> 108
   Leu Gln Leu Ile Tyr Asn Leu Thr Leu Cys Glu Leu Asn Gly Thr Asp
   1 5 10 15
   Trp Leu
<210> 109
   <211> 21
   <212> DNA
   <213> Artificial Sequence
<220>
   <223> Description of Artificial Sequence:Synthetic DNA
<400> 109
   gggatccttt tgtggagccg t 21
<210> 110
   <211> 24
   <212> DNA
   <213> Artificial Sequence
<220>
   <223> Description of Artificial Sequence:Synthetic DNA
<400> 110
   ggggaattcg ggatagggaa tgtg 24
<210> 111
   <211> 27
   <212> DNA
   <213> Artificial Sequence
<220>
   <223> Description of Artificial Sequence:Synthetic DNA
<400> 111
   gggggatcct gttggtaata gagtctg 27
<210> 112
   <211> 28
   <212> DNA
   <213> Artificial Sequence
<220>
   <223> Description of Artificial Sequence:Synthetic DNA
<400> 112
   ggtgaattcg ttttatttcc ctccgggc 28
<210> 113
   <211> 18
   <212> DNA
   <213> Artificial Sequence
<220>
   <223> Description of Artificial Sequence:Synthetic DNA
<400> 113
   gatagagtct gcccttag 18
<210> 114
   <211> 18
   <212> DNA
   <213> Artificial Sequence
<220>
   <223> Description of Artificial Sequence:Synthetic DNA
<400> 114
   ggtttcacct agaatggc 18
<210> 115
   <211> 17
   <212> DNA
   <213> Artificial Sequence
<220>
   <223> Description of Artificial Sequence:Synthetic DNA
<400> 115
   gcttctgaga tgagtga 17
<210> 116
   <211> 18
   <212> DNA
   <213> Artificial Sequence
<220>
   <223> Description of Artificial Sequence:Synthetic DNA
<400> 116
   caaccaggcg taaacact 18
<210> 117
   <211> 17
   <212> DNA
   <213> Artificial Sequence
<220>
   <223> Description of Artificial Sequence:Synthetic DNA
<400> 117
   ctgagcaatt acagaag 17
<210> 118
   <211> 18
   <212> DNA
   <213> Artificial Sequence
<220>
   <223> Description of Artificial Sequence:Synthetic DNA
<400> 118
   gactgatggt ctggaaag 18
<210> 119
   <211> 18
   <212> DNA
   <213> Artificial Sequence
<220>
   <223> Description of Artificial Sequence:Synthetic DNA
<400> 119
   ctgtatccga ttcaaacc 18
<210> 120
   <211> 18
   <212> DNA
   <213> Artificial Sequence
<220>
   <223> Description of Artificial Sequence:Synthetic DNA
<400> 120
   aggttggctg gtggtctt 18
<210> 121
   <211> 18
   <212> DNA
   <213> Artificial Sequence.
<220>
   <223> Description of Artificial Sequence:Synthetic DNA
<400> 121
   tcgctcacta cctgtttc 18
<210> 122
   <211> 18
   <212> DNA
   <213> Artificial Sequence
<220>
   <223> Description of Artificial Sequence:Synthetic DNA
<400> 122
   tgtgcccgcc ttgcctca 18
<210> 123
   <211> 18
   <212> DNA
   <213> Artificial Sequence
<220>
   <223> Description of Artificial Sequence:Synthetic DNA
<400> 123
   aaaccaattg cccccgtc 18
<210> 124
   <211> 18
   <212> DNA
   <213> Artificial Sequence
<220>
   <223> Description of Artificial Sequence:Synthetic DNA
<400> 124
   tatatcactg tcacagcc 18
<210> 125
   <211> 18
   <212> DNA
   <213> Artificial Sequence
<220>
   <223> Description of Artificial Sequence:Synthetic DNA
<400> 125
   caaattgcca acagaatg 18
<210> 126
   <211> 20
   <212> DNA
   <213> Artificial Sequence
<220>
   <223> Description of Artificial Sequence:Synthetic DNA
<400> 126
   caacttgacg ctatgtgagc 20
<210> 127
   <211> 20
   <212> DNA
   <213> Artificial Sequence
<220>
   <223> Description of Artificial Sequence:Synthetic DNA
<400> 127
   gccgcggaac catcaagcac 20
<210> 128
   <211> 20
   <212> DNA
   <213> Artificial Sequence
<220>
   <223> Description of Artificial Sequence:Synthetic DNA
<400> 128
   gactgctagg gcttctgcac 20
<210> 129
   <211> 18
   <212> DNA
   <213> Artificial Sequence
<220>
   <223> Description of Artificial Sequence:Synthetic DNA
<400> 129
   cgttgaccgt agtggagc 18
<210> 130
   <211> 22
   <212> DNA
   <213> Artificial Sequence
<220>
   <223> Description of Artificial Sequence:Synthetic DNA
<400> 130
   ccccatttcc ctctagcgac tg 22
   <210> 131
   <211> 22
   <212> DNA
   <213> Artificial Sequence
**<220>**
   <223> Description of Artificial Sequence:Synthetic DNA
<400> 131
   cggccgtgtg gttctcgcca at 22
   <210> 132
   <211> 24
   <212> DNA
   <213> Artificial Sequence
<220>
   <223> Description of Artificial Sequence:Synthetic DNA
<400> 132
   ggggaattcg ggatagggaa tgtg 24
<210> 133
   <211> 22
   <212> DNA
   <213> Artificial Sequence
<220>
   <223> Description of Artificial Sequence:Synthetic DNA
<400> 133
   ggggatcctt ttgtggagcc gt 22
<210> 134
   <211> 28
   <212> DNA
   <213> Artificial Sequence
<220>
   <223> Description of Artificial Sequence:Synthetic DNA
<400> 134
   ggtgaattcg ttttatttcc ctccgggc 28
<210> 135
   <211> 27
   <212> DNA
   <213> Artificial Sequence
<220>
   <223> Description of Artificial Sequence:Synthetic DNA
<400> 135
   gggggatcct gttggtaata gagtctg 27
<210> 136
   <211> 18
   <212> DNA
   <213> Artificial Sequence
<220>
   <223> Description of Artificial Sequence:Synthetic DNA
<400> 136
   ggtttcacct agaatggc 18
<210> 137
   <211> 18
   <212> DNA
   <213> Artificial Sequence
<220>
   <223> Description of Artificial Sequence:Synthetic DNA
<400> 137
   gatagagtct gcccttag 18
<210> 138
   <211> 17
   <212> DNA
   <213> Artificial Sequence
<220>
   <223> Description of Artificial Sequence:Synthetic DNA
<400> 138
   gcttctgaga tgagtga 17
<210> 139
   <211> 17
   <212> DNA
   <213> Artificial Sequence
<220>
   <223> Description of Artificial Sequence:Synthetic DNA
<400> 139
   ctgagcaatt acagaag 17
<210> 140
   <211> 18
   <212> DNA
   <213> Artificial Sequence
<220>
   <223> Description of Artificial Sequence:Synthetic DNA
<400> 140
   caaccaggcg taaacact 18
<210> 141
   <211> 19
   <212> DNA
   <213> Artificial Sequence
<220>
   <223> Description of Artificial Sequence:Synthetic DNA
<400> 141
   gactgcttta cggtctctc 19
<210> 142
   <211> 18
   <212> DNA
   <213> Artificial Sequence
<220>
   <223> Description of Artificial Sequence:Synthetic DNA
<400> 142
   gatgcctgac acattgcc 18
<210> 143
   <211> 19
   <212> DNA
   <213> Artificial Sequence
<220>
   <223> Description of Artificial Sequence:Synthetic DNA
<400> 143
   ctgcaagact cgaactgaa 19
<210> 144
   <211> 20
   <212> DNA
   <213> Artificial Sequence
<220>
   <223> Description of Artificial Sequence:Synthetic DNA
<400> 144
   ccccattgtt ggacctgtcc 20
<210> 145
   <211> 19
   <212> DNA
   <213> Artificial Sequence
<220>
   <223> Description of Artificial Sequence:Synthetic DNA
<400> 145
   gtcacaacag ggaccgagc 19
<210> 146
   <211> 27
   <212> DNA
   <213> Artificial Sequence
<220>
   <223> Description of Artificial Sequence:Synthetic DNA
<400> 146
   ccaagctccc ctgaaggagg ctgtcac 27
<210> 147
   <211> 27
   <212> DNA
   <213> Artificial Sequence
<220>
   <223> Description of Artificial Sequence:Synthetic DNA
<400> 147
   ccaagctccc ctgaaggagg ctgtcac 27
<210> 148
   <211> 25
   <212> DNA
   <213> Artificial Sequence
<220>
   <223> Description of Artificial Sequence:Synthetic DNA
<400> 148
   agcatcccag acatggttaa agggg 25
<210> 149
   <211> 14
   <212> DNA
   <213> Artificial Sequence
<220>
   <223> Description of Artificial Sequence:Synthetic DNA
<400> 149
   ccaccccttt aacc 14
<210> 150
   <211> 14
   <212> DNA
   <213> Artificial Sequence
<220>
   <223> Description of Artificial Sequence:Synthetic DNA
<400> 150
   ccaccccttg aacc 14
<210> 151
   <211> 14
   <212> DNA
   <213> Artificial Sequence
<220>
   <223> Description of Artificial Sequence:Synthetic DNA
<400> 151
   cctgtcattg aacc 14
<210> 152
   <211> 14
   <212> DNA
   <213> Artificial Sequence
<220>
   <223> Description of Artificial Sequence:Synthetic DNA
<400> 152
   ccacccctgt aacc 14
<210> 153
   <211> 14
   <212> DNA
   <213> Artificial Sequence
<220>
   <223> Description of Artificial Sequence:Synthetic DNA
<400> 153
   ccaccccttt aacc 14
<210> 154
   <211> 14
   <212> DNA
   <213> Artificial Sequence
<220>
   <223> Description of Artificial Sequence:Synthetic DNA
<400> 154
   ggtcaaatgt aacc 14
<210> 155
   <211> 14
   <212> DNA
   <213> Artificial Sequence
<220>
   <223> Description of Artificial Sequence:Synthetic DNA
<400> 155
   ccaccccttt gacc 14
<210> 156
   <211> 14
   <212> DNA
   <213> Artificial Sequence
<220>
   <223> Description of Artificial Sequence:Synthetic DNA
<400> 156
   aaggccactt gacc 14
<210> 157
   <211> 14
   <212> DNA
   <213> Artificial Sequence
<220>
   <223> Description of Artificial Sequence:Synthetic DNA
<400> 157
   ccaccccttt cacc 14
<210> 158
   <211> 15
   <212> DNA
   <213> Artificial Sequence
<220>
   <223> Description of Artificial Sequence:Synthetic DNA
<400> 158
   ccaccccgtt tcacc 15
<210> 159
   <211> 14
   <212> DNA
   <213> Artificial Sequence
<220>
   <223> Description of Artificial Sequence:Synthetic DNA
<400> 159
   caattggttt cacc 14
<210> 160
   <211> 14
   <212> DNA
   <213> Artificial Sequence
<220>
   <223> Description of Artificial Sequence:Synthetic DNA
<400> 160
   ccaccccgtc aact 14
<210> 161
   <211> 14
   <212> DNA
   <213> Artificial Sequence
<220>
   <223> Description of Artificial Sequence:Synthetic DNA
<400> 161
   agtgtgcgtc aact 14
<210> 162
   <211> 14
   <212> DNA
   <213> Artificial Sequence
<220>
   <223> Description of Artificial Sequence:Synthetic DNA
<400> 162
   ccaccccttt agcc 14
<210> 163
   <211> 15
   <212> DNA
   <213> Artificial Sequence
<220>
   <223> Description of Artificial Sequence:Synthetic DNA
<400> 163
   ccaccccttt tagcc 15
<210> 164
   <211> 14
   <212> DNA
   <213> Artificial Sequence
<220>
   <223> Description of Artificial Sequence:Synthetic DNA
<400> 164
   caactgtttt agcc 14
<210> 165
   <211> 14
   <212> DNA
   <213> Artificial Sequence
<220>
   <223> Description of Artificial Sequence:Synthetic DNA
<400> 165
   ccacccctgt aacc 14
<210> 166
   <211> 14
   <212> DNA
   <213> Artificial Sequence
<220>
   <223> Description of Artificial Sequence:Synthetic DNA
<400> 166
   ccaccccttt aacc 14
<210> 167
   <211> 14
   <212> DNA
   <213> Artificial Sequence
<220>
   <223> Description of Artificial Sequence:Synthetic DNA
<400> 167
   ctacccctgt aacc 14
<210> 168
   <211> 14
   <212> DNA
   <213> Artificial Sequence
<220>
   <223> Description of Artificial Sequence:Synthetic DNA
<400> 168
   ccaccccttt aacc 14
<210> 169
   <211> 14
   <212> DNA
   <213> Artificial Sequence
<220>
   <223> Description of Artificial Sequence:Synthetic DNA
<400> 169
   ccacccctat aacc 14
<210> 170
   <211> 14
   <212> DNA
   <213> Artificial Sequence
<220>
   <223> Description of Artificial Sequence:Synthetic DNA
<400> 170
   ggcaaatgat aacc 14
<210> 171
   <211> 15
   <212> DNA
   <213> Artificial Sequence
<220>
   <223> Description of Artificial Sequence:Synthetic DNA
<400> 171
   ccaccccctt aaacc 15
<210> 172
   <211> 15
   <212> DNA
   <213> Artificial Sequence
<220>
   <223> Description of Artificial Sequence:Synthetic DNA
<400> 172
   agggagtggt aaacc 15
<210> 173
   <211> 20
   <212> DNA
   <213> Artificial Sequence
<220>
   <223> Description of Artificial Sequence:Synthetic DNA
<400> 173
   cgtacggcga tagggacacc 20
<210> 174
   <211> 20
   <212> DNA
   <213> Artificial Sequence
<220>
   <223> Description of Artificial Sequence:Synthetic DNA
<400> 174
   ggcatatatc atcactggcg 20
<210> 175
   <211> 14
   <212> DNA
   <213> Artificial Sequence
<220>
   <223> Description of Artificial Sequence:Synthetic DNA
<400> 175
   ccaccccttt aacc 14

## Description

The present invention concerns polynucleic acids isolated from a porcine reproductive and respiratory syndrome virus (PRRSV), polypeptides encoded by the polynucleic acids, the use of such polypeptides in the manufacture of vaccines which protect pigs from a PRRSV and methods of making the polypeptides and polynucleic acids.

Porcine reproductive and respiratory syndrome (PRRS), a new and severe disease in swine, was first reported in the U.S.A. in 1987, and was rapidly recognized in many western European countries (reviewed by Goyal, J. Vet. Diagn. Invest., 1993, 5:656-664; and in U.S. Application Serial Nos. 08/131,625 and 08/301,435). The disease is characterized by reproductive failure in sows and gilts, pneumonia in young growing pigs, and an increase in preweaning mortality (Wensvoort et al., Vet. Q., 13:121-130, 1991; Christianson et al., 1992, Am. J. Vet. Res. 53:485-488; U.S. Application Serial Nos. 08/131,625 and 08/301,435).

The causative agent of PRRS, porcine reproductive and respiratory syndrome virus (PRRSV), was identified first in Europe and then in the U.S.A. (Collins et al., 1992, J. Vet. Diagn. Invest., 4:117-126). The European strain of PRRSV, designated as Lelystad virus (LV), has been cloned and sequenced (Meulenberg et al., 1993, Virology, 192:62-72 and J. Gen. Virol., 74:1697-1701; Conzelmann et al., 1993, Virology, 193:329-339).

PRRSV was classified within a single genus arterivirus in the new virus family of *Arteriviridae,* which includes equine arteritis virus (EAV), lactate dehydrogenase-elevating virus (LDV) and simian hemorrhagic fever virus (SHFV) (Plagemann and Moennig, 1992, Adv. Virus. Res., 41:99-192; Godeny et al., 1993, Virology, 194:585-596; U.S. Application Serial Nos. 08/131,625, 08/301,435 and Cavanaugh D., 1997, Arch. Virol. 142:629-633). This group of single plus-strand RNA viruses shares many characteristics such as genome organization, replication strategy, morphology and macrophage tropism (Meulenberg et al., 1993; U.S. Application Serial Nos. 08/131,625 and 08/301,435). Subclinical infections and persistent viremia with concurrent antibody production are also characteristic histopathologic properties of the arteriviruses.

Antigenic, genetic and pathogenic variations have been reported among PRRSV isolates (Wensvoort et al., 1992, J. Vet. Diagn. Invest., 4:134-138; Mardassi et al., 1994, J. Gen. Virol., 75:681-685; U.S. Application Serial Nos. 08/131,625 and 08/301,435). Furthermore, U.S. and European PRRSV represent two distinct genotypes (U.S. Application Serial Nos. 08/131,625 and 08/301,435). Antigenic variability also exists among different North American isolates as well (Wensvoort et al., 1992). Marked differences in pathogenicity have been demonstrated not only between U.S. and European isolates, but also among different U.S. isolates (U.S. Application Serial Nos. 08/131,625, and WO 96/06619 A1).

The genomic organization of arteriviruses resembles coronaviruses and toroviruses in that their replication involves the formation of a 3'-coterminal nested set of subgenomic mRNAs (sg mRNAs) (Chen et al., 1993, J. Gen. Virol. 74:643-660; Den Boon et al., 1990, J. Virol., 65:2910-2920; De Vries et al., 1990, Nucleic Acids Res., 18:3241-3247; Kuo et al., 1991, J. Virol., 65:5118-5123; Kuo et al., 1992; U.S. Application Serial Nos. 08/131,625 and 08/301,435). Partial sequences of several North American isolates have also been determined (U.S. Application Serial Nos. 08/131,625 and 08/301,435; Mardassi et al., 1994, J. Gen. Virol., 75:681-685).

The genome of PRRSV is polyadenylated, about 15 kb in length and contains eight open reading frames (ORFs; Meulenberg et al., 1993; U.S. Application Serial Nos. 08/131,625 and 08/301,435). ORFs 1a and 1b probably encode viral RNA polymerase (Meulenberg et al., 1993). ORFs 5, 6 and 7 were found to encode a glycosylated membrane protein (E), an unglycosylated membrane protein (M) and a nucleocapsid protein (N), respectively (Meulenberg et al., 1995). ORFs 2 to 4 appear to have the characteristics of membrane-associated proteins (Meulenberg et al., 1993; U.S. Application Serial No. 08/301,435). The ORFs 2 to 4 of LV encode virion-associated proteins designated as GP₂, GP₃ and GP₄, respectively (Van Nieuwstadt et al, 1996, 70:4767-4772).

The major envelope glycoprotein of EAV encoded by ORF 5 may be the virus attachment protein, and neutralizing monoclonal antibodies (MAbs) are directed to this protein (de Vries, J. Virol, 1992; 66:6294-6303; Faaberg, J. Virol, 1995; 69:613-617). The primary envelope glycoprotein of LDV, a closely related member of PRRSV, is also encoded by ORF 5, and several different neutralizing MAbs were found to specifically immunoprecipitate the ORF 5 protein (Cafruny et al., Vir. Res., 1986; 5:357-375). Therefore, it is likely that the major envelope protein of PRRSV encoded by ORF 5 may induce neutralizing antibodies against PRRSV.

Several hypervariable regions within the ORF5 were identified and were predicted to be antigenic (U.S. Application Serial Nos. 08/131,625 and 08/301,435). It has been proposed that antigenic variation of viruses is the result of direct selection of variants by the host immune responses (reviewed by Domingo et al., J. Gen. Virol, 1993, 74:2039-2045). Thus, these hypervariable regions are likely due to the host immune selection pressure and may explain the observed antigenic diversity among PRRSV isolates.

The M and N proteins of U.S. PRRSV isolates; are highly conserved (U.S. Application Serial No. 08/301,435). The M and N proteins are integral to preserving the structure of PRRSV virions, and the N protein may be under strict functional constraints. Therefore, it is unlikely either that (a) the M and N proteins are subjected to major antibody selection pressure or that (b) ORFs 6 and 7, which are likely to encode the M and N proteins, are responsible for or correlated to viral virulence. Interestingly, however, higher sequence variation of the LDV M protein was observed between LDV isolates with differing neurovirulence (Kuo et al., 1992, Vir. Res. 23:55-72).

ORFs 1a and 1b are predicted to translate into a single protein (viral polymerase) by frameshifting. ORFs 2 to 6 may encode the viral membrane associated proteins.

The various strains of PRRSV continue to be characterized (Halbur et al., J. Vet. Diagn. Invest. 8:11-20 (1996); Meng et al., J. Vet. Diagn. Invest. 8:374-381 (1996); Meng et al., J. Gen. Virol. 77:1265-1270 (1996); Meng et al., J. Gen. Virol. 76:3181-3188 (1995); Meng etal., Arch. Virol. 140:745-755 (1995); Halbur et al., Vet. Pathol. 32:200 204 (1995); Morozov et a]., Arch. Virol. 140:1313-1319 (1995); Meng et al., J. Gen Virol. 75:1795-1801 (1994); Halbur et al., J. Vet. Diagn. Invest. 6:254-257 (1994), all of which are incorporated herein by reference in their entireties).

PRRSV isolates ISU-12 (3 x plaque purified) and ISU-55 were deposited at the American Type Culture Collection, 12301 Parklawn Drive, Rockville, Maryland 20852, USA on 30 October 1992 and 29 September 1993 respectively under accession nos. VR-2385 and VR-2430. ISU-55 is of low pathogenicity and produces only 10-25% consolidation of lung tissues.

PRRSV is an important cause of pneumonia in nursery and weaned pigs. PRRSV causes significant economic losses from pneumonia in nursery pigs (the exact extent of which are not fully known). Reproductive disease was the predominant clinical outcome of PRRSV infections during the past few years, due to the early prevalence of relatively low virulence strains of PRRSV. Respiratory disease has now become the main problem associated with PRRSV, owing to the increasing prevalence of relatively high virulence strains of PRRSV. A need is felt for a vaccine to protect against disease caused by the various strains of PRRSV.

Surprisingly, the market for animal vaccines in the U.S. and worldwide is larger than the market for human vaccines. Thus, there exists an economic incentive to develop new veterinary vaccines, in addition to the substantial public health benefit which is derived from protecting farm animals from disease.

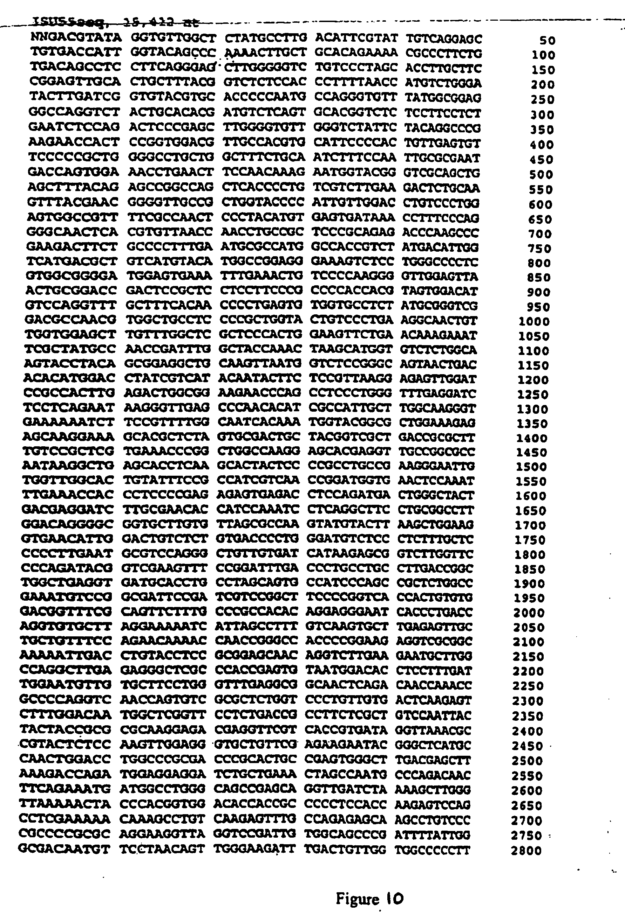

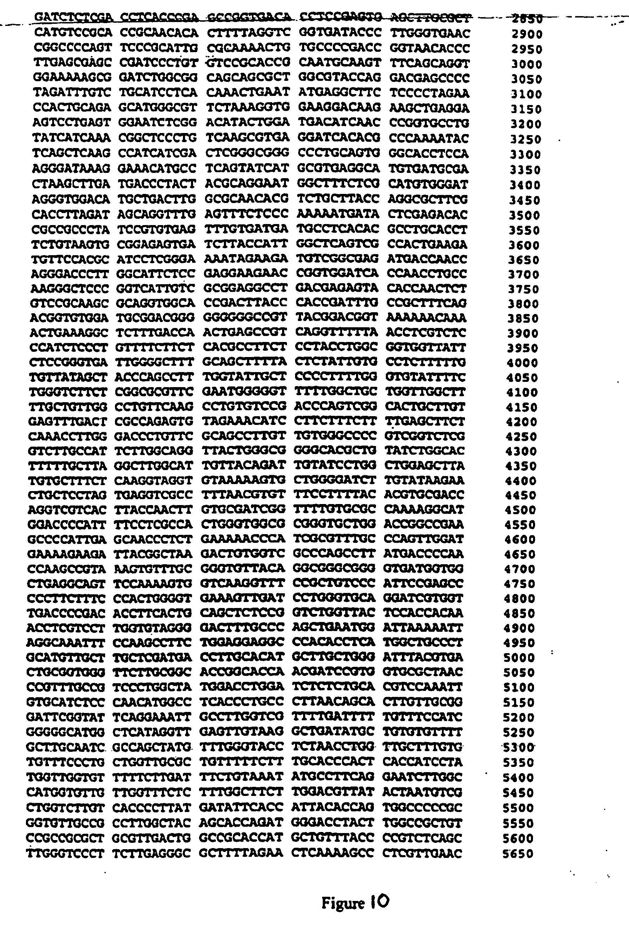

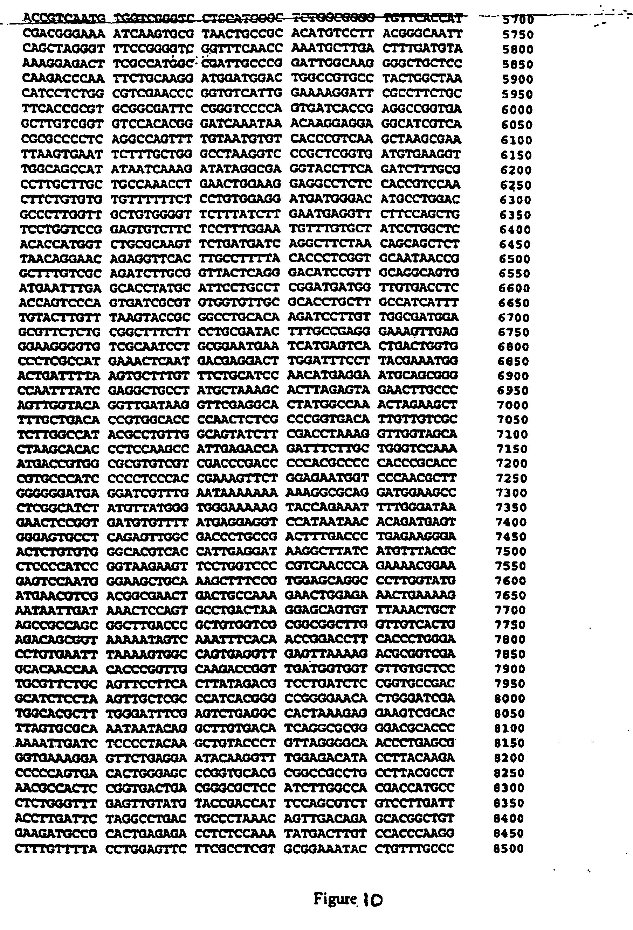

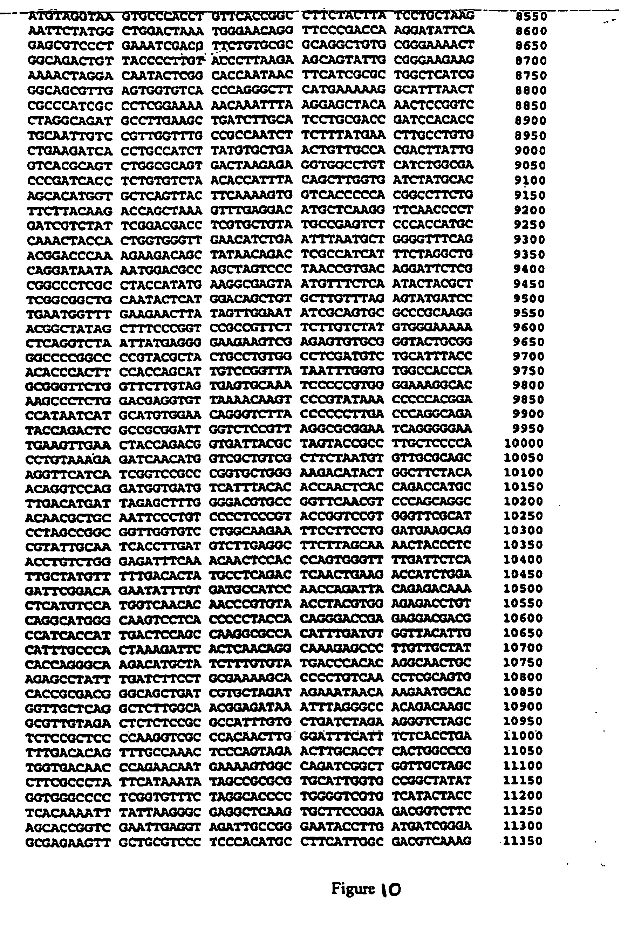

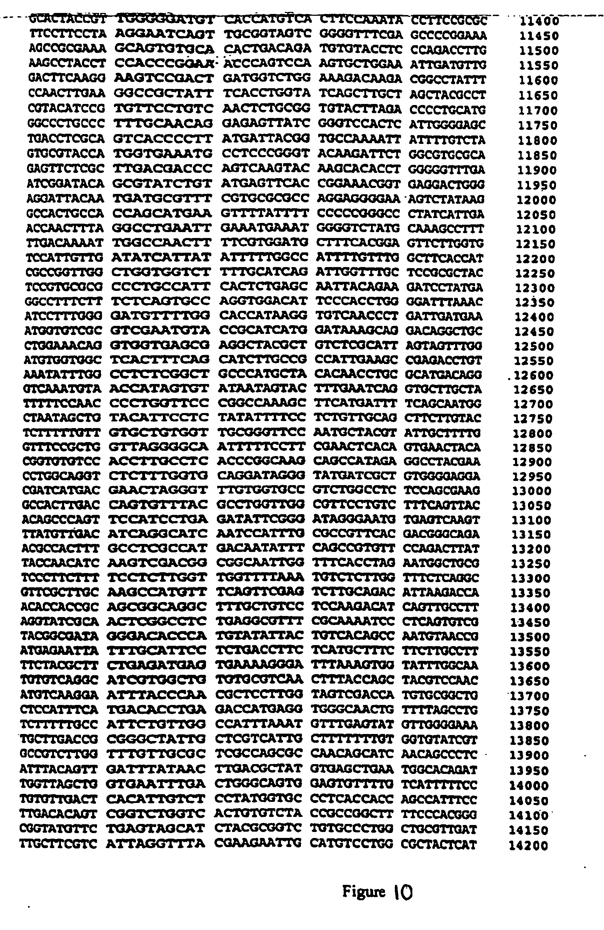

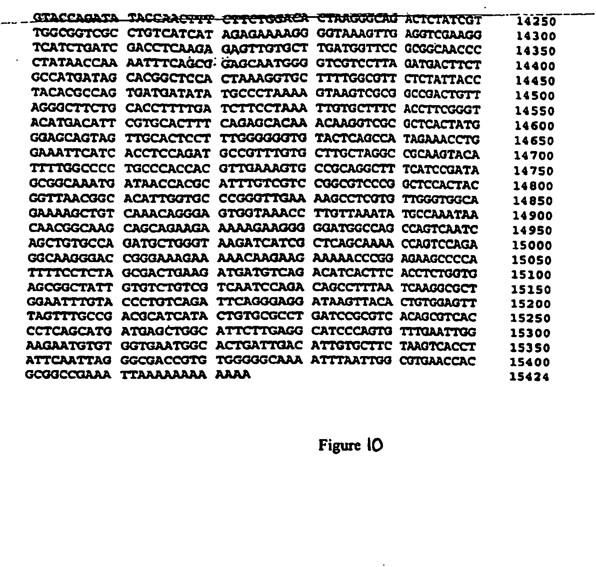

According to one aspect of the present invention there is provided an isolated DNA sequence including a porcine reproductive and respiratory syndrome virus (PRRSV) consisting of the nucleotide sequence of high passage ISU-55 depicted in Figure 10.

In another aspect of the present invention there is provided an isolated DNA sequence comprising an open reading frame of the PRRSV of claim 1, which open ready frame is selected from nucleotides 191-7699 of the nucleotide sequence of high passage ISU-55 depicted in Figure 10 (ORF 1a), nucleotides 7687-12069 of the nucleotide sequence of high passage ISU-55 depicted in Figure 10 (ORF 1b), nucleotides 12074-12841 of the nucleotide sequence of high passage ISU-55 depicted in Figure 10 (ORF 2), nucleotides 12697-13458 of the nucleotide sequence of high passage ISU-55 depicted in Figure 10 (ORF-3), nucleotides 13242-13775 of the nucleotide sequence of high passage ISU-55 depicted in Figure 10 (ORF 4) and nucleotides 13789-14388 of the nucleotide sequence of high passage ISU-55 depicted in Figure 10 (ORF 5).

The present invention further provides a composition for inducing antibodies against PRRSV comprising amount of PRRSV encoded by the nucleotide sequence of high passage ISU-55 depicted in Figure 10 (ISU-55) effective to induce said antibodies in a pig and a physiologically acceptable carrier.

In a different aspect, the present invention provides a method of distinguishing PRRSV strain ISU-55 from other strains of PRRSV comprising:
(a) amplifying a DNA sequence of the PRRSV using the following two primers:
   55F 5'-CGTACGGCGATAGGGACACC-3' and
   3RFLP 5'-GGCATATATCATCACTGGCG-3'
(b) digesting the amplified sequence of step (a) with DraI; and
(c) correlating the presence of three restriction fragments of 626 bp, 187 bp and 135 bp with a PRRSV ISU-55 strain

Following is a description by way of example only with reference to the accompanying drawings of embodiments of the present invention.

In the drawings:
Fig. 1 shows 20 overlapping cDNA clones sequenced from the VR 2385 cDNA library.
Fig. 2 shows the DNA alignment of the leader sequence of VR 2385 and LV.

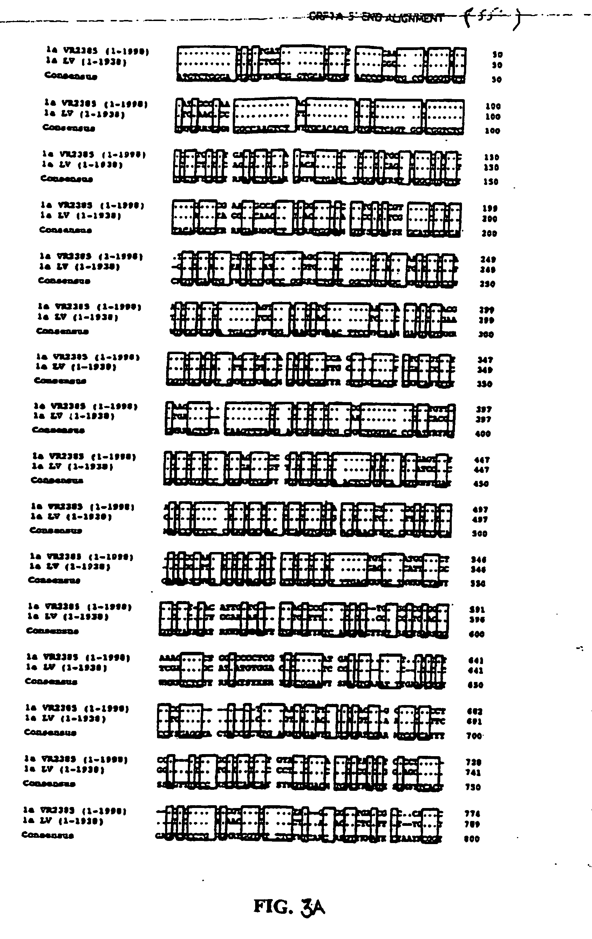

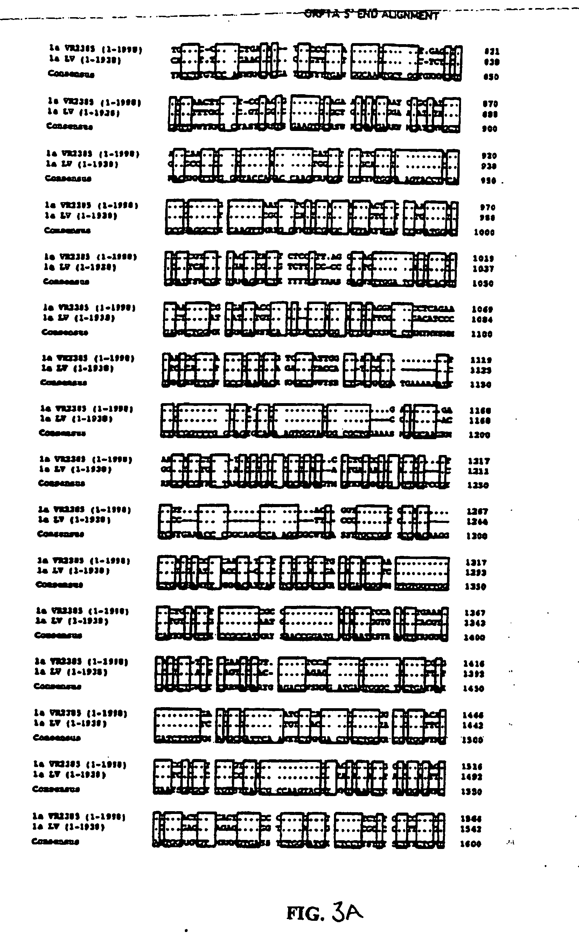

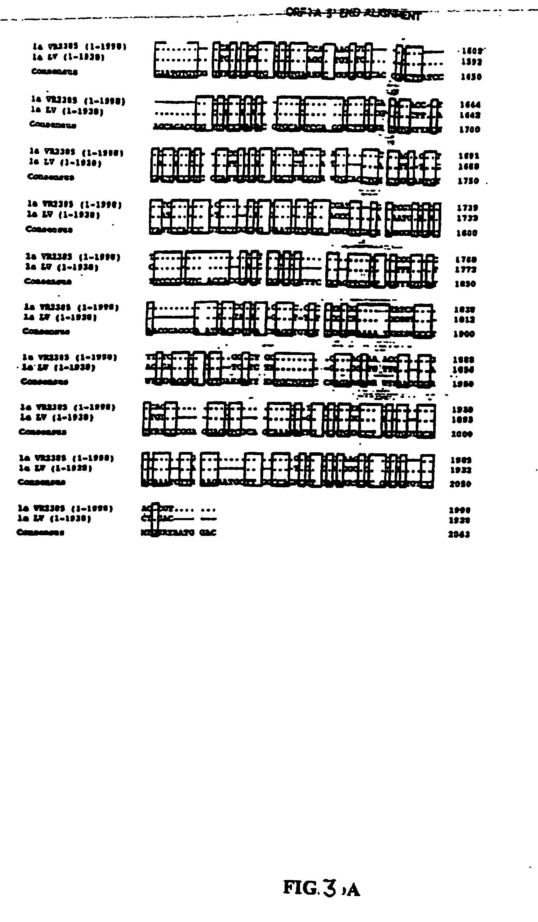

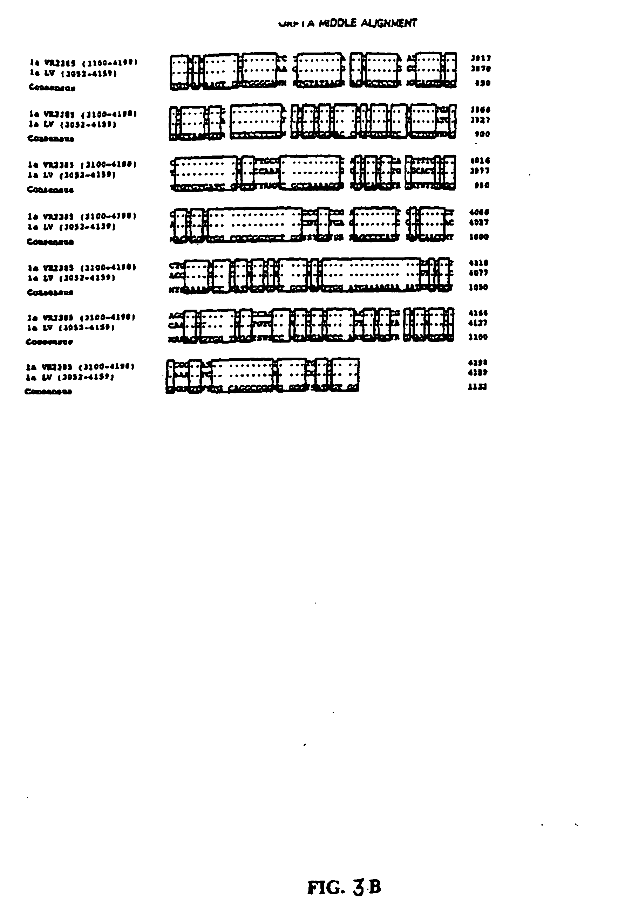

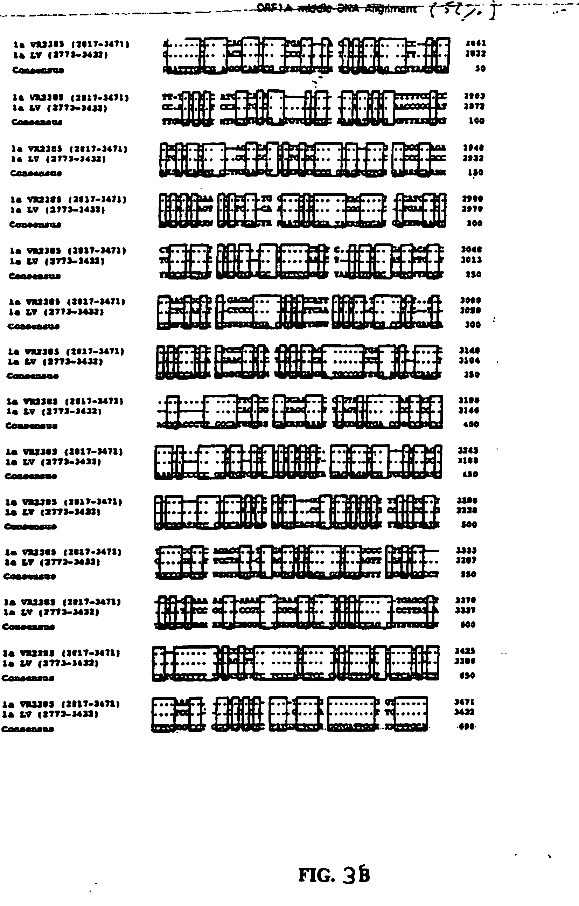

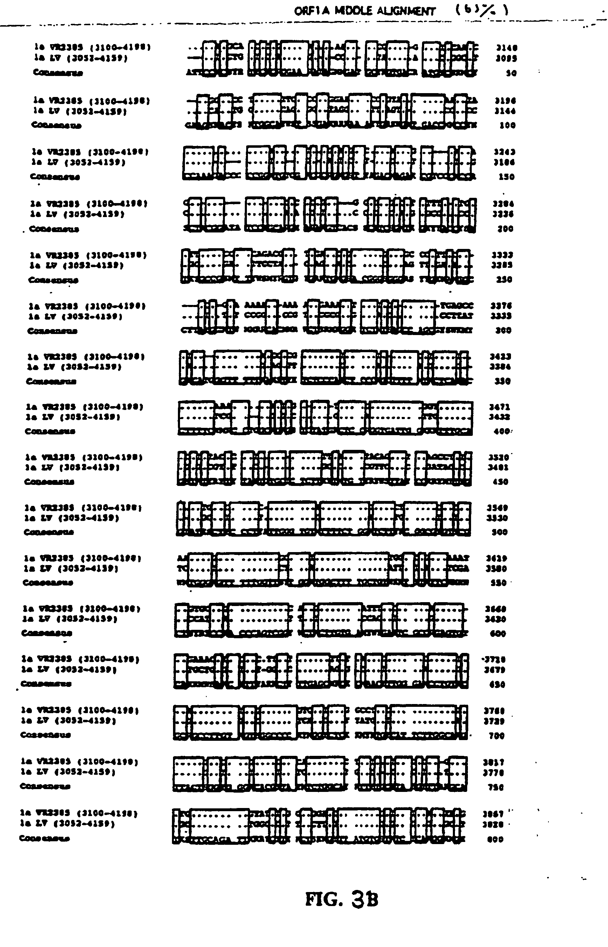

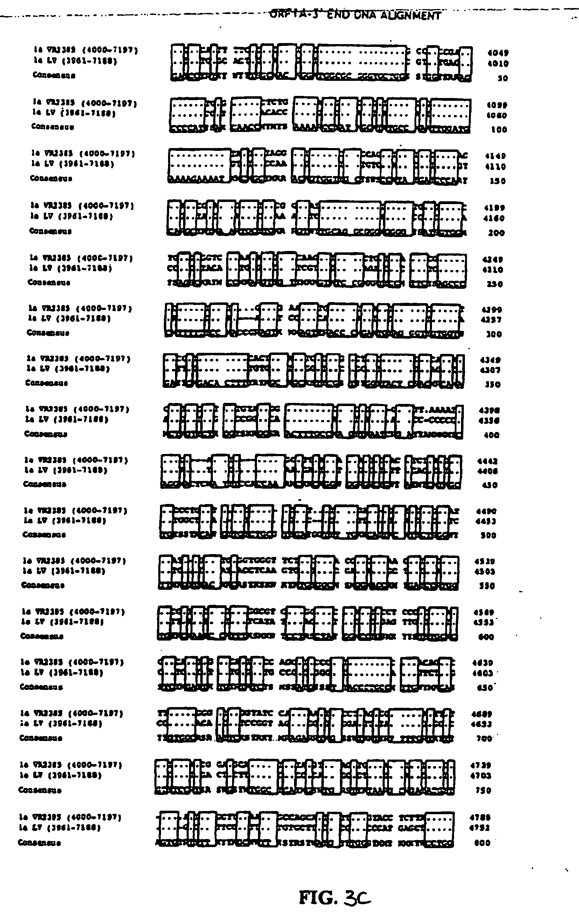

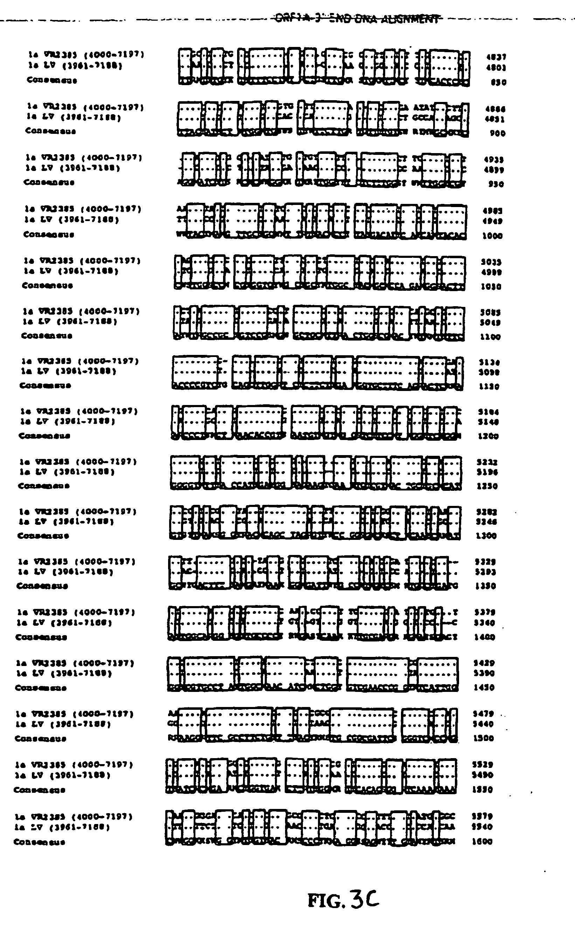

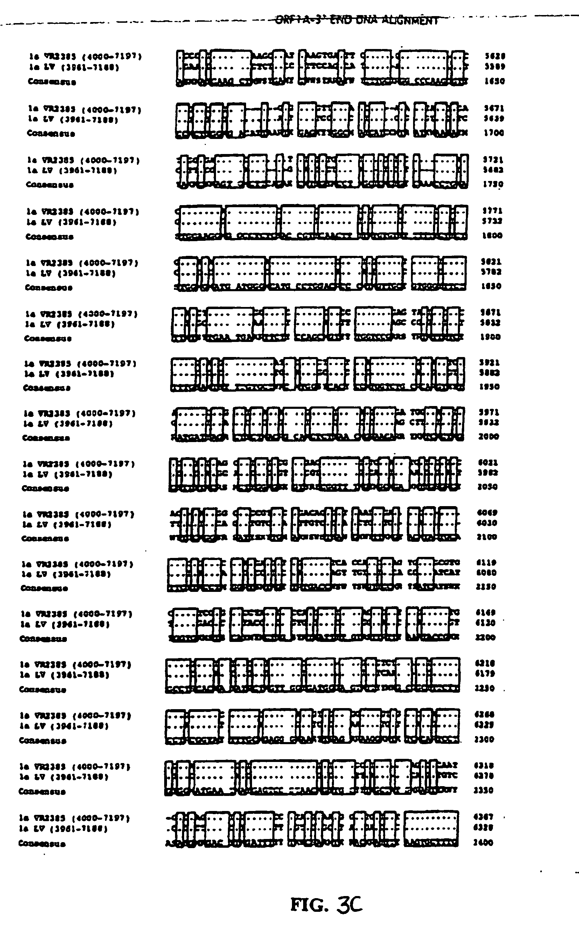

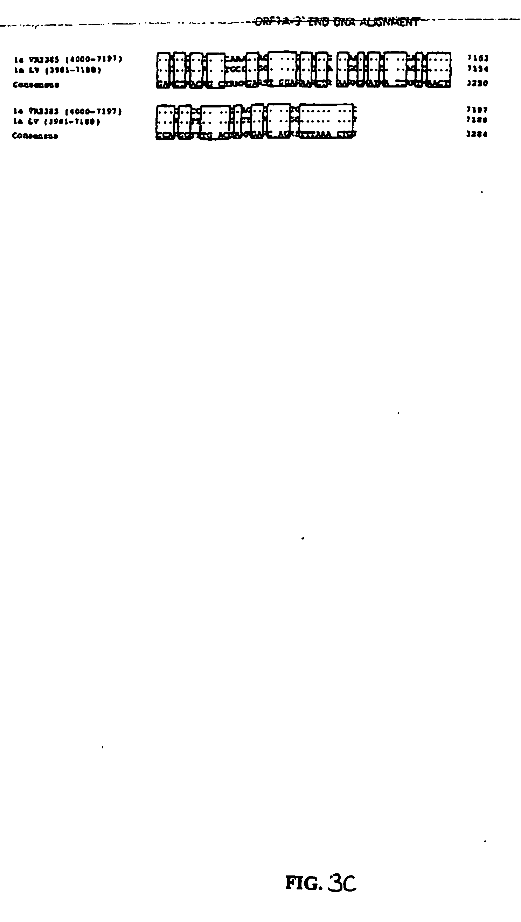

Fig. 3 shows alignments of ORF1a of VR 2385 and LV. Fig. 3A shows the 5' end alignment.
Fig. 3B shows the middle DNA alignment. Fig. 3C shows the 3' end alignment.
Fig. 4 shows the results of nested RT PCR with leader and ORF specific primers to amplify PCR products corresponding to mRNAs 4a, 5a and 7a.
Fig. 5 shows the DNA sequence alignment of low passage and high passage ISU 55.
Fig. 6 shows the ORF maps of ISU-55 high passage and low passage strains.
Fig. 7 is a restriction map showing the addition DraI site in the sequence of the high passage ISU-55 strain.
Fig. 8 shows the results of a RFLP test on total RNA isolated from ISU-55 hp, ISU-121p and ISU-12hp strains and used in RT PCR with primers 55F and 3RFLP.
Fig. 9 shows a genomic map and list of ORFs of ISU-55hp.
Fig. 10 shows the nucleotide sequence of ISU-55.

In the present invention, a "porcine reproductive and respiratory syndrome virus" or "PRRSV" refers to a virus which causes the diseases PRRS, PEARS, SIRS, MSD and/or PIP (the term "PIP" now appears to be disfavored), including the Iowa strain of PRRSV, other strains of PRRSV found in the United States strains of PRRSV found in Canada (e.g., IAF-exp91), strains of PRRSV found in Europe (e.g., Lelystad virus, PRRSV-10), and closely-related variants of these viruses which may have appeared and which will appear in the future.

The "Iowa strain" of PRRSV includes (a) PRRSV isolates deposited in the American Type Culture Collection by the present inventors and/or described in this application and/or in either of prior U.S. Application Serial Nos. 08/131,625 and 08/301,435.

A vaccine is effective if it protects a pig against infection by a porcine reproductive and respiratory syndrome virus (PRRSV). A vaccine protects a pig against infection by a PRRSV if, after administration of the vaccine to one or more unaffected pigs, a subsequent challenge with a biologically pure virus isolate (e.g., VR 2385) results in a lessened severity of any gross or histopathological changes (e.g., lesions in the lung) and/or of symptoms of the disease, as compared to those changes or symptoms typically caused by the isolate in similar pigs which are unprotected (i.e., relative to an appropriate control). More particularly, a vaccine may be shown to be effective by administering the vaccine to one or more suitable pigs in need thereof, then after an appropriate length of time (e.g., 1-4 weeks), challenging with a large sample (10³⁻⁷ TCID₅₀) of a biologically pure PRRSV isolate. A blood sample is then drawn from the challenged pig after about one week, and an attempt to isolate the virus from the blood sample is then performed.

Isolation of the virus is an indication that the vaccine may not be effective, and failure to isolate the virus is an indication that the vaccine may be effective.

Thus, the effectiveness of a vaccine may be evaluated quantitatively (i.e., a decrease in the percentage of consolidated lung tissue as compared to an appropriate control group) or qualitatively (e.g., isolation of PRRSV from blood, detection of PRRSV antigen in a lung, tensil or lymph node tissue sample by an immunoperoxidase assay method etc.). The symptoms of the porcine reproductive and respiratory disease may be evaluated quantitatively (e.g., temperature/ fever), semi-quantitatively (e.g., severity of respiratory distress or qualitatively (e.g., the presence or absence of one or more symptoms or a reduction in severity of one or more symptoms, such as cyanosis, pneumonia, heart and/or brain lesions, etc.).

An unaffected pig is a pig which has either not been exposed to a porcine reproductive and respiratory disease infectious agent, or which has been exposed to a porcine reproductive and respiratory disease infectious agent but is not showing symptoms of the disease. An affected pig is one which shows symptoms of PRRS or from which PRRSV can be isolated.

The clinical signs or symptoms of PRRS may include lethargy, respiratory distress, "thumping" (forced expiration), fevers, roughened haircoats, sneezing, coughing, eye edema and occasionally conjunctivitis. Lesions may include gross and/or microscopic lung lesions, myocarditis, lymphadenitis, encephalitis and rhinitis. In addition, less virulent and non-virulent forms of PRRSV and of the Iowa strain have been found, which may cause either a subset of the above symptoms or no symptoms at all. Less virulent and non-virulent forms of PRRSV can be used according to the present invention to provide protection against porcine reproductive and respiratory diseases nonetheless.

The phrase "polynucleic acid" refers to RNA or DNA, as well as mRNA and cDNA corresponding to or complementary to the RNA or DNA isolated from the virus or infectious agent. An "ORF" refers to an open reading frame, or polypeptide-encoding segment, isolated from a viral genome, including a PRRSV genome. In the present polynucleic acid, an ORF can be included in part (as a fragment) or in whole, and can overlap with the 5'- or 3'-sequence of an adjacent ORF

A "polynucleotide" is equivalent to a polynucleic acid, but may define a distinct molecule or group of molecules (e.g., as a subset of a group of polynucleic acids).

Features of the invention will become apparent in the course of the following descriptions of exemplary embodiments, which are given for illustration of the invention, and are not intended to be limiting thereof.

### EXAMPLE 1

Porcine Reproductive and Respiratory Syndrome Virus (PRRSV), Modified Live Virus vaccine was prepared as a lyophilized viral cake and reconstituted with sterile water and administered by either the subcutaneous (SC) or intramuscular (IM) route. The objective of this study was to confirm the immunogenicity of a PRRSV vaccine in three week-old swine by vaccinating either IM or SC with one 2 mL dose. Also to be determined was whether the PRRSV vaccine was safe and efficacious in three week-old pigs vaccinated with a single 2 mL dose, given with IM or SC, in protecting pigs against challenge with a virulent PRRSV strain, ISU-12.

### Animal Selection

Seventy crossbred PRRSV seronegative pigs (IDEXX ELISA sample to positive ratio of <0.4) were purchased from Evergreen Partners, Morris, MN and utilized in this study. All pigs were three weeks old at the time of vaccination.

### Composition of the Vaccine

The PRRSV vaccine comprising virus strain ISU-55 was produced at virus passage level X+5. The vaccine was stored between 2°-7°C prior to use. The vaccine was titrated in five replicates.

### Vaccination Schedule - Efficacy Testing

The stock vaccine was prepared by reconstituting the lyophilized virus portion with sterile water. The stock vaccine was diluted to the minimum protective dose level (approximately 10⁴ TCID₅₀ per dose) in culture medium. A representative aliquot of the prepared vaccine was retained at -70°C for quantitation of viral antigen. The 70 PRRSV seronegative susceptible pigs used in this study were randomly distributed into four treatment groups and vaccinated as follows:

| Group | Vaccine | Route | Dose | Number | Vaccination |
|---|---|---|---|---|---|
| Group A | PRRSV vaccine | IM | 2 mL | 20 pigs | Vaccination at 3 weeks of age. |
| Group B | PRRSV vaccine | SC | 2 mL | 20 pigs | Vaccination at 3 weeks of age. |
| Group C (Controls) | N/A* | N/A | N/A | 20pigs | N/A |
| Group D (Controls) | N/A | N/A | N/A | 10 pigs | N/A |

| | | | | | |
|---|---|---|---|---|---|
| *N/A - Not applicable | | | | | |

Injection sites were in the right neck (IM) or in the right flank fold (SC). The control pigs (Groups C and D) were not vaccinated with any vaccine or placebo vaccine.

Prior to vaccination, all pigs were bled for a prevaccination serology. Control animals were bled prior to challenge to ensure that they remained seronegative to PRRSV (IDEXX ELISA S/P ratio <0.4).

### Challenge and Observation Procedure

Thirty-six (36) days after the vaccination, each of the 20 pigs in Groups A, B and C were commingled in a common isolation room and challenged with virulent PRRSV. Group D animals were left as nonchallenged controls. The virulent ISU-12 PRRSV challenge virus was obtained from Iowa State University, Arnes, Iowa. The virulent ISU-12 PRSV challenge virus was maintained as a frozen (-70°C) stock after expansion in PSP36 cells. Individual pigs were challenged intranasally with 2 mL of the challenge virus. The PRRSV challenge stock was thawed and diluted to 10⁴ TCID₅₀ per 2 mL just before challenge. The challenge virus was held on ice during challenge. An aliquot of the challenge virus preparation was retained and held at -70°C for subsequent titration on PSP36 cells. The animals were observed on -1, and 0 days post challenge (DPC) to establish a baseline and 1 to 10 DPC for various clinical signs.

### Clinical Observation

The pigs were evaluated each day for post challenge clinical signs such as inappetence, lethargy, depression, diarrhea, neurological symptoms, dyspnea, cyanosis and death.

### Lung Lesion Scoring

The lungs of each individual pig were examined for gross lesions at necropsy 10 days post challenge. The scorer of gross lung lesions was blinded to the identity of the treatment group to which each pig belonged. Briefly, the score for lung lesions in each lobe were recorded by estimating the percent of the lobe exhibiting PRRSV-like lesions (based on color and texture) and multiplied by the number of points possible for that lobe. Maximum score for each lobe was determined by the relative percentage of the total lung volume occupied by the lobe. Then the scores from the dorsal and ventral aspects of all lobes were added to obtain the total score for each pig. The maximum total score possible for each animal was 100.

### Statistical Analysis

The clinical sign and gross lung lesion scores for the vaccinates and the controls were compared using analysis of variance (General Linear Model). The use of analysis of variance models using nonranked gross lung lesion scores was justified by the fit of the scores within a normal probability distribution. A comparison of the residuals of the parametric analysis indicated they were distributed normally, substantiating the major assumptions for analysis of variance. Therefore, data analysis using ranked gross lung lesion scores was not necessary. All statistical analyses were performed on an IBM computer using SAS software.

### RESULTS AND DISCUSSION

### PRRSV Antigen Titers in the VS Code Vaccine

The PRRSV vaccine antigen titration results are shown in Table 1. The average PRRSV titer per dose of vaccine from five replicate titrations was 10^{3.92} TCID₅₀.

### Clinical Observations

Following vaccination, there were no clinical signs observed in any of the vaccinated pigs. Following challenge with virulent PRRSV ISU-12 p6, the vaccinates and control pigs did not show significant clinical signs of respiratory or neurologic disease during the 10 day post challenge observation period.

### Gross Lung Lesion Pathology

The results of gross lung lesion scoring are given in Table 2. Following PRRSV challenge, the gross lung lesion scores ranged from 0-29 with a mean score of 14.15 in the IM vaccinated pigs (Group A), from 1-27 with a mean score of 11.20 in the SC vaccinated pigs (Group B), from 7-57 with a mean score of 25-80 in the nonvaccinated challenge control pigs (Group C), and 1-28 with a mean score of 10.90 in the non-vaccinated nonchallenged control pigs (Group D). Both IM and SC vaccinated pigs had significantly less lung lesions than the nonvaccinated challenged control pigs (p<0.05). The vaccinated pigs did not have significantly different gross lung lesion scores than the gross lung lesion scores from nonvaccinated nonchallenged pigs (P>0.05). The nonvaccinated challenged control pigs had significantly higher gross lung lesions than the nonvaccinated nonchallenged control pigs (P<0.05).

### CONCLUSION

The results of the study demonstrate that Porcine Reproductive and Respiratory Syndrome Virus, Modified Live Virus Vaccine is efficacious for use in healthy pigs three weeks of age or older as an aid in the prevention of respiratory disease caused by virulent PRRSV challenge. One hundred percent of the three week-old pigs vaccinated with the modified live vaccine did not show any adverse local or systemic clinical effects following vaccination. These pigs remained healthy and active for the entire 36 day post vaccination observation period. Pigs vaccinated with a dose of 10^{3.92} TCID₅₀ vaccine either intramuscularly or subcutaneously showed significant reduction (p<0.05) in gross lung lesion development over nonvaccinated challenged control pigs following challenge with a heterologous virulent PRRSV challenge strain, ISU-12. The post challenge gross lung lesion scores of vaccinated pigs were statistically indistinguishable from the nonvaccinated nonchallenged controls (p>0.05). Analysis of the residuals of the parametric analysis of variance indicated that they were distributed normally, substantiating the major assumptions for analysis of variance. One hundred percent of the vaccinated pigs remained free of clinical signs during the post challenge period.

**Table 1 PRRSV Immunogenicity Study: PRRSV Antigen Level of Vaccine***

| Replicate Titration Number | Viral Titer per 2 mL dose |
|---|---|
| 1 | 10^{3.80} |
| 2 | 10^{3.93} |
| 3 | 10^{4.13} |
| 4 | 10^{3.93} |
| 5 | 10^{3.80} |
| Average | 10^{3.92} |

| | |
|---|---|
| * in log TCID₅₀ | |

**Table 2. PRRSV Immunogenicity Study: PRRSV Gross Lung Lesion Scoring 10 DPC**

| | Group | | | |
|---|---|---|---|---|
| Pig Number | A IM Vaccinates | B SC Vaccinates | C Non Vaccinated Challenged Controls | D Non Vaccinated Non Challenged Controls |
| 1 | 21 | 7 | 53 | 4 |
| 2 | 1 | 12 | 7 | 8 |
| 3 | 19 | 5 | 57 | 1 |
| 4 | 12 | 17 | 12 | 2 |
| 5 | 29 | 3 | 18 | 3 |
| 6 | 5 | 18 | 35 | 11 |
| 7 | 18 | 19 | 20 | 24 |
| 8 | 6 | 5 | 28 | 14 |
| 9 | 4 | 7 | 32 | 28 |
| 10 | 0 | 8 | 41 | 14 |
| 11 | 16 | 3 | 27 | |
| 12 | 12 | 27 | 19 | |
| 13 | 9 | 16 | 40 | |
| 14 | 29 | 19 | 10 | |
| 15 | 17 | 1 | 24 | |
| 16 | 20 | 13 | 15 | |
| 17 | 26 | 12 | 9 | |
| 18 | 21 | 14 | 9 | |
| 19 | 6 | 13 | 35 | |
| 20 | 12 | 5 | 25 | |
| Mean | 14.15 | 11.20 | 25.80 | 10.90 |
| Standard Deviation | 8.86 | 6.84 | 14.47 | 9.3 |

### EXAMPLE 7

### Complete sequence of PRRSV isolate VR 2385

### Materials and methods.

Virus and Cells. The PRRSV isolate VR2355, passage 7 was used in this study. A continuous cell line, ATCC CRL11171 was used for growth of the virus and isolation of viral RNA and total RNA from the virus-infected cell culture.

Cloning of cDNA and PCR amplification. For characterization of the ORF I region' of genome of VR2385 a random cDNA λ library was constructed using the Uni-Zap cDNA cloning kit (Stratagene, La Jolla, CA). Briefly, the CRL11171 cells were infected with VR2385 virus at a M.O.I. of 0.1 and the total RNA from infected cells was isolated at 24 hrs post infection by using a guanidinium thiocyanate method. Initially, probe specific for 5' end of ORF2 was used to screen the random cDNA library. Plaques that hybridized with the probe were isolated and purified. The phagemids containing viral cDNA inserts were rescued by *in vitro* excision using ExAssist helper phage and *E*. *coli* SOLR cells (Stratagene, LaJolla, CA). After hybridizations with ORF1-specific overlapping fragments, several recombinant phagemids with virus specific cDNA inserts with sizes ranging from 2 to 6 kb were selected. The plasmids containing virus cDNA inserts were subsequently purified and sequenced by Sanger's dideoxynucleotide chain termination method with an automated DNA sequencer (Applied Biosystems, Foster City, CA). Universal, reverse and PRRSV-specific internal primers were used to determine the sequence. At least 2 independent cDNA clones representing sequence of ORFs 1a and 1b were sequenced. One region, not represented in the library (nt 1950 -2050) was PCR amplified with primers IM687 (5'-CCCCATTGTTGGACC TCTCC-3') and IM2500(5'-GTCACAACAGGGACCGAGC-3') using Tag DNA polymerase with addition of the proofreading Tag Extender (Stratagene). The sequencing data were assembled and analyzed using MacVector (International Biotechnologies, Inc., CT) and GeneWorks (IntelliGenetics, CA) computer programs.

Primer extension experiments and RNA sequencing. Primer extension experiments were performed using SureScript Preamplification System for First Strand cDNA Synthesis (Gibco BRL). ³²P-labeled oligonucleotide RNS (5'-CCAAGCTCCCCTGAAGGAGGCTGT CAC-3') was mixed with 0.5 µg of viral RNA of VR2385 in total volume of 12 µl and RNA was denatured for 10 min at 90°C. The sample was adjusted to a total volume 19 µl with first strand cDNA buffer and incubated for 5 min at 42 °C for primer annealing. Super Script II reverse transcriptase was then added to the reaction and the reaction mixture was incubated at 42°C or 50°C for min. Samples were analyzed in 40% polyacrylamide gel. Primer extension products were run next to the sequencing reactions of pPR59 clone, containing partial sequence of the leader. Oligonucleotide RNS served as a primer for the sequencing reaction.

Direct sequencing of purified viral RNA was performed using RT RNA Sequencing Kit (USB, Cleveland, Ohio) with γ³²P-labeled oligonucleotide RNS (5'-CCAAGCTCCCCTGAAGGAGGCT GTCAC-3') and 151Ext (5'-AGCATCCC-AGACATGGTTAAAGGGG-3'). Sequencing was performed according to the manufacturer's instructions using 0.5 µg of purified viral RNA per sequencing reaction.

### Results.

**Leader sequence of PRRSV VR2385.** Previously, oligo dT and random cDNA libraries of PRRSV VR2385 in λZap vector and here constructed the sequence for portion of ORF1b and complete ORFs-2-7 were determined. The partial leader sequence of VR2385, 161 nucleotides upstream of the ATG start codon of ORF1, was obtained from clone pPR59. It has been shown previously that the leader sequence of LDV is 156 nucleotides, and that the leader sequence of LV (a European isolate of PRRSV) was 221 nucleotides. In order to determine the complete leader sequence of U.S. PRRSV, primer extension experiments were performed. In one experiment cDNA was synthesized using SuperScript II reverese transcriptase at 42°C and 50°C. In another experiment rTth DNA polymerase in the presence of Mn was used for cDNA synthesis at 60°C to minimize potential of secondary structures in leader RNA during cDNA synthesis. In all experiments the length of generated cDNA fragments were the same, about 190 nucleotides. In order to detect the complete leader sequence of PRRSV VR2385, direct sequencing of viral RNA was performed. Virion RNA isolated from virus purified through sucrose gradient was used in a direct RNA sequencing reaction. Direct RNA sequencing was performed with a primer complementary to the leader sequence at positions between 10 and 67 nt upstream of the AUG start codon of ORF1a. In addition to the 161 nt leader sequence previously detected by screening of the cDNA library with leader specific probe, an additional 27 nucleotides of the leader sequence were identified. The two nucleotides at the extreme 5' end of the leader could not be identified due to the strong bands observed in all four lanes in the sequencing gel. The size of the leader determined by direct RNA sequencing correlated with results of the primer extension experiments. To further confirm the data obtained by direct RNA sequencing, RT-PCR was performed with a 16 b.p. primer, corresponding to the extreme 5' end of the leader, and an antisense primer located 10 nt upstream of the 3' end of the leader. An expected 180 b.p. PCR fragment was amplified which is in agreement with the results obtained by direct RNA sequencing. Therefore, the putative size of the leader of PRRSV VR2385 was 190 nt, which is smaller than those reported for LV (221 nt), EAV (212 nt) and SHFV(208 nt), but larger than the leader sequence reported for LDV (156 nt). The sequence of the junction region at the 3' end of the leader was TTTAACC. The ATG start codon of ORF1a is located immediately downstream of this sequence. Similar results were also reported for LV, LDV and SHFV, in which the start codon of ORF1a is also located after the junction sequence. However, the genome of EAV leader junction sequence was reported 13 nt upstream of the start codon of ORF1a. The percentage of nucleotide sequence identity between the leader sequence of VR2385 and those of LV, LDV and SHFV were 55%, 47% and 38%, respectively. Surprisingly, only the last 44 nucleotides at the 3' end of the leader of VR2385 possess significant homology with the leader sequence of LV (86% identity in this region). Relatively higher homology was also found in this 44 nt region between VR2385 and LDV (64%) and SHFV(63%). No significant homology was found between leader sequences of VR2385 and EAV.

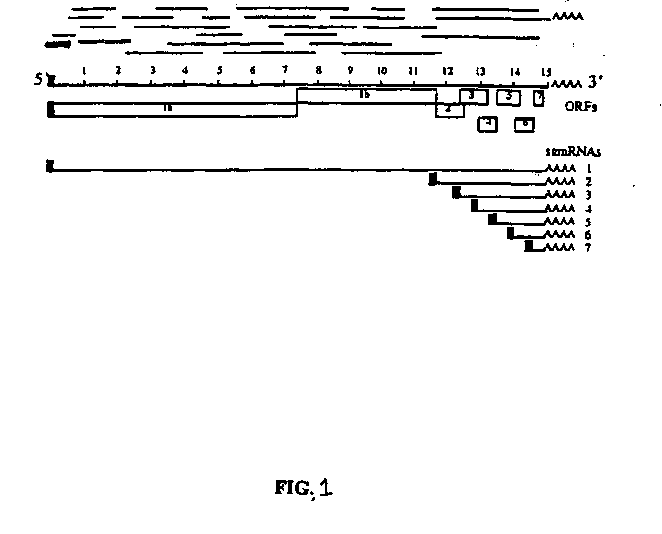

**Cloning and sequencing of PRRSV genome.** To analyze ORF1 of PRRSV VR 2385, a random primed cDNA library in λZap vector was constructed from total RNA of virus-infected cells. More than twenty overlapping cDNA clones from cDNA library were selected and sequenced (Fig.1). For most regions, the sequence was determined from at least two independent clones. The region corresponding to nucleotides 1900-2050 was not represented in the cDNA library, and this genomic region was PCR amplified and sequenced. Sequence analysis showed that the genomic RNA of PRRSV (U.S. isolate VR2385), excluding the polyA sequence, is 15100 nucleotides in length.

**Functional domains in ORFs 1a/1b and homology with related viruses.** The predicted size of ORF1a is 7197 nucleotides. It extends from nucleotides 191 to 7387 (excluding the stop codon TAG) and encodes a 2399 amino acid polyprotein. The leader-genome junction region is similar to that of LV, and the ATG start codon is located immediately after TTTAACC sequence of the leader. Differences were identified when compared the ORF1 sequences of LV and VR2385. ORF la in LV is 7188 nucleotides long and encodes 2396 amino acids, which is only 3 amino acids shorter than that of VR2385. Pairwise comparison of nucleotide sequences of VR2385 and LV indicated that the 5' end of ORF1a is more divergent than the 3' end. The nucleotide sequence 55% identities between VR2385 and LV is 61% in the 3' end of ORF 1a, (from nucleotides 3050 to 7387) in the first 1500 nucleotides of ORF 1a 55%, and 46% in a region between nucleotides 1500 to 2500. The most variable region within ORF1a was located between nucleotides 2500 and 3000, where there was no significant homology between VR2385 and LV. The amino acid identity was 49% for region from 1 to 530 aa, 55% for region from 1100 to 2399 amino acids, and no significant homology in the region extending from amino acids 530 to 1100. Comparison of the ORF1a sequences of VR2385 and LDV revealed that there is a 52% homology in first 2000 nucleotides and 55% homology in the last 3800 nucleotides of ORF 1a (corresponding to 3400-7197 nt in VR2385 and 2850-6678 nt in LDV). The region between 2000 to 3400 nt of VR2385 and 2500 to 2850 nt of LDV is highly variable with more than 500 nt deletion in LDV genome. Comparison of the predicted amino acid sequences showed that there is a 36% of homology for the region extended from amino acids 1 to 500, and 39% for the region, that includes the last 1300 amino acids of predicted proteins (1120 to 2353 aa in VR2385 and 940 to 2226 aa in LDV).

Analysis of the predicted protein encoded by ORF1a of VR2385 revealed the presence of two papain-like cysteine protease domains (aa 63-165 and aa 261-347) and one 3C-like serine protease domain (aa 1542-1644), similar to those described for other arteriviruses and coronaviruses. The hydrophilic profiles of ORF1a proteins of VR2385 were similar to those of LV and LDV. The 5' half of the proteins (first 1100 aa in VR2385) were mostly hydrophilic, the extreme 3' end (aa 2230-2399 in VR2385) was hydrophilic and the 3' half of the protein contains 4 hydrophobic regions (1129-1207 aa, 1240-1286 aa, 1478-1643 aa and 1856-2076 regions of VR2385).

The VR2385 ORF1b is 4389 nucleotides long and it extends from nucleotide 7369 to 11757 (excluding stop codon TGA), and encoded a 1463 aa protein. Comparison of the nucleotide and predicted amino acid sequences of VR2385 ORF1b with those of LV, LDV and EAV confirmed that ORF1b is more conserved than ORF1a. Nucleotide and amino acid homology between VR2385 and LV was 64 and 67% in ORF1b and 58 and 53% in ORF1a, respectively. Comparison of the predicted proteins of VR2385 and EAV showed a 36% homology. The predicted ORF1b protein of VR2385 contains a putative polymerase domain (amino acids 373-576), a putative zinc finger domain (amino acid 647-689), and an RNA helicase domain (amino acids 793-1015) similar to those described for LV, LDV, EAV and coronaviruses.

Molecular characterization of ORF1 regions of coronaviruses and arteriviruses showed that the ORF1 polyprotein is expressed through two overlapping ORFs, ORF1a and ORF1b. The expression of ORF1b, which overlaps with ORF1a in -1 frame, takes place through a so-called ribosomal frameshifting mechanism which allows the ribosome to bypass the ORF1a stop codon and translate ORF1b-encoded protein. The frameshift region consists of a "slippery sequence" followed by pseudoknot structure. Analysis of the ORF1a/ORF1b junction region of VR2385 indicated that the potential slippery sequence (5'-UUUAAAC-3') is located 3 nucleotides upstream of the stop codon of ORF1a and the proposed pseudoknot structure. This region is very conserved in corona- and arteriviruses and the nucleotide sequence homology in this region between VR2385 and LV was 86%.

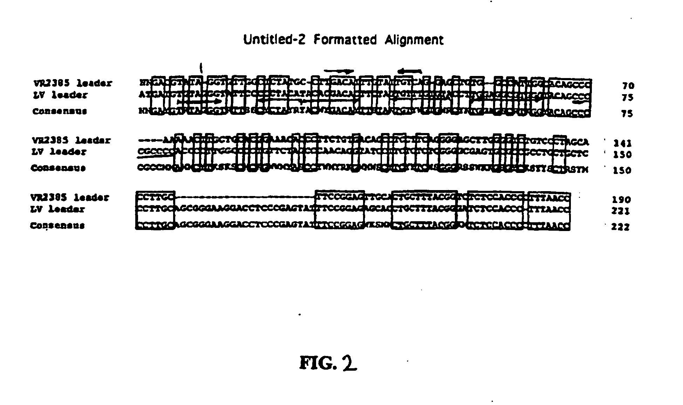

Comparison of the leader sequences of VR2385 and LV indicated that these two viruses diverged from each other by point mutations and possibly through recombination. The extensive sequence differences in the leader sequences of these two viruses indicated the leader that sequence in PRRSV is not conserved, and is subject to extensive mutational changes. The most conserved region in the leader was the last 44 nucleotides at the 3' end, where nucleotide sequence acid identity was 86% between VR2385 and LV, and 68% between VR2385 and LDV. The putative leader sequence of VR2385 was 190 nt, which is 31 nt shorter than that of LV, and 35 nt longer than that of LDV. As shown in Fig. 2 there is a 20 nt deletion in the VR2385 leader (located after nucleotide 145) compared to the leader sequence of the LV. Comparison of the leader sequences of VR2385 and LDV indicates that the highest homology score was obtained when a 20 nt gap was introduced into the corresponding region of the leader sequence of LDV (Fig.2. Similarly, the highest homology score was obtained when a 50 nt gap was introduced into the LDV leader during alignment of the LV and LDV leader sequences. This result suggests that this region of the leader is not critical for virus replication, and deletions may occur in this region of the leader during virus evolution. This observation also could explain the observed differences in the length leader sequences among VR 2385, LV and LDV.

### EXAMPLE 3

### Characterization of the leader sequence and leader-body junction sites in subgenomic mRNAs of PRRSV VR 2385

In order to determine the complete leader sequence of PRRSV VR2385, several approaches were utilized including screening of oligo dT cDNA library with leader-specific ³²P-labeled PCR probe, RNA ligation of the viral RNA (RNA circularization) with T4 RNA ligase followed by RT-PCR with ORF7 and leader specific primers, and direct sequencing of the 5' end of viral RNA (Example 2).First, a 100 b.p. fragment of leader sequence was used as a probe to detect cDNA clones containing the leader sequence from an oligo dT λ library. Eight cDNA clones were analyzed and sequence, and these clones were found to represent leader sequences of mRNAs 7 (5 clones), 6 (2 clones) and 2 (1 clone). The size of leader sequence varied from 160 to 163 nucleotides in 6 of the 7 clones. In one of the clones which represents mRNA6, the leader specific sequence was 172 nucleotides. It is possible that strong secondary structure within the leader of the virus prevented complete cDNA synthesis of the leader RNA during the construction of the λ Zap library. In a second experiment, the 3' and 5' ends of viral RNA were ligated head to tail by using T4 RNA ligase. After phenol chloroform extraction and precipitation, the ligated RNA was subjected to an RT-PCR reaction with primers IM1003 (antisense oligonucleotide, complementary to the 3' end of the leader sequence) and IM1004 (oligonucleotide, corresponding to a segment of the 3' non-coding region of the genome, 100 nucleotides upstream of the poly(A) tail). A diffuse band of the PCR products with sizes ranging from 250 to 350 nucleotides was purified from agarose gel, and cloned into the pSK + vector. Seven independent clones were sequenced. Sequence analysis indicated that the polyA sequence at the 3' end of the genome and the leader sequence at the 5' end of the genome were ligated together in all 7 clones, but only 95-96 nucleotides from the 3' end of the leader sequence were ligated with 3' end of the viral genome. The sizes of the polyA sequenced clones varied in each clone ranging from 9 to 42 nucleotides, indicating that sequenced clones were independent. The putative full-length leader sequence of VR2385 was determined by direct RNA sequencing of the 5'end, of virion RNA isolated from sucrose gradient purified virus (Example 2).

**Leader mRNA junction sequences and intergenic regions within the genome of VR2385.** In order to characterize leader body junction regions of sg RNAs of the VR2385 strain, RT-PCR was performed with leader specific primer and primers, specific for each sg mRNA. Total RNAs isolated at 20 hours post infection (h.p.i.) were used for RT-PCR. The predominant bands were isolated from agarose gel, cloned and sequenced. Direct sequencing of the PCR products was also performed. In order to identify leader body junction region in the genome of the PRRSV, a leader specific ³²P-labeled probe was used to screen a random cDNA library generated from viral RNA, and several clones containing leader -ORFla junction regions were isolated and sequenced. The leader body junction regions of sg mRNAs 2 to 7 were characterized.

Table 3 summarizes the leader-body junction regions of all sg mRNAs and their corresponding regions in the virus genome. Only a single junction site was detected for sg mRNAs 2,3, and 6, whereas two sites were detected for sg mRNAs 4, 5 and 7, designated as 4a, 4b, 5a, 5b and 7a, 7b. The leader genome junction region in VR2385 was represented by a sequence CCACCCCTTTAACC, which is similar to that of -

**Table 3 Sequence of the leader body junction regions of subgenomic mRNAs of VR2385**

| RNA | SEQUENCE | N of clones |
|---|---|---|
| 5'-leader | CCACCCCTTTAACC | 4 |
| mRNA2 | CCACCCCttgaacc | 3 |
| genome | cctgtcattgaacc | |
| mRNA3 | CCACCCCtgtaacc | 2 |
| | CCACCCCTTtaacc | 1 |
| genome | ggtcaaatgtaacc | |
| mRNA4a | CCACCCCTttgacc | 1 |
| genome | aaggccacttgacc | |
| mRNA4b | CCACCCCtttcacc | 2 |
| | CCACCCCgtttcacc | 1 |
| genome | caattggtttcacc | |
| mRNA5.a | CCACCCcgtcaact | 1 |
| genome | agtgtgcgtcaact | |
| mRNA5.b | CCACCCCtttagcc | 2 |
| | CCACCCCttttagcc | 1 |
| genome | caactgttttagcc | |
| mRNA6 | CCACCCCTgtaacc | 3 |
| | CCACCCCTTtaacc | 1 |
| genome | ctacccctgtaacc | |
| mRNA7.a | CCACCCCTTtaacc | 5 |
| | CCACCCCTataacc | 1 |
| genome | ggcaaatgataacc | |
| mRNA7.b | CCACCCCCTtaaacc | 1 |
| genome | agggagtggtaaacc | |

The leader body mRNAs junction regions varied in sg mRNAs 3, 4, 5b, 6 and 7a. Table 4 compares the intergenic regions in genomes of VR2385, LV, LDV and EAV. The intergenic regions of VR2385, LV and LDV are very similar. Most variations were found in the first three nucleotides of these regions, whereas the last four nucleotides are conserved and in most regions are represented by the sequence AACC. Variations were also found in the first two nucleotides of this junction sequence (GACC and CACC in the intergenic region of ORF4 of VR2385, AGCC in the intergenic region of ORF5b of VR2385, GACC in the intergenic region of ORF3 in LV, and ACC in the intergenic region of ORF2 of LDV). The intergenic region for sg mRNA5a of VR2385 is GUCAACU, which is similar to that of EAV.

**Table 4. Sequence of the 3' end of the leader in the genome (RNA1) and junction sites of subgenomic mRNAs 2 to 7 of VR2385, LV, LDV and EAV.**

| **RNA** | **VR2385** | **LV** | **LDV** | **EAV** |
|---|---|---|---|---|
| 1 | UUUAACC | UUUAACC | UAUAACC | AUCAACU |
| 2 | UUGAACC | GUAAACC | UAU-ACC | UUCAACU |
| 3.1 | UGUAACC | GUUGACC | UGUAACC | GUCAA-U |
| 3.2 | | | | AUCAACU |
| 3.3 | | | | AU-AAUU |
| 4a | CUUGACC | | | |
| 4b | UUUCACC | UUCAACC | UGUAACC | GUCAACU |
| 5.a | GUCAACU | | | |
| 5.b | UUUAGCC | UACAACC | UAUAACC | GUCAACU |
| 6 | UAUAACC | CUCAACC | UAAAACC | GUCAACC |
| 7.a | GAUAACC | | | |
| 7.b | GUAAACC | GUUAACC | CCUAACC | CUCAACU |

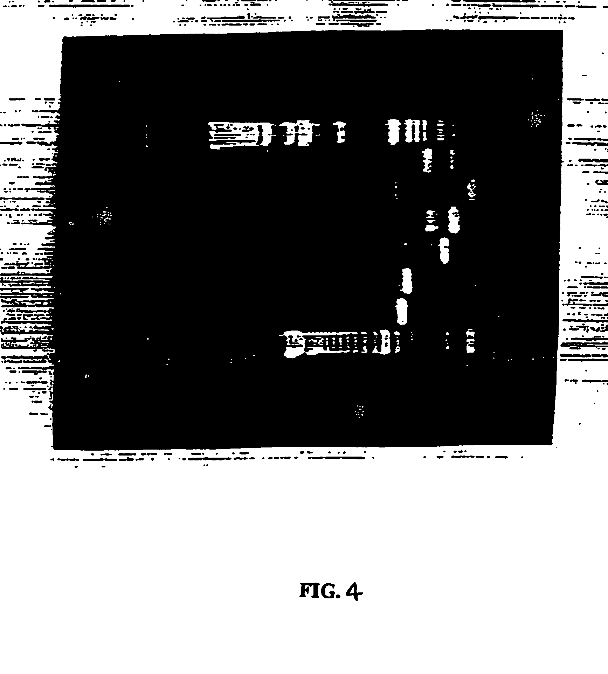

The position of intergenic sites upstream of the start codon of the corresponding ORFs vary from 4 to 231 nucleotides. Table 5 compares the location of intergenic sites in the genomes of VR2385 and LV (numbers represents distance in nucleotides between the intergenic site and AUG start codon of the corresponding ORF.) The locations of these sites in the genome of VR2385 and LV differ in sg mRNAs 3, 4, 5 and 6. Three alternative intergenic sites for the synthesis of sg mRNAs 4,5 and 7 of VR2385 genome were also identified. Previously, that only six bands of sg mRNAs were detected in the cells infected with VR2385 by Northern blot hybridization analysis. To confirm that the additional sg mRNAs are actually synthesized during the replication of VR2385, a nested RT-PCR was performed by using leader and ORF specific primers. The amplified PCR products were similar in sizes corresponding to additional mRNAs 4a 5a and 7a (Fig 4). The results indicated that the intergenic sites 4b and 5b of sg mRNAs 4 and 5 which is located closer to the start codon of the corresponding ORF were frequently used in sg mRNA synthesis. The sg mRNAs 4 and 5 were predominantly generated from intergenic sites 4b and 5b while only a minor population was generated by using alternative sites 4a and 5a. In the case of sg mRNA7 the integenic site 7a located 123 nt upstream of start codon of ORF7 was frequently used, whereas site 7b located 9 nt upstream of start codon of ORF7 was less involved in sg mRNA7 synthesis.

**Table 5. Location of the intergenic sites inside of the genome of VR2385 and LV.**

| RNA | Position of the junction site | |
|---|---|---|
| | **VR2385** | **LV** |
| RNA2 | 20 | 38 |
| RNA3 | 83 | 11 |
| RNA4 | 231 &4 | 83 |
| RNA5 | 157 & 40 | 32 |
| RNA6 | 17 | 24 |
| RNA7 | 123 &9 | 9 |

Comparison of the leader genome junction sequence with sequences of the intergenic regions and sequences of leader body junction regions in sg mRNAs indicated that only the last seven nucleotides of leader (TTTAACC) possess homology with the sequences of the intergenic regions in the genome of VR2385. The overall homology varies from 5 to 7 nt, and the only exception was sg mRNA6 where 11 out of 12 nt in the intergenic region are similar to the 3' end of the leader sequence. In the leader body junction regions of the sg mRNA, the CCACCCC sequence is conserved and generated from leader. The sequence following CCACCCC, however, varied for different sg mRNAs, but has a high level of homology with the TTTAACC sequence at the 3' end of the leader. The variations in the leader body junction sequences detected for different sg mRNAs indicates that leader body joining is imprecise. Nucleotide sequence comparisons between the 3' end of the leader, leader body junction regions of the sg mRNAs and intergenic regions within the genome of VR2385 allowed detection of regions of actual joining between leader and body of sg mRNA

**Conclusions.** The mechanism of subgenomic mRNA synthesis of U.S. isolates of PRRSV is similar to that of LV, LDV and EAV. Intergenic regions detected in VR2385 were more variable and were located at different sites when compared to LV. Variations in leader body junction sequences indicate that leader body joining is imprecise. The locations of actual leader body joining sites in sg mRNAs suggest that mechanism(s) other than leader priming may be involved in the synthesis of sg mRNAs. Alternative leader-body junction sites in the genomes of U.S. isolates of PRRSV can result in the variation of the number of sg mRNA among different strains of PRRSV.

### EXAMPLE 4

The following provides a reliable test for the identification and differentiation of high passage ISU55 strain of PRRSV from field isolates of PRRSV. In previous studies the sequence of the low passage ISU55 strain (passage 7) was determined and this sequence was used to develop an RFLP test for differentiation of ISU55 hp strain. As a first step, the sequence of ISU55 p-7 was analyzed to identify variable regions containing unique restriction sites. After computer sequence analysis and comparison with sequences of different PRRSV strains, a specific region containing two unique restriction sites was identified at the 3' end of ORF4. These two restriction sites were DraI (TTT/AAA) at position 1510 and Ball (MscI) (TGG/CCA) at position 1697 relative to the location upstream of the ATG start codon of ORF2 in ISU55 (p-7) sequence. These two restriction sites were present only in the corresponding region of ISU55 strain but not in the other PRRSV strains.

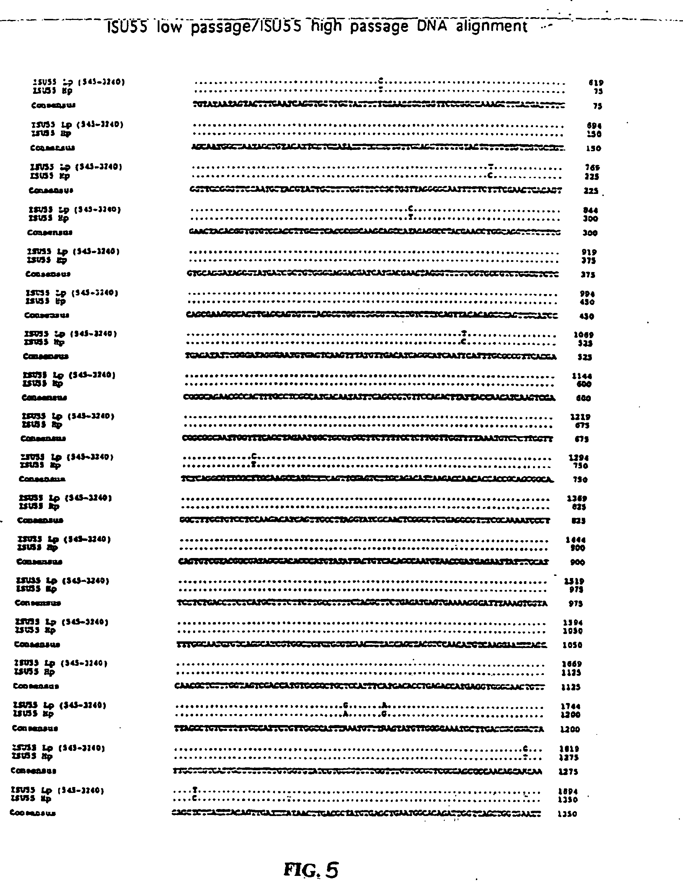

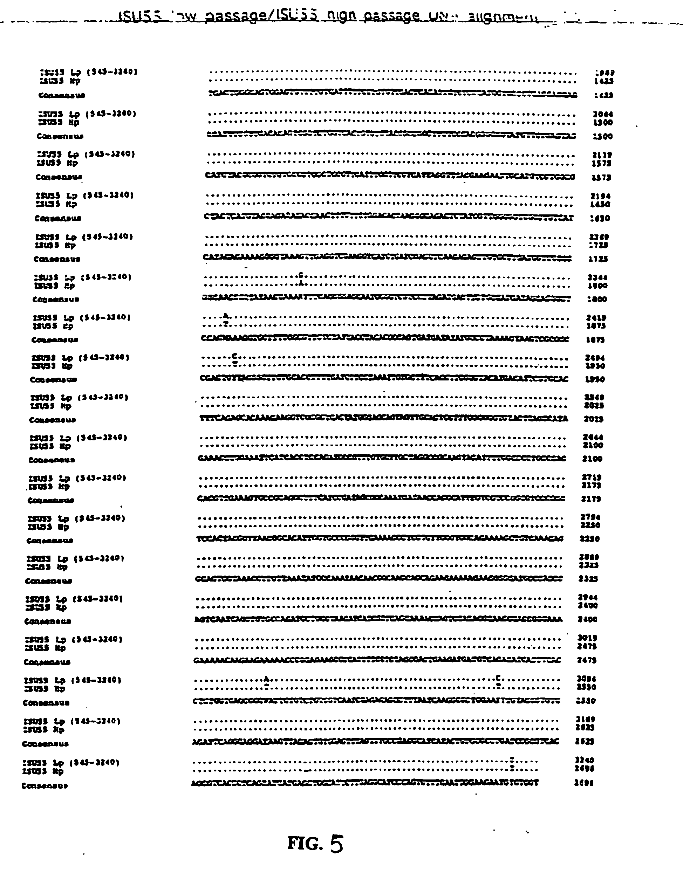

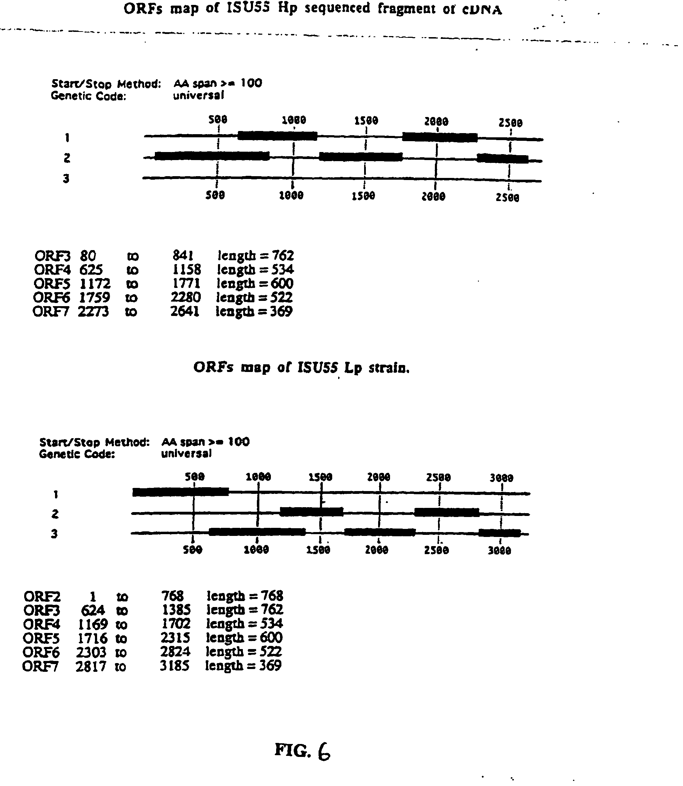

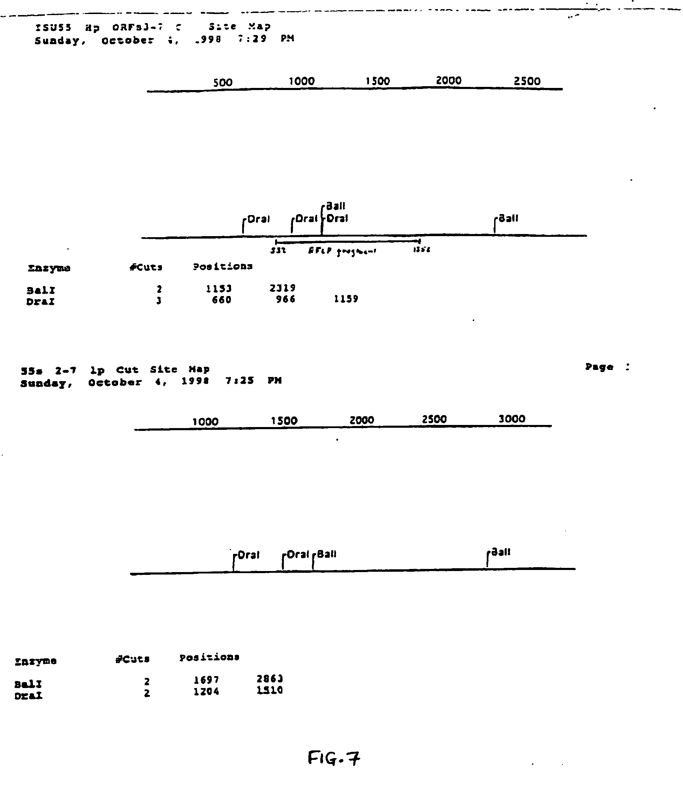

In order to confirm the results of the computer analysis, the sequence of high passage ISU55 strain was determined. The genomic region including ORFs 3 to 7 (2696 b.p.) was amplified by PCR and sequenced. The sequence of high passage ISU55 was compared with that of the ISU55 passage 7. The results of this comparison are shown in Fig. 5 (cDNA alignment), Fig. 6 (ORF maps) and Fig. 7 (restriction pattern with restriction enzymes DraI and BalI). The sequences of the low passage and high passage of PRRSV ISU55 were very conserved. There were only 15 nucleotide substitutions in high passage ISU55 strain. The sizes and relative positions of ORFs 3 to 7 remain the same. A single nucleotide change in the high passage virus created an additional DraI site in the sequence of the high passage virus compared to the low passage ISU55 (Fig. 7). This finding affords an opportunity to distinguish the high passage virus from the low passage ISU55 virus. A BLAST search was conducted to compare specific regions of ISU55 high passage strain with other PRRSV sequences available in the GenBank database. The 237b.p. fragment including the unique restriction sites of ISU 55 high passage strain (two DraI sites at position 966 and 1159 and BalI site at position 1157, restriction map of ISU55 hp.) was used as the template for comparison. The results of the blast search indicated that these sites are unique for ISU55 high passage strain and are not present in 24 other PRRSV isolates available in the database.

The ORF5 (603 bp.) of ISU55 high passage strain was also compared with the ORF5 of other PRRSV strains. As expected, ISU55 passage 7 strain has the highest homology score and there are only 3 nucleotide substitutions. The strain with the second highest homology score was NADC8 which has 33 nucleotide substitutions in ORF5 compared to the ORF5 of ISU55 hp strain. The rest of PRRSV strains compared in the BLAST search displayed more variations (up to 63 nt changes) in the ORF5. These data clearly indicate that ISU55 PRRSV strain is different from all other PRRSV strains characterized so far.

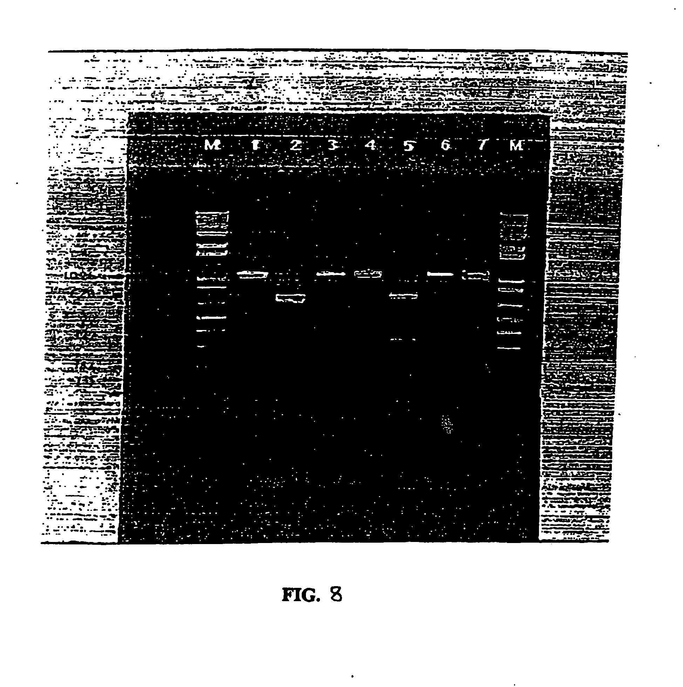

An PCR-RFLP was developed to differentiate ISU55 high passage strain from ISU55 lp virus and other strains of PRRSV. For the RFLP test two primers were synthesized: forward primer 55F 5'-CGTACGGCGATAGGGACACC-3' (pos. 823) and reverse primer 3RFLP 5'-GGCATATATCATCACTGGCG-3' (pos. 1838) (positions from the 5' end of 2696 bp. sequenced fragment of ISU55 high passage strain). The reverse primer for the PCR-RFLP test was the same as the one used in a PCR-RFLP test to differentiate MLV ResPRRSV vaccine strain since this primer has been used in the PCR-RFLP with a large number of PRRSV isolates and shown to be specific (Wesley et al, J. Vet. Diagn. Invest. 10:140-144 (1998); Wesley et al, Amer. Assoc. Of Swine Practitioners, pp. 141-143 (1996); Andreyev et al, Arch. Virol. 142:993-1001 (1997); Mengeling, et al, 1997, all of which are incorporated herein by reference in their entireties). These two primers amplify a 1026 bp cDNA fragment of PRRSV ISU55 high passage strain. After digestion with restriction enzyme with DraI three fragments (626 bp, 187 bp and 135 bp) will be generated. After digestion with BalI, two fragments with sizes 626 and 322 bp will be formed. After PCR and restriction enzyme digestions of other PRRSV strains, a 1026 bp fragment will be formed according to the analysis of computer data. To validate the PCR-RFLP test, total RNA was isolated from ISU55 hp, ISU12 1p, ISU12 hp strains and subjected to RT-PCR with primers 55F and 3RFLP. A 1026 bp fragment was amplified from all the isolates. These fragments were purified and digested with restriction enzymes DraI and BalI. The resulting products were analyzed in 1.5% agarose gel. Fig.8 shows the results of the test. Line one shows an untreated 1026 bp PCR fragment of ISU55hp strain. Line 2 and 5 shows PCR products of-ISU55 hp digested with DraI (line 2) and BalI (line 5). The 626 bp, 187 bp and 135 bp fragments were formed after digestion with DraI, and 626 and 322 bp. fragments were formed after digestion with BalI. Lines 3, 4, 6, and 7 show results of DraI digestion (lines 3 and 4) and BalI digestion (lines 6 and 7) of PCR products of ISU12 lp (lines 3 and 6) and ISU12 hp (lines 4 and 7) strains. In all reactions with ISU12 lp and hp strains a PCR fragment of 1026 bp was detected. These data correlate with the predictions for the PCR-RFLP differentiation test for the ISU55 hp strain.

### EXAMPLE 5

### Sequencing of the genome of the attenuated PRRSV Vaccine Strain (ISU55 p49).

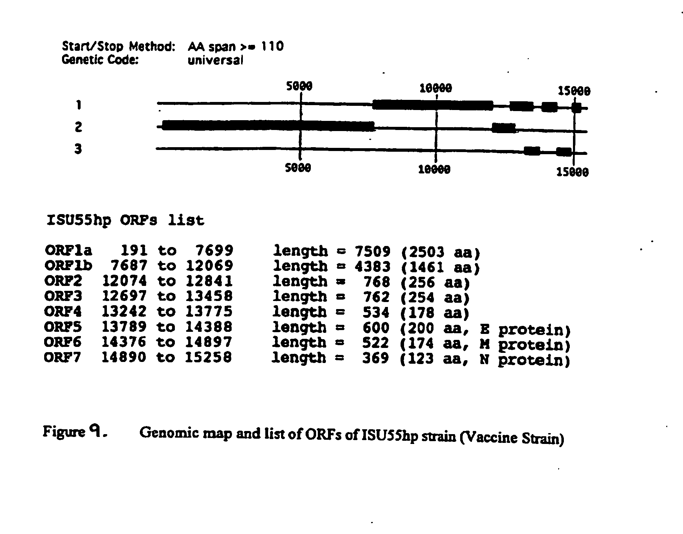

After the sequence of VR2385 strain was determined, generated sequencing information was used in order to determine sequence of the attenuated vaccine PRRSV strain (Vaccine Strain). The entire genome of Vaccine Strain was amplified in 21 overlapping fragments and sequenced. When sequencing data were combined, the entire size of the gnome was 15,412 nucleotides, which is 309 nt longer compare to the length of the genome of VR2385 strain. The ORFs map and their locations are shown on Figure 9 Genome comparison of Vaccine Strain and VR2385 strains showed the same sizes and relative locations of the ORFs 1b through ORF7. The ORF1a was the most variable and one 309 nucleotides deletion was found in the genome of VR2385 compared to the sequence of Vaccine Strain. This deletion was in frame and located in the region of ORF1a at position 3242 nucleotide from the 5' end of genome of VR2385 PRRSV. Another three nucleotides were deleted in the region 2504-2515 nt of the genome causing 1 aa deletion compare to the genome of Vaccine Strain. Results of genome comparison of different ORFs of Vaccine Strain and VR2385 strain are summarized in the Table 6. Overall DNA homology between these strains was about 91 % with 14094 nucleotides identical in both strains. Not including 309 nt deletion DNA homology was 93%. Leader sequence was determined only for VR2385 strain by direct sequencing of the viral RNA and 17 bp primer specific for 5' end of the leader of VR2385 was used to amplify 170 nt portion of the leader of Vaccine Strain. Comparison of these sequences showed overall homology of 94% with single nucleotides deletions in both strains: nucleotides A (pos. 75) and G (pos. 119) of VR2385 leader are missing in the leader of Vaccine Strain, and nucleotides A (pos. 87) and G (pos. 124) of Vaccine Strain leader are missing in the leader of VR2385 strain.

The ORF1a in the Vaccine Strain extends from nts 191 to 7699 and encodes 2503 amino acid (aa) protein, which is 103 aa longer compare to the ORF 1a protein of VR2385 strain. Overall aa identity in between ORF1a predicted proteins of the Vaccine Strain and VR2385 was 88% (92% not including deletion in VR2385). Comparison with ORF1a protein of LV showed approximately 47% of aa identity overall, but several regions with different protein similarity can be identified. Relatively conservative 5' end (aa 1 to 529 in Vaccine Strain and aa 1 to 521 in LV, 50% aa identity), relatively conservative 3' end (aa 1232 to 2503 in Vaccine Strain and aa 1115 to 2396 in LV, 58% aa identity), and hypervariable region (HVR) in between (aa 530 to 1231 in Vaccine Strain and aa 522 to 1114 in LV, aa identity less than 40%). When we studied homology in HVR in more details, we were able to detect one short region (94 aa), where aa homology was 50% between Vaccine Strain and LV. This region extends from aa 1015 to 1108 in the Vaccine Strain and aa 929 to 1021 in LV. Interestingly, in exception of the first four aa (ITRK) this region was deleted in VR2385 strain. To summarize, homology in the ORF 1a predicted protein can be presented as follows: conservative region 1 (aa 1 to 529 in the vaccine Strain/VR2385 strain, aa 1 to 521 in LV, 90% a identity between Vaccine Strain and VR2385, 50% aa identity between Vaccine/VR2385 strains and LV), hypervariable region (HVR) (aa 530 to 1231 in the Vaccine Strain, aa 520 to 1127 in the VR2385 strain, aa 522 to 1232 in LV, 84% aa identity between Vaccine Strain and VR2385, 103 aa deletion in the ORF la protein of VR2385, less then 40% aa identity between Vaccine/VR2385 strains and LV), and conservative region 2 (aa 1232 to 2503 in the Vaccine Strain, aa 1128 to 2399 in the VR2385 strain, aa 1128 to 2396 in LV, 96% aa identity between Vaccine Strain and 57 % aa identity between Vaccine/VR2385 strains and LV). The 94 amino acid fragment (aa 929 to 1021) in the HVR of the Vaccine Strain posses 50% aa homology with LV, and this region is deleted in VR2385 strain.

The ORF 1b in the Vaccine Strain extends from nts 7687 to 12069 and encodes 1461 aa protein, which is similar in size to that of VR2385. Nucleotide and amino acid comparison showed, that ORF1b is much more conservative compare to ORF1a. Nucleotide homology between Vaccine Strain and VR2385 was 93 %, with 97% homology of their predicted proteins. Comparison with ORF1b of LV (1462 aa) showed 67 % of aa identity. One variable region was detected at the 3' end of ORF1b (aa 1367-1461) compare ₁₀ LV.

The ORF2 to ORF7 region of the vaccine strain showed similar genome organization to that of VR2385, with similar sizes and relative locations of the ORFs. Data of homology comparison between Vaccine Strain and VR2385 are presented in the Table 6, Nucleotide (amino acid) identity of Vaccine Strain with LV was 66% (61 %) for ORF2, 61% (55%) for ORF3, 66 % (67%) for ORF4, 63% (51%) for ORF5. 68% (79%) for ORF6, and 60 % (58%) for ORF7.

**Table 6. Comparison of the ORFs and DNA homology between VR2385 p8, ISU55 p49 (Vaccine Strain) and LV**

| ORF | Size of the ORF (nucleotides) | | | Homology with VR2385:nt(aa) | |
|---|---|---|---|---|---|
| | VR2385 | ISU55 | LV | ISU55 | LV |
| 1a | 7197 | 7509 | 7188 | 89(88) | |
| 1b | 4383 | 4383 | 4386 | 93(97) | 64(67) |
| 2 | 768 | 768 | 747 | 97(95) | 65(60) |
| 3 | 762 | 762 | 795 | 94(95) | 64(55) |
| 4 | 534 | 534 | 549 | 96(97) | 66(66) |
| 5 | 600 | 600 | 603 | 93(90) | 63(54) |
| 6 | 522 | 522 | 519 | 97(98) | 68(78) |
| 7 | 369 | 369 | 384 | 96(94) | 60(57) |

## Claims

1. An isolated DNA sequence encoding a porcine reproductive and respiratory syndrome virus (PRRSV) consisting of the nucleotide sequence of high passage ISU-55 depicted in Figure 10.

2. An isolated DNA sequence comprising an open reading frame of the PRRSV of Claim 1, selected from which open reading frame is nucleotides 191-7699 of the nucleotide sequence of high passage ISU-55 depicted in Figure 10 (ORF 1a), nucleotides 7687-12069 of the nucleotide sequence of high passage ISU-55 depicted in Figure 10 (ORF I b), nucleotides 12074-12841 of the nucleotide sequence of high passage ISU-55 depicted in Figure 10 (ORF 2), nucleotides 12697-13458 of the nucleotide sequence of high passage ISU-55 depicted in Figure 10 (ORF 3), nucleotides 13242-13775 of the nucleotide sequence of high passage ISU-55 depicted in Figure 10 (ORF 4) and nucleotides 13789-14388 of the nucleotide sequence of high passage ISU-55 depicted in Figure 10 (ORF 5).

3. A composition comprising the polypeptides encoded by the isolated DNA sequence of Claim 1.

4. A composition for inducing antibodies against PRRSV comprising an amount of PRRSV encoded by the nucleotide sequence of high passage ISU-55 depicted in Figure 10 (ISU-55) effective to induce said antibodies in a pig and a physiologically acceptable carrier.

5. The composition of Claim 4, wherein said composition reduces lung lesions in five-week-old colostrum-deprived, caesarean-derived pigs by a statistically significant amount wherein said amount is significant a p value less than 0.01, relative to lung lesions in uninoculated five-week-old colostrum-deprived, caesarean-derived pigs.

6. The composition of Claim 4 further comprising an adjuvant.

7. Use of an effective amount of the composition of Claim 4 for the manufacture of a vaccine for protecting a pig from a porcine reproductive and respiratory disease.

8. The use of Claim 7, **characterized in that** said vaccine is administered orally or parentally.

9. The use of Claim 7, **characterized in that** said vaccine is administered intramuscularly, intradermally, intravenously, intraperitoneally, subcutaneously or intranasally.

10. A method of distinguishing PRRSV strain ISU-55 from other strains of PRRSV comprising:
(a) amplifying a DNA sequence of the PRRSV using the following two primers:
55F 5'-CGTACGGCGATAGGGACACC-3' and
3RFLP 5'-GGCATATATCATCACTGGCG-3'
(b) digesting the amplified sequence of step (a) with DraI; and
(c) correlating the presence of three restriction fragments of 626 bp, 187 bp and 135 bp with a PRRSV ISU-55 strain.

## Patentansprüche

1. Eine isolierte DNA-Sequenz, die ein porzines reproduktives und respiratorisches Syndromvirus (PRRSV) codiert, bestehend aus der Nukleotidsequenz des high passage ISU-55, die in Figur 10 dargestellt ist.

2. Eine isolierte DNA-Sequenz, umfassend ein offenes Leseraster des PRRSV nach Anspruch 1, wobei aus diesem offenen Leseraster die Nukleotide 191-7699 der Nukleotidsequenz des high passage ISU-55, die in Figur 10 dargestellt ist (ORF 1 a), die Nukleotide 7687-12069 der Nukleotidsequenz des high passage ISU-55, die in Figur 10 dargestellt ist (ORF 1 b), die Nukleotide 12074-12841 der Nukleotidsequenz des high passage ISU-55, die in Figur 10 dargestellt ist (ORF 2), die Nukleotide 12697-13458 der Nukleotidsequenz des high passage ISU-55, die in Figur 10 dargestellt ist (ORF 3), die Nukleotide 13242-13775 der Nukleotidsequenz des high passage ISU-55, die in Figur 10 dargestellt ist (ORF 4), und die Nukleotide 13789-14388 der Nukleotidsequenz des high passage ISU-55, die in Figur 10 dargestellt ist (ORF 5), ausgewählt sind.

3. Eine Zusammensetzung, welche die Polypeptide, die von der isolierten DNA-Sequenz nach Anspruch 1 codiert werden, umfasst.

4. Eine Zusammensetzung zum Induzieren von Antikörpern gegen PRRSV, welche eine Menge an PRRSV, das von der Nukleotidsequenz des high passage ISU-55, die in Figur 10 dargestellt ist (ISU-55), codiert wird, die wirksam ist, um die Antikörper in einem Schwein zu induzieren, und einen physiologisch verträglichen Träger umfasst.

5. Die Zusammensetzung nach Anspruch 4, wobei die Zusammensetzung Lungenläsionen bei fünf Wochen alten kolostrumdeprivierten durch Kaiserschnitt erhaltenen Schweinen um einen statistisch signifikanten Betrag reduziert, wobei der Betrag signifikant ist bei einem p-Wert von weniger als 0,01, bezogen auf Lungenläsionen bei nicht inokulierten fünf Wochen alten kolostrumdeprivierten durch Kaiserschnitt erhaltenen Schweinen.

6. Die Zusammensetzung nach Anspruch 4, welche weiterhin ein Hilfsmittel umfasst.

7. Verwendung einer wirksamen Menge der Zusammensetzung nach Anspruch 4 zur Herstellung eines Impfstoffes zum Schutz eines Schweins vor einer porzinen reproduktiven und respiratorischen Krankheit.

8. Die Verwendung nach Anspruch 7, **dadurch gekennzeichnet, dass** der Impfstoff oral oder parenteral verabreicht wird.

9. Die Verwendung nach Anspruch 7, **dadurch gekennzeichnet, dass** der Impfstoff intramuskulär, intradermal, intravenös, intraperitoneal, subkutan oder intranasal verabreicht wird.

10. Ein Verfahren zur Unterscheidung von PRRSV-Stamm ISU-55 von anderen Stämmen des PRRSV, umfassend:
(a) Amplifizieren einer DNA-Sequenz des PRRSV unter Verwendung der folgenden zwei Primer:
55F 5'-CGTACGGCGATAGGGACACC-3' und
3RFLP 5'-GGCATATATCATCACTGGCG-3'
(b) Verdauen der amplifizierten Sequenz von Schritt (a) mit DraI; und
(c) Korrelieren des Vorliegens von drei Restriktionsfragmenten von 626 bp, 187 bp und 135 bp mit einem PRRSV ISU-55-Stamm.

## Revendications

1. Séquence d'ADN isolée codant un virus du syndrome dysgénésique et respiratoire porcin (PRRSV) constituée de la séquence nucléotidique de ISU-55 de fort passage décrite sur la figure 10.

2. Séquence d'ADN isolée comprenant un cadre de lecture ouvert du PRRSV selon la revendication 1, cadre de lecture ouvert à partir duquel sont choisis les nucléotides 191 à 7699 de la séquence nucléotidique de ISU-55 de fort passage décrite sur la figure 10 (ORF 1a), les nucléotides 7687 à 12069 de la séquence nucléotidique de ISU-55 de fort passage décrite sur la figure 10 (ORF 1b), les nucléotides 12074 à 12841 de la séquence nucléotidique de ISU-55 de fort passage décrite sur la figure 10 (ORF 2), les nucléotides 12697 à 13458 de la séquence nucléotidique de ISU-55 de fort passage décrite sur la figure 10 (ORF 3), les nucléotides 13242 à 13775 de la séquence nucléotidique de ISU-55 de fort passage décrite sur la figure 10 (ORF 4) et les nucléotides 13789 à 14388 de la séquence nucléotidique de ISU-55 de fort passage décrite sur la figure 10 (ORF 5).

3. Composition comprenant les polypeptides codés par la séquence d'ADN isolée selon la revendication 1.

4. Composition pour induire des anticorps contre PRRSV comprenant une quantité de PRRSV codé par la séquence nucléotidique de ISU-55 de fort passage décrite sur la figure 10 (ISU-55) efficace pour induire lesdits anticorps chez un porc et un support acceptable d'un point de vue physiologique.

5. Composition selon la revendication 4, laquelle composition réduit les lésions pulmonaires chez des porcs qui sont nés par césarienne, privés de colostrum, âgés de cinq semaines d'une quantité significative d'un point de vue statistique, dans laquelle ladite quantité est une valeur p significative inférieure à 0,01 par rapport à des lésions pulmonaires chez des porcs nés par césarienne, privés de colostrum, âgés de cinq semaines n'ayant pas reçu d'inoculation.

6. Composition selon la revendication 4 comprenant en outre un adjuvant.

7. Utilisation d'une quantité efficace de la composition selon la revendication 4 pour la fabrication d'un vaccin pour protéger un porc d'une maladie dysgénésique et respiratoire porcine.

8. Utilisation selon la revendication 7, **caractérisée en ce que** ledit vaccin est administré par voie orale ou parentérale.

9. Utilisation selon la revendication 7, **caractérisée en ce que** ledit vaccin est administré par voie intramusculaire, intradermique, intraveineuse, intrapéritonéale, sous-cutanée ou intranasale.

10. Procédé pour distinguer une souche ISU-55 de PRRSV d'autres souches de PRRSV, comprenant :
(a) l'amplification d'une séquence d'ADN du PRRSV en utilisant les deux amorces suivantes :
55F 5'-CGTACGGCGATAGGGACACC-3' et
3RFLP 5'-GGCATATATCATCACTGGCG-3'
(b) la digestion de la séquence amplifiée de l'étape (a) avec DraI ; et
(c) la mise en corrélation de la présence de trois fragments de restriction de 626 pb, 187 pb et 135 pb avec une souche ISU-55 de PRRSV.
